# EUROPEAN PATENT SPECIFICATION

(11) **EP 2 601 295 B1**
(45) Date of publication and mention of the grant of the patent: **13.12.2017**
(21) Application number: 11724948.2
(22) Date of filing: 06.05.2011
(51) Int. Cl.: C12N 15/82

(54) **PROCESS OF TRANSFECTING PLANTS**
VERFAHREN ZUR TRANSFEKTION VON PFLANZEN
PROCÉDÉ DE TRANSFECTION DE PLANTES

(30) Priority: 11.08.2010 EP 10008393; 07.08.2010 EP 10008267
(43) Date of publication of application: 12.06.2013
(73) Proprietor: Nomad Bioscience GmbH, 80333 München (DE)
(72) Inventor: GIRITCH, Anatoli, 06108 Halle (DE); SYMONENKO, Yuri, 06110 Halle (DE); HAHN, Simone, 06217 Merseburg (DE); TIEDE, Doreen, 06366 Köthen (DE); SHVARTS, Anton, Dr, 117246 Moscow (RU); ROEMER, Patrick, 06780 Zoerbig (DE); GLEBA, Yuri, 10117 Berlin (DE)
(74) Representative: Blodig, Wolfgang
(86) International application number: PCT/EP2011/002279
(87) International publication number: WO 2012/019660

(56) References cited:
- WO-A2-2006/003018
- US-A1- 2004 180 352
- CHEN XINLU ET AL: "A high-throughput transient gene expression system for switchgrass (Panicum virgatum L.) seedlings.", BIOTECHNOLOGY FOR BIOFUELS 2010 LNKD- PUBMED:20459651, vol. 3, 7 May 2010 (2010-05-07), page 9, XP002606783, ISSN: 1754-6834
- MARILLONNET SYLVESTRE ET AL: "Systemic Agrobacterium tumefaciens-mediated transfection of viral replicons for efficient transient expression in plants", NATURE BIOTECHNOLOGY, NATURE PUBLISHING GROUP, NEW YORK, NY, US LNKD- DOI:10.1038/NBT1094, vol. 23, no. 6, 1 June 2005 (2005-06-01), pages 718-723, XP002407902, ISSN: 1087-0156
- GLEBA ET AL: "Viral vectors for the expression of proteins in plants", CURRENT OPINION IN BIOTECHNOLOGY, LONDON, GB LNKD- DOI:10.1016/J.COPBIO.2007.03.002, vol. 18, no. 2, 17 April 2007 (2007-04-17) , pages 134-141, XP022032092, ISSN: 0958-1669
- POGUE GREGORY P ET AL: "Making an ally from an enemy: Plant virology and the new agriculture.", ANNUAL REVIEW OF PHYTOPATHOLOGY. VOLUME 40 ANNUAL REVIEWS, 4139 EL CAMINO WAY, P. O. BOX 10139, PALO ALTO, CA, 94303-0139, USA SERIES : ANNUAL REVIEW OF PHYTOPATHOLOGY (ISSN 0066-4286), 2002, pages 45-74, ISSN: null

## Description

### FIELD OF THE INVENTION

The present invention relates to a process of generating or altering a trait in a plant growing on a field. The invention also relates to a process of producing a protein of interest in a plurality of plants on a field. The invention also relates to a process of protecting crop plants on a field from a pest.

### BACKGROUND OF THE INVENTION

Current genetic engineering processes for agriculture are all based on stable genetic modification of crop species, demonstrated first in 1983 (Fraley et al 1983; Barton et al 1983) and commercialized since 1996. Although the agriculture process based on plant stable genetic transformation is a reality today and is a basis of a very successful new practices, it has multiple limitations, the main ones being very long time and high cost required for development of transgenic crops. General consensus among the companies involved in plant biotechnology is that the R&D process requires, depending on the crop species, between 8 and 16 years, and the total average cost is estimated to be between $100 and $150 million. Because of these limitations, after more than 25 years since the discovery of a plant genetic transformation process, only a handful traits and few GM crop species have been commercialized thus far.

It is known that plant cells and whole plants can also be re-programmed transiently (i.e. without stable integration of new genetic material on a plant chromosome), and the transient processes, such as viral infections, are fast. Such transient processes could in principle allow a very fast modification of plant metabolism in favor of certain traits or products that are of interest to the user. Clearly, such processes require a DNA or RNA vector (a virus or a bacterium), that has been engineered to effectively and safely transfect the plant, with the resultant effect being devoid of undesired side effects. Earlier attempts to use vectors based on plant viruses have been partially successful in that they allow transfection of plants for manufacturing of high-value recombinant proteins such as certain biopharmaceuticals (Gleba et al 2007, 2008; Lico et al 2008). Use of viruses for manipulation of other traits, such as input traits (for example, herbicide resistance, Shiboleth et al 2001; Zhang and Ghabiral 2006) have been described in the literature, but virus transfection introduces so many undesired changes in the infected host that this kind of transient process is not pursued anymore for input traits. Transient processes can also be built around the ability of *Agrobacterium* species to transfer part of its Ti plasmid to eukaryotic, in particular, plant cell. Use of *Agrobacterium-*based transfection is a basis for genetic manipulations such as genetic transformation protocols and of laboratory transient transfection assays. Industrial applications of *Agrobacterium-*based transfection have also been limited to recombinant protein manufacturing, because the optimal application conditions such as vacuum infiltration of plants with bacterial suspensions cannot be used on a large scale in the field, whereas spraying aerial parts or watering plants with bacterial solutions results in a supposedly very small proportion of plant cells to be transfected, and previous studies simply did not address that specific question. The combination of *Agrobacterium* delivery and use of virus as a secondary messenger in one process has been successful in manufacturing high-value recombinant proteins including complex biopharmaceuticals such as full IgG antibodies. However, when it comes to traits such as input traits or traits requiring subtle targeted reprogramming of plant cell metabolism, this magnifection process has the same limitations as viral vectors have.

There is considerable knowledge in the area of the use of microorganisms for controlling certain processes that require interaction of microbes with plants, including use of microorganisms such as *Lactobacillus* and *Saccharomyces* yeasts for biomass fermentation (preparation of fermented food, drinks), for biocontrol (*Agrobacterium, Myrotecium,* strains), and use of strains of *Rhizobium* for improved nitrogen fixation. In research papers and patents in which microorganisms have been explored as biocontrol agents, there is a considerable body of knowledge of how the living cells should be applied to plant surfaces; in particular, studies have been performed that identified spraying conditions and adjuvants (wetters, stickers, etc.) to be used in the spray mixtures. Examples of such research are numerous. The following papers exemplify the state of the art in this area: Arguelles-Arias et al 2009; Nam et al 2009; reviewed by Johnson 2009.

There are registered *Agrobacterium rhizogenes*/*radiobacter* strains that have been used for decades for control of crown gall disease in vineyards and orchards. There are two commercially used strains, one being a natural strain carrying plasmid (K84), and the other, a genetically modified derivative that has been modified through deletion of the gene necessary for the conjugative plasmid transfer (K1026). (Kerr and Tate 1984; Vicedo et al 1993; Reader et al 2005; Kim et al 2006; reviewed in Moore 1988).

*Agrobacterium tumefaciens* and *A. rhizogenes* are broadly used in research laboratories worldwide for transient transfection and stable genetic transformation of plants. These applications are based on the ability of *Agrobacterium* to transfer genetic information to eukaryotic cells. Many of the genetically modified plants cultivated today, such as soybeans, canola and cotton, have been generated through *Agrobacterium*-mediated genetic transformation. The essential difference between the transient and stable transformation is that in the process of stable transformation, *Agrobacterium*-delivered DNA is eventually integrated into a plant chromosome, and is afterwards inherited by the plant progeny. Such integration events are rare even in laboratory experiments specifically designed to provide massive contacts between plant cells and bacteria; thus for the selection of stable transformants, specific selective screening methods have to be utilized. Subsequently, the knowledge accumulated in this science domain is of limited value to those interested in transient processes that have to be designed so as to have a massive character and affect multiple cells of the plant body.

Transient transfection, on the other hand, takes into account only earlier steps of *Agrobacterium-*driven DNA delivery into a nucleus of a plant cell, along with the fact that such delivered DNA molecules, if properly designed to constitute a transcription unit carrying plant-specific promoter and terminator and a coding part, will be transcribed in a nucleus even in the absence of said DNA integration into a plant chromosome, such expression resulting in a transient reprogramming of a plant cell. Such reprogramming has been first achieved early on and has been developed into a standard laboratory tool for rapid evaluation of different genetic experiments. Whereas there is considerable body of knowledge about *Agrobacterium-*mediated DNA transfer to plant cells, with exception of few cases, that information is limited to laboratory scale experiments, and thus far, there were very few attempts to develop industrial scale applications involving *Agrobacterium* as a DNA vector. One of the limitations of laboratory applications is the fact that *Agrobacterium-*based DNA delivery requires certain treatments that are difficult or impossible to apply in open field or on a large scale. In typical transient experiments, cultured plant cells or parts of plants are treated with an excess of bacteria to provide for maximum delivery. In typical research experiments, one is also interested in expression levels that are not economically viable if done on an industrial scale. In general, the research done in this domain has led the inventors to the conclusion that the parameters seriously affecting transient expression are those allowing for the best interaction access of agrobacteria to plant cells within a plant body. Most such studies utilize vacuum infiltration, injection into plant leaf or surfactant treatment, wounding of plant surface e.g. with razor blades, or combination thereof. In fact, the only group that is developing an *Agrobacterium-*based transfection process for commercial production of recombinant proteins that does not involve further (virus-based) amplification of the original DNA, is the group of Medicago (D'Aoust et al 2008, 2009; Vezina et al, 2009) that is entirely relying on vacuum infiltration as a delivery method. However, because of being based on great excess of bacteria to plant cell ratio, current laboratory protocols used for transient transfection of plants do not have serious translational value, i.e. they cannot be directly replicated on an industrial level. Except in few cases (e.g. Vaquero et al, 1999, D'Aoust et al, 2008, 2009) they also have not addressed quantitatively the issue of efficiency of the transient transfection process. (Examples of such research are multiple, we provide a citation for just a few representative ones: Li et al, 1992; Liu et al, 1992; Clough and Bent, 1998; De Buck et al, 1998, 2000; Chung et al, 2000; Yang et al, 2000; Zambre et al, 2003; Wroblewski et al, 2005; Lee and Yang, 2006; Zhao et al, 2006; Shang et al, 2007; Jones et al., 2009; Li et al, 2009; De Felippes and Weigel, 2010). Except in two cases described below, there were no attempts in the literature to quantify the efficiency of the transient process or to provide sufficient understanding that would lead to potential commercial large-scale exploitation of the phenomenon.

One of the industrial processes being under development today is magnifection, a process that is based on vacuum-infiltration of agrobacteria into leaves of plants. The magnifection process (trademarked by Icon Genetics GmbH as magnICON® and covered by several patents/patent applications) is a simple and indefinitely scalable protocol for heterologous protein expression in plants, which is devoid of stable genetic transformation of a plant, but instead relies on transient amplification of viral vectors delivered to multiple areas of a plant body (systemic delivery) by *Agrobacterium* as DNA precursors. Such a process is in essence an infiltration of whole mature plants with a diluted suspension of agrobacteria carrying T-DNAs encoding viral RNA replicons. In this process, the bacteria assume the (formerly viral) functions of primary infection and systemic movement, whereas the viral vector provides for cell-to-cell (short distance) spread, amplification and high-level protein expression. Initial demonstration that viral infection can be initiated by agrobacteria delivering a viral genome copy into a plant cell comes from the pioneering work of Grimsley et al, 1986, in which a DNA virus has been delivered, and a first, although very inefficient, infection with tmv, a cytoplasmic RNA virus delivered as a DNA copy, came from the work of Turpen et al 1993. Current technology, however, is extremely efficient and a few adult tobacco plants are sufficient for early construct optimization and fast production of milligram to gram quantities of recombinant protein for pre-clinical or clinical evaluation, or, in case of individualized vaccines, for manufacturing. The scale-up (industrial) version is essentially the same, but is built around fully assembled viral vectors (rather than pro-vectors requiring *in planta* assembly) and requires apparatuses for high-throughput *Agrobacterium* delivery to whole plants by vacuum infiltration. The process can be scaled up but it requires submersion of aerial parts of plants into bacterial suspension under vacuum (the process involves inverting plants grown in pots or in trays), a procedure that imposes imitations on the volumes of biomass that can be treated in this way, on the throughput of the process, on the ways the plants can be cultivated prior to treatment, and it also carries certain costs that limit the use of the process to high-cost products, such as recombinant biopharmaceuticals only. The magnifection process is efficient as it allows transfection of almost all leaf cells in treated plants, or approximately 50% of the total aerial plant biomass (the rest being stems and petioles). The process has been optimized in many ways, in particular through improvement of viral replicon release through optimization of the posttranslational modification of the primary DNA transcripts (Marillonnet et al, 2005). However, the current process has been built entirely around bacterial delivery methods such as injection into plant leaf or vacuum-infiltration (e.g. Simmons et al, 2009), wounding of leaves (Andrews and Curtis, 2005), or pouring agrobacteria into soil ('agrodrenching', Ryu et al, 2004; Yang et al, 2008), whereas said methods can not be applied for the mass treatment of the plants in a field (reviewed in Gleba et al, 2004, 2007, 2008; Lico et al, 2008; original articles include Giritch et al. 2006; Marillonnet et al., 2004, 2005; Santi et al, 2006; and ideologically similar papers from other research groups - Voinnet et al, 2003; Sudarshana et al, 2006; Gao et al, 2006; Mett et al, 2007; Lindbo, 2007a,b; Plesha et al, 2007, 2009; Huang et al, 2006; Regnard et al 2009; Green et al, 2009; Shoji et al, 2009).

It should be mentioned that although *Agrobacterium tumefaciens* and *A. thizogenes* are the DNA vectors that are used in the majority of cases, there are other species of bacteria that can perform similar DNA transfer to plant cells (Broothaerts et al, 2005).

The attempts to quantify *Agrobacterium* treatment on whole plants after vacuum-infiltration have been performed by a few research groups only. In the papers of Joh et al, 2005, 2006, it has been concluded that the highest used bacteria density of 10⁹ cfu/ml was the best (as opposed to 10⁸ cfu/ml or 10⁷ cfu/ml), as measured by the total recombinant protein expression. In experiments of Lindbo, 2007a,b, essentially similar results have been obtained as in our work, however, no counting of transfected cells has been performed and the conclusions were derived from recombinant protein expression levels.

The attempts to use *Agrobacterium* treatment on whole plants without vacuum-infiltration result in a very low number of initially transfected cells, thus greatly limiting the practical application of the process. One way of circumventing this initial limitation disclosed in literature is the use of an efficient secondary messenger such as a plant virus that would allow amplifying the initially inefficient process by complementing it with a virus-based cell-to-cell and systemic movement (Azhakanandam et al, 2007). The *Agrobacterium-*based delivery of DNA copies of plant viruses or plant viral vectors has been described a long time ago (Grimsley et al, 1986, and for TMV - Turpen et al, 1993), and it allows to spread initial replicon delivered to a few cells in plant to the rest of the body by using viral infection process such as cell-to-cell movement and systemic movement of virus. Such a process has limited practical utility for our purposes, because viral infection dramatically changes plant performance; all currently entertained applications are in the area of recombinant protein manufacturing in plants (reviewed by Gleba et al, 2007, 2008). It should also be noted that the cited paper of Azhakanandam et al, 2007 does not even attempt to quantify the efficiency of the initial transfection and it is based on very high amount of agrobacteria in the transfection media.

WO 2006/003018 mentions that Agrobacteria could be delivered into plant tissue by using a high-pressure spray device to deploy a mixture of agrobacteria together with an abrasive to plants. Chen et al., Biotechnology for Biofuels, 2010, 3:9 describes a high-throughput transient gene expression system for switchgrass seedlings, wherein, *inter alia,* a "vortex-carborundum-sonication" treatment of seedlings is used.

Departing from the prior art, it is an object of the present invention to provide an efficient process of transiently transfecting plants so as to be applicable to many plants growing on a farm field. It is also an object of the invention to provide a process of altering a trait in plants growing on a farm field. Notably, it is an object of the invention to provide an efficient process allowing transient plant transfection using *Agrobacterium* on a large scale without the need for the application of pressure differences to introduce *Agrobacterium* into the intercellular space of plants..

### SUMMARY OF THE INVENTION

Accordingly, the present invention provides the following:
(1) A process of generating or altering a trait in a plant, comprising
   (i) growing said plant up to a desired growth state;
   (ii) expressing, in said plant, a protein or an RNA capable of generating or altering said trait, comprising spraying aerial parts of said plant with an aqueous suspension containing cells of an *Agrobacterium* strain and at least one abrasive suspended in said suspension,
   said *Agrobacterium* strain comprising a DNA molecule comprising a nucleic acid construct containing a DNA sequence of interest, said DNA sequence of interest encoding said protein or said RNA, as further defined in claim 1.
(2) A process of producing a protein of interest in a plant, comprising
   (i) growing said plant up to a desired growth state;
   (ii) expressing, in said plant, said protein of interest, comprising spraying aerial parts of said plant with an aqueous suspension containing cells of an *Agrobacterium* strain and at least one abrasive suspended in said suspension,
   said *Agrobacterium* strain comprising a DNA molecule comprising a nucleic acid construct containing a DNA sequence of interest, said DNA sequence of interest encoding said protein of interest, as further defined in claim 2.
(3) A process of protecting crop plants on a field from a pest, comprising
   (i) growing said plants in soil of said field;
   (ii) determining, in a desired growth state of said plants, infestation of at least one of said plants by a pest;
   (iii) expressing, in said plants, a protein or an RNA that is detrimental to the pest determined in the previous step, comprising spraying aerial parts of said plants with an aqueous suspension containing cells of an *Agrobacterium* strain and at least one abrasive suspended in said suspension,
   said *Agrobacterium* strain comprising a DNA molecule comprising a nucleic acid construct containing a DNA sequence of interest operably linked to a promoter, said DNA sequence of interest encoding said protein or said RNA, as further defined in claim 3.
(4) The process according to any one of (1) to (3), wherein said aqueous suspension contains said cells of said *Agrobacterium* strain in a concentration of at most 2.2·10⁷, preferably of at most 1.1·10⁷, more preferably of at most 4.4·10⁶, more preferably of at most 1.1·10⁶ cfu/ml of said suspension.
(5) The process according to any one of (1) to (4), wherein said abrasive is a particulate inorganic carrier for wettable powders, such as silica or carborundum.
(6) The process according to (5), wherein said aqueous suspension contains said abrasive in an amount of between 0.02 and 2, preferably between 0.05 and 1 and more preferably between 0.1 and 0.5 % by weight of said suspension.
(7) The process according to (5) or (6), wherein the median particle size of the abrasive added to the suspension is between 0.1 and 30, preferably between 0.1 and 10, more preferably between 0.5 and 10, and most preferably between 0.5 and 5 µm.
(8) The process according to any one of (5) to (7), wherein the abrasive has a D90 value of at most 40 µm, preferably of at most 30 µm; and wherein the abrasive does not contain particles having a size above 45 µm, preferably not above 40 µm.
(9) The process according to any one of (1) to (8), wherein the spray adjuvant is an organo-silicone surfactant.
(10) The process according to (9), wherein the organo-silicone surfactant is a polyalkyleneoxide-modified heptamethyltrisiloxane.
(11) The process according to any one of (1) to (10), wherein said nucleic acid construct is flanked by a T-DNA border sequence on at least one side, which permits the transfer of said nucleic acid construct into cells of said plant.
(12) The process according to any one of (1) to (11), wherein said nucleic acid construct encodes a replicating viral vector encoding said protein of interest, said replicating viral vector being incapable of system movement in said plant.
(13) The process according to any one of (1) to (11), wherein said DNA sequence of interest is operably linked to a promoter active in plant cells.
(14) The process according to any one of (1) to (13), wherein said plant is dicotyledonous plant.
(15) The process according to (14), wherein said plant is tobacco or other species of the *Nicotiana* genus, sugar beets or other species of the *Beta* genus, tomato, potato, pepper, soybean, alfalfa, pea, beans, rapeseed or other species of the *Brassica* genus, cotton.
(16) The process according to any one of (1) to (13), wherein said plant is monocotyledonous plant.
(17) The process according to (16), wherein said plant is rice, maize, wheat, barley, oats, millet, sorghum.
(18) The process according to (2), wherein said protein is a cellulase used in saccharification of cellulose or hemicellulose polymers.

Also described is a use of particulate inorganic material for transient transfection of plants. In said use, plants may be sprayed with an aqueous suspension containing *Agrobacterium* cells and the particulate inorganic material.

Further described is a process of transfecting a plant, comprising spraying aerial parts of said plant with an aqueous suspension containing at least one abrasive in said suspension, followed by spraying said aerial parts of said plant with an aqueous suspension containing cells of an *Agrobacterium* strain comprising a DNA molecule comprising a nucleic acid construct containing a DNA sequence of interest. Said DNA sequence of interest may encode a protein or RNA to be expressed in said plant.

Moreover, described is a process of transfecting a plant with a nucleic acid construct or a DNA sequence of interest, comprising spraying said plant such as aerial parts of said plant with an aqueous suspension containing cells of an *Agrobacterium* strain, said *Agrobacterium* strain comprising a DNA molecule comprising a nucleic acid construct containing a DNA sequence of interest; wherein said aqueous suspension contains said cells of said *Agrobacterium* strain in a concentration of at most 2.2·10⁷, preferably of at most 1.1·10⁷, more preferably of at most 4.4·10⁶, more preferably of at most 1.1·10⁶ cfu/ml of said suspension; and wherein said suspension further comprises an agricultural spray adjuvant, preferably a non-ionic surfactant or wetting agent.

The inventors of the present invention have found a way of strongly increasing the probability of achieving plant transfection by *Agrobacerium.* The inventors have found that addition of a particulate material insoluble in aqueous *Agrobacterium* suspensions strongly increases the transfection efficiency achievable by spraying of aerial parts of the plant with the suspension. The high efficiency achieved allows for the first time transfection of plants with *Agrobacterium* suspensions on a large scale such as on agricultural fields, whereby the cumbersome infiltration methods making use of pressure differences can be avoided. The invention also allows transfection of plants that have so far not been amenable to spray transformation with *Agrobacterium* suspensions.

### DESCRIPTION OF THE FIGURES

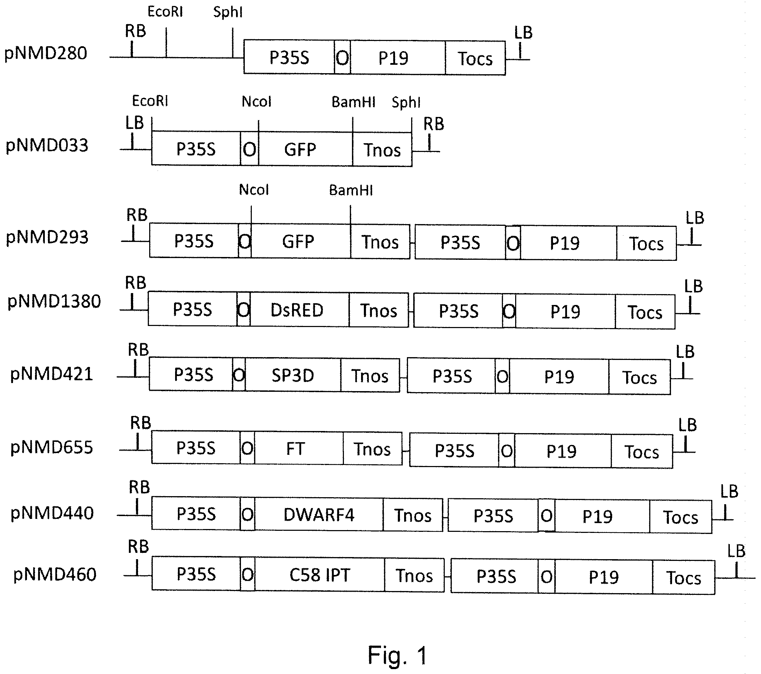

Fig. 1 shows schematically vectors used in the examples. RB and LB stand for the right and left borders of T-DNA. P35S: cauliflower mosaic virus 35S promoter; O: omega translational enhancer; Tnos: nopaline synthase terminator; Tocs: ocs terminator.

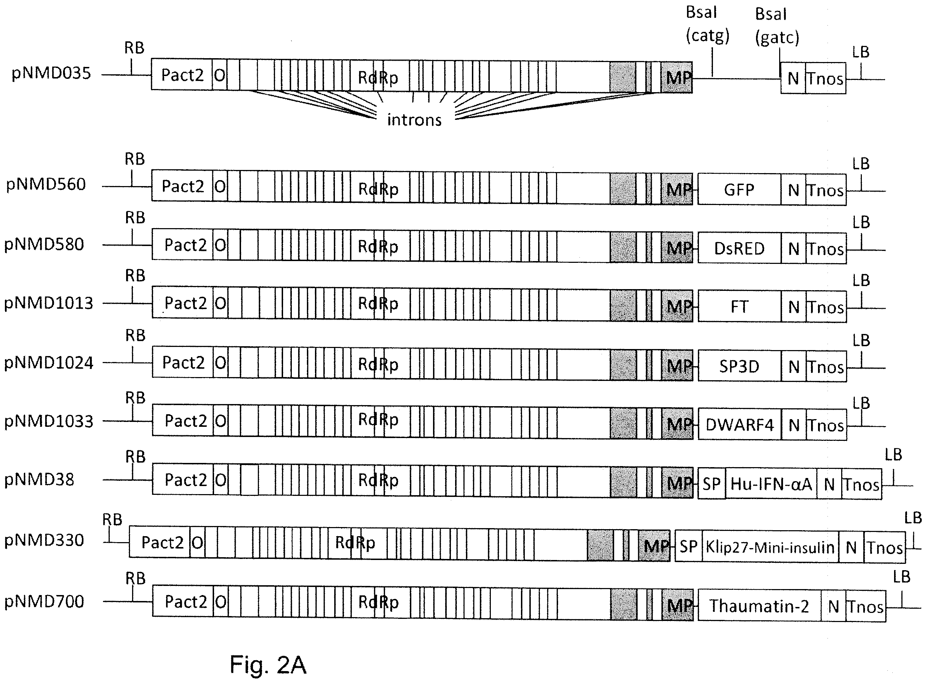

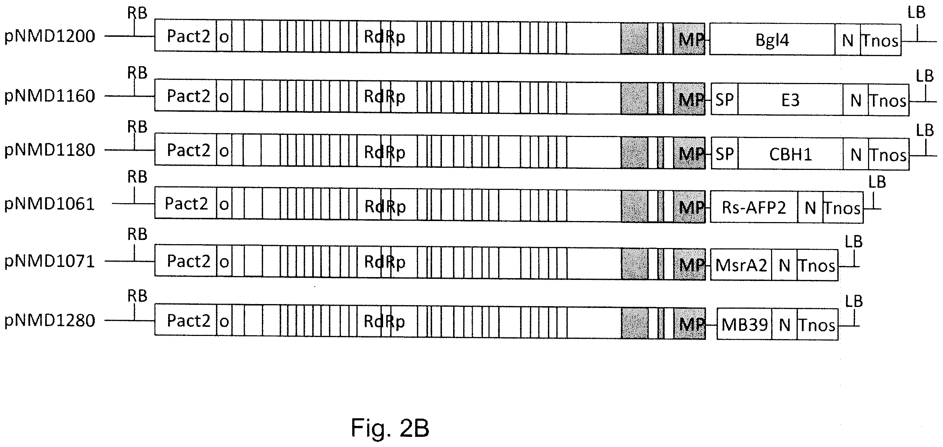

Fig. 2A and B depict TMV-based viral vectors with cell-to cell movement ability. Pact2: promoter of *Arabidopsis* actin2 gene; o: 5' end from TVCV (turnip vein clearing virus); RdRp: RNA-dependent RNA polymerase open reading frame (ORF) from cr-TMV (crucifer-infecting tobamovirus); MP: movement protein ORF from cr-TMV; N: 3'-non-translated region from cr-TMV; Tnos or nos: nopaline synthase terminator; SP: signal peptide; white segments interrupting grey segments in the RdRp and MMP ORFs indicate introns inserted into these ORFs for increasing the likelihood of RNA replicon formation in the cytoplasm of plant cells, which is described in detail in WO2005049839.

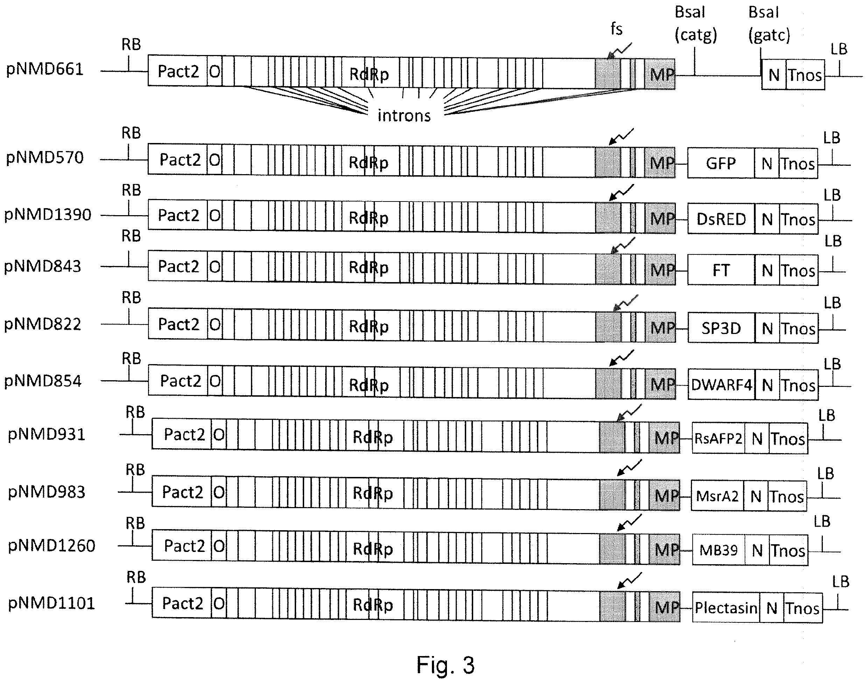

Fig. 3 depicts TMV-based vectors lacking cell-to cell movement ability. A point mutation in the MP ORF leads to a frame shift (fs) preventing correct MP translation.

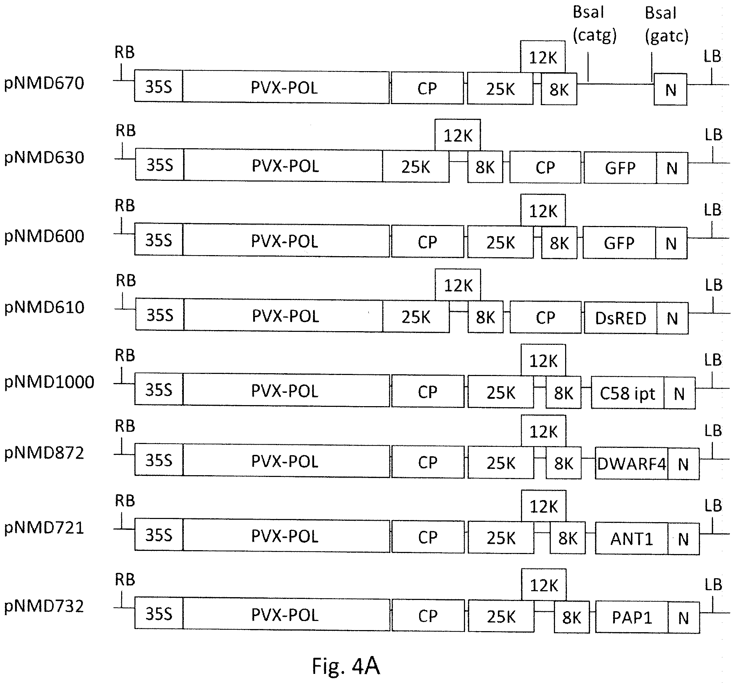

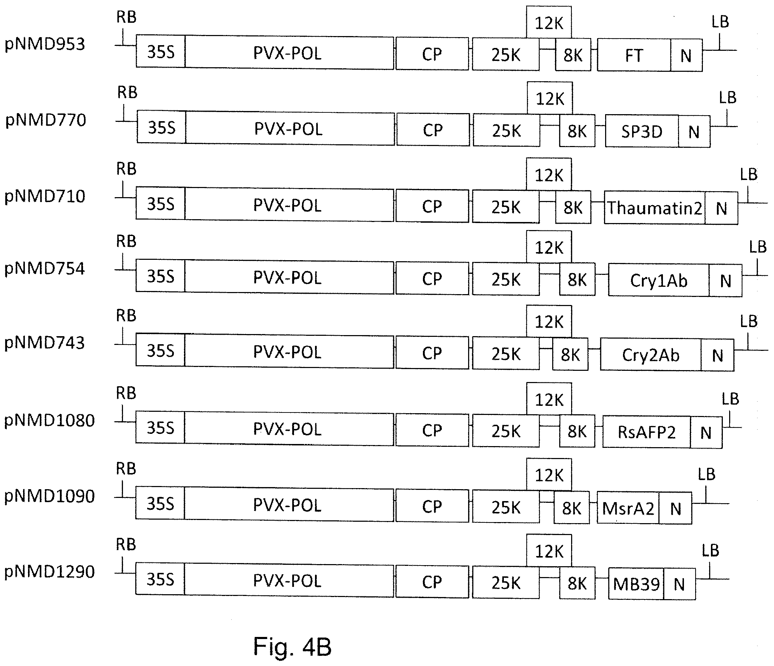

Fig. 4A and B depict PVX (potato virus X)-based vectors with cell-to-cell movement ability. PVX-pol: RNA-dependent RNA polymerase from PVX; CP: coat protein ORF; 25K, 12K and 8 together indicate the 25KDA, 12 kDa and 8 kDa triple gene block modules from PVX; N: 3'-untranslated region from PVX.

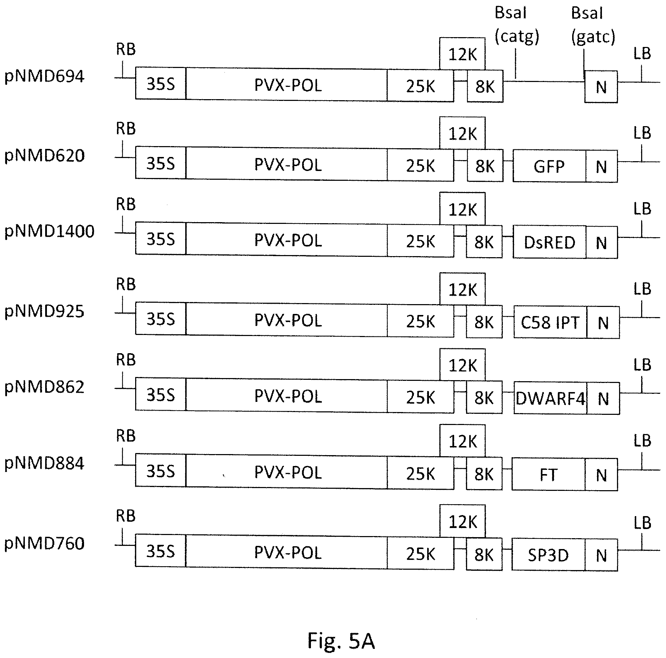

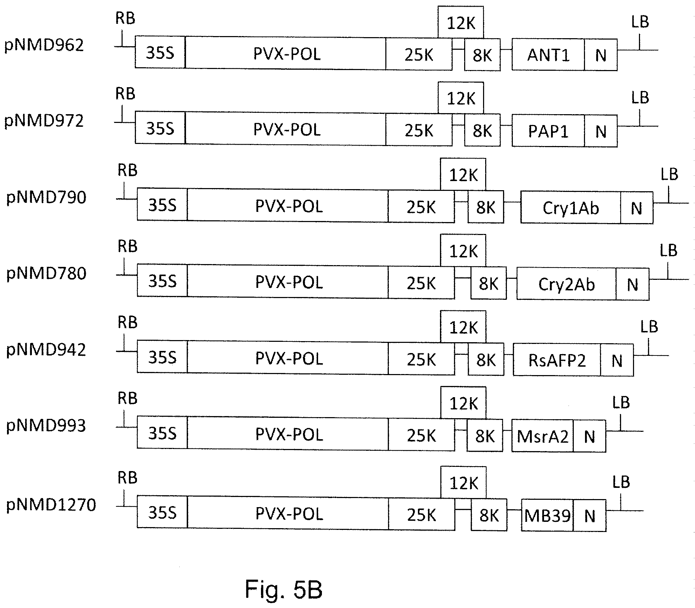

Fig. 5A and B depict PVX-based vectors with deletion of the coat protein coding sequence disabled for both systemic and cell-to cell movement.

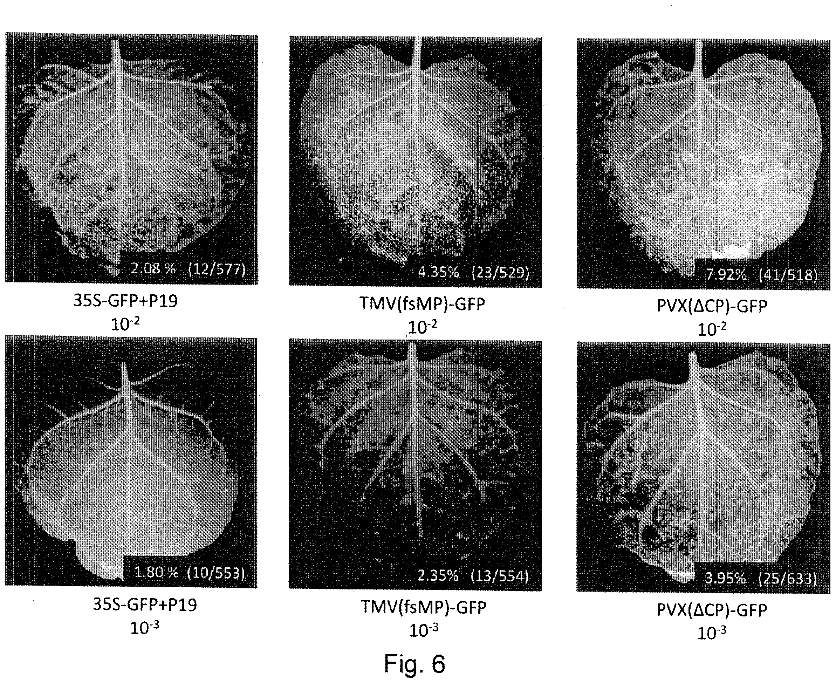

Fig. 6 photographs showing GFP fluorescence 4 dpi (days post inoculation) under uv light due to GFP expression after dipping leaves from *Nicotiana benthamiana* plants for 1 minute into diluted agrobacterial cultures containing 0.1 % by weight of the surfactant Silwet L-77 as described in Example 2. Numerals 10⁻² and 10⁻³ show the dilution factor of the overnight agrobacterial cultures of OD=1.5 at 600 nm and thus indicate a 100-fold and 1000-fold dilution, respectively. The vectors used are indicated and can be associated with the appropriate vector shown in Fig. 1 to 5. 35S-GFP+P19-transcriptional vector expressing GFP under the control of 35S promoter and co-expressed with P19 suppressor of silencing (pNMD293); TMV(fsMP)-GFP and PVX(ΔCP)-GFP-viral vectors lacking cell-to-cell movement ability (pNMD570 and pNMD620, respectively). The percentage of GFP-expressing cells (indicated) was counted after the isolation of protoplasts from the left half of the leaf blade.

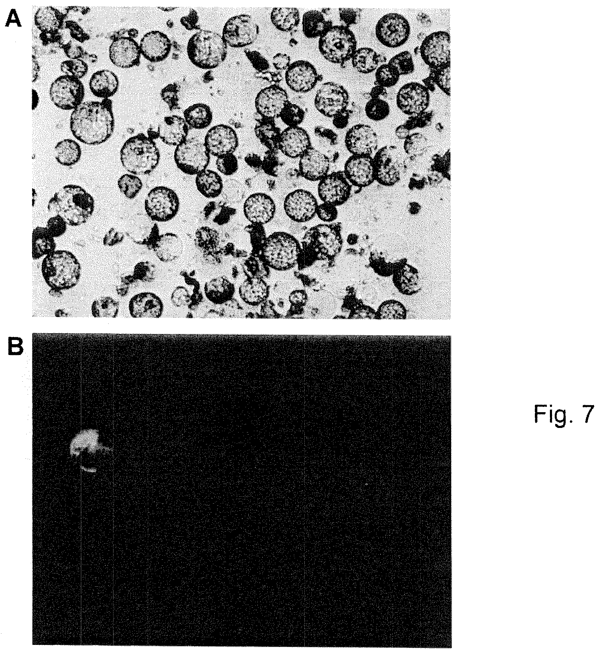

Fig. 7 shows photographs of isolated protoplasts for counting of GFP expressing cells. GFP-expressing protoplasts isolated after the dipping of *Nicotiana benthamiana* leaves in agrobacterial suspension (OD600=1.5, dilution factor 10⁻³). TMV(fsMP)-GFP-TMV-based viral vector lacking cell-to-cell movement ability (pNMD570). 0.1% Silwet, 1min dipping, protoplasts were isolated at 4dpi.

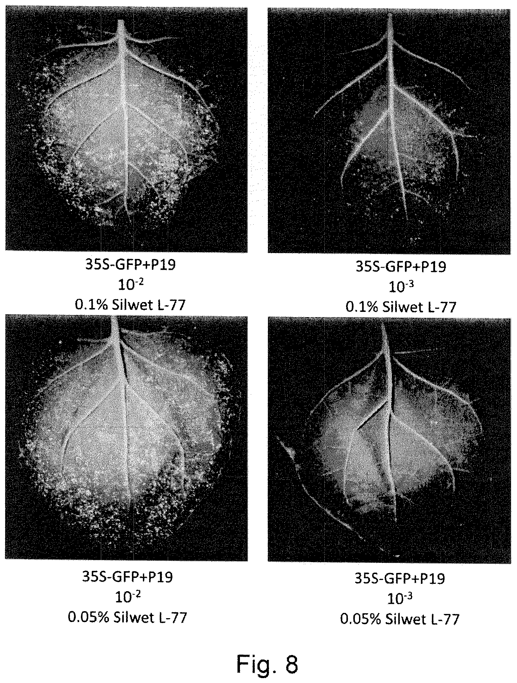

Fig. 8 Influence of Silwet L-77 concentration and agrobacterial culture density on the transfection efficiency after the dipping of *Nicotiana benthamiana* leaves in agrobacterial suspension (OD600=1.5, dilution factors 10⁻² and 10⁻³). 35S-GFP + P19 - transcriptional vector expressing GFP under the control of 35S promoter and co-expressed with P19 suppressor of silencing (pNMD293). Concentration of Silwet 0.1% and 0.05%, 10 sec dipping, 8 dpi.

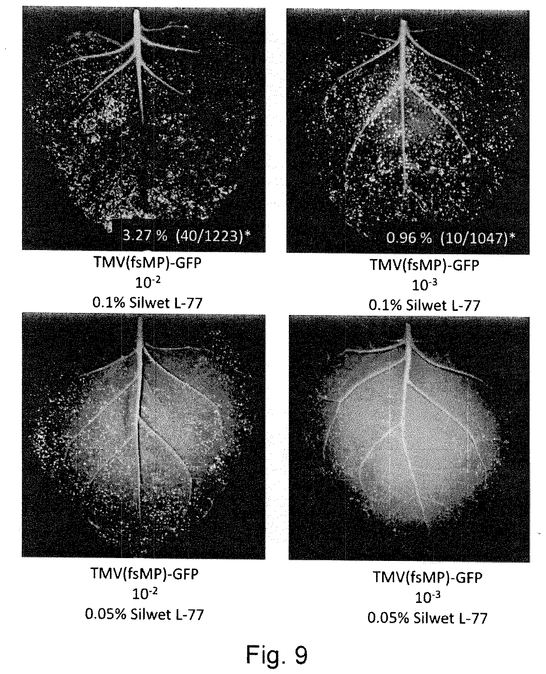

Fig. 9 Influence of Silwet L-77 concentration and agrobacterial culture density on the transfection efficiency after the dipping of *Nicotiana benthamiana* leaves in agrobacterial suspension (OD600=1.5, dilution factors 10⁻² and 10⁻³). TMV(fsMP)-GFP - TMV-based viral vector lacking cell-to-cell movement ability (pNMD570). Concentration of Silwet - 0.1% and 0.05%, 10 sec dipping, 8 dpi. Percent of GFP-expressing cells was counted after the isolation of protoplasts from the left half of the leaf blade.

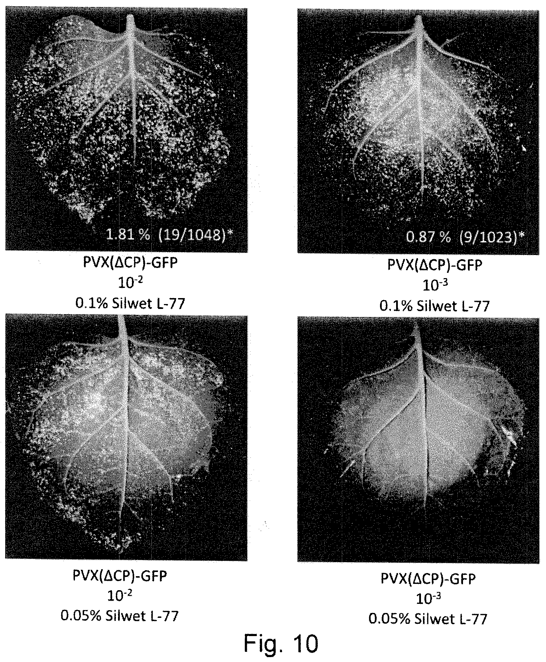

Fig. 10 Influence of Silwet L-77 concentration and agrobacterial culture density on the transfection efficiency after the dipping of Nicotiana benthamiana leaves in agrobacterial suspension (OD600=1.5, dilutions factors 10⁻² and 10⁻³). PVX(ΔCP)-GFP - PVX-based viral vector lacking cell-to-cell movement ability (pNMD620). Concentration of Silwet 0.1% and 0.05%, 10 sec dipping, 8 dpi. Percent of GFP-expressing cells was counted after the isolation of protoplasts from the left half of the leaf blade.

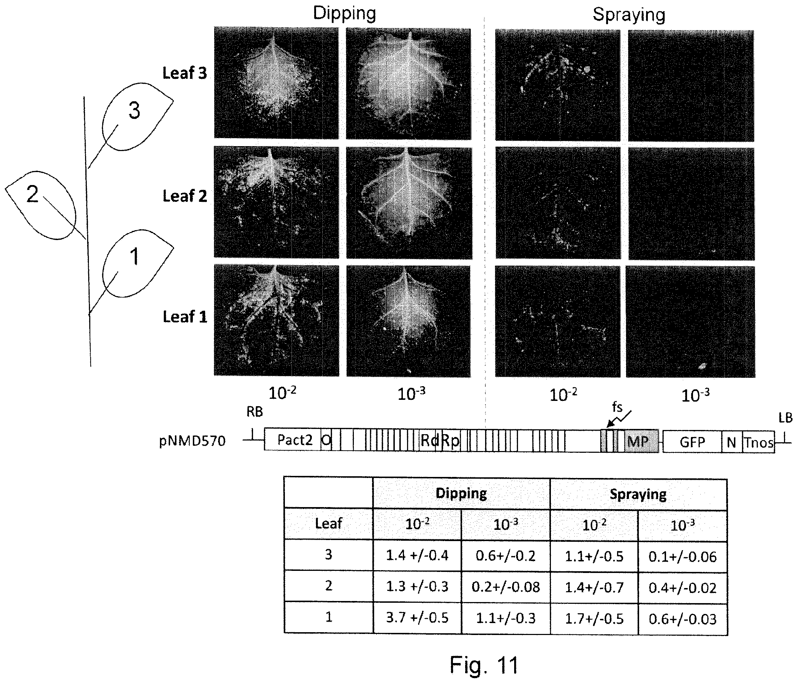

Fig. 11 Comparison of transfection rates achieved by dipping and spraying *Nicotiana benthamiana* into/with agrobacterial suspensions. Dilution factors and transfection rates are indicated. The Silwet L-77 concentration was 0.1 weight-%. Agrobacterial culture (pNMD570, TMV-based viral vector lacking cell-to-cell movement ability) was grown to OD600=1.5 and diluted 100-fold (10⁻²) and 1000-fold (10⁻³) in buffer for infiltration supplemented with 0.1% Silwet-77. Dipping duration 10 sec. Pictures are taken at 8 dpi. Percent of GFP-expressing cells was counted after the isolation of protoplasts from the left half of the leaf blade.

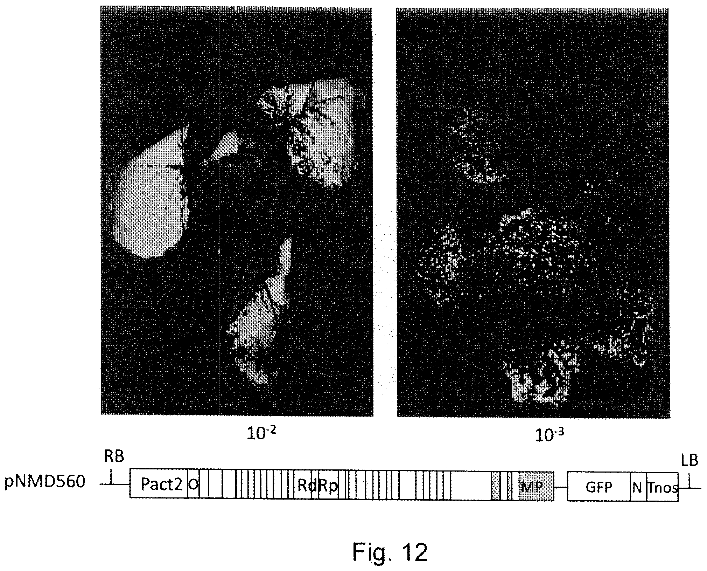

Fig. 12 shows photographs of GFP expression after delivery of diluted agrobacteria to *Nicotiana benthamiana* by spraying with surfactant: TMV-based viral vector with cell-to-cell movement ability (TMV-GFP, pNMD560); agrobacterial suspensions of OD600=1.5 were diluted by dilution factors of 10⁻² or 10⁻³ as indicated; 0.1% SilwetL-77, photograph taken 8dpi.

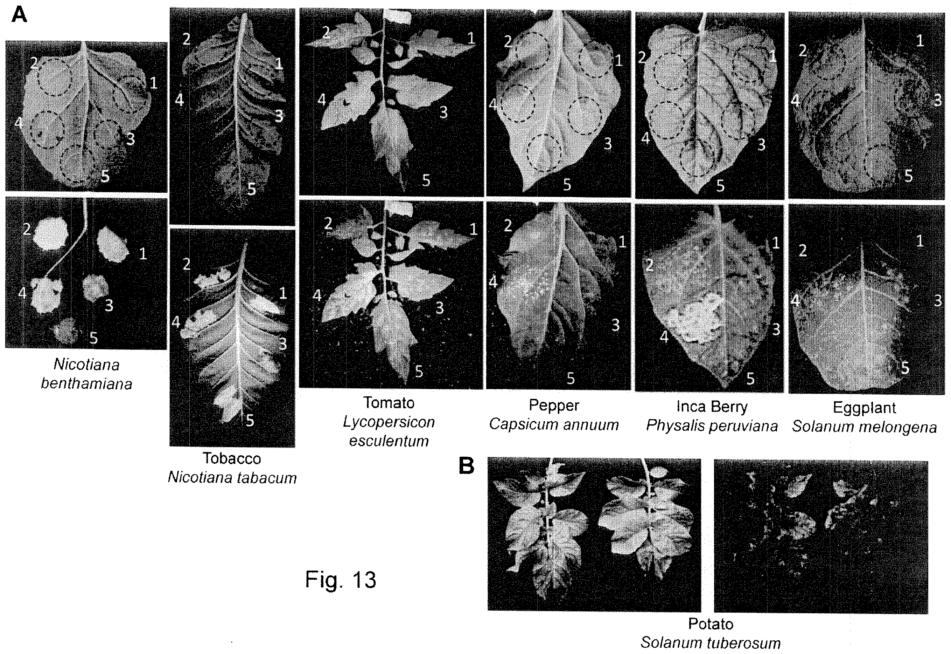

Fig. 13A shows the results of transfection experiments by infiltrating (using a needleless syringe) different plants with 100-fold (dilution factor 10⁻²) dilutions of OD600=1.5 agrobacterial cultures. The suspensions used for spraying contained 0.1 % by weight Silwet L-77 as described in example 3. For each case, the same leaf is shown under normal light and under uv light showing GFP expression. Dashed circles indicate the treated leaf area. Numerals next to the treated leaf area indicate the strain/vector used as follows:
   A) Syringe infiltration. Vectors: 1 - TMV(fsMP)-GFP (pNMD570); 2 - TMV(MP)-GFP pNMD560); 3 - PVX(ΔCP)-GFP (pNMD620); 4 - PVX(CP)-GFP (pNMD630); 5 - 35S-GFP + P19 (pNMD293). Agrobacterial cultures were grown to OD600=1.5 and diluted 100 fold; photographs taken 8 dpi.
   B) Similar as in A, however transfection was performed by vacuum infiltration. Vector: PVX(CP)-GFP (pNMD630). Agrobacterial cultures were grown to OD600=1.5 and diluted 100-fold; photographs taken 43 dpi.

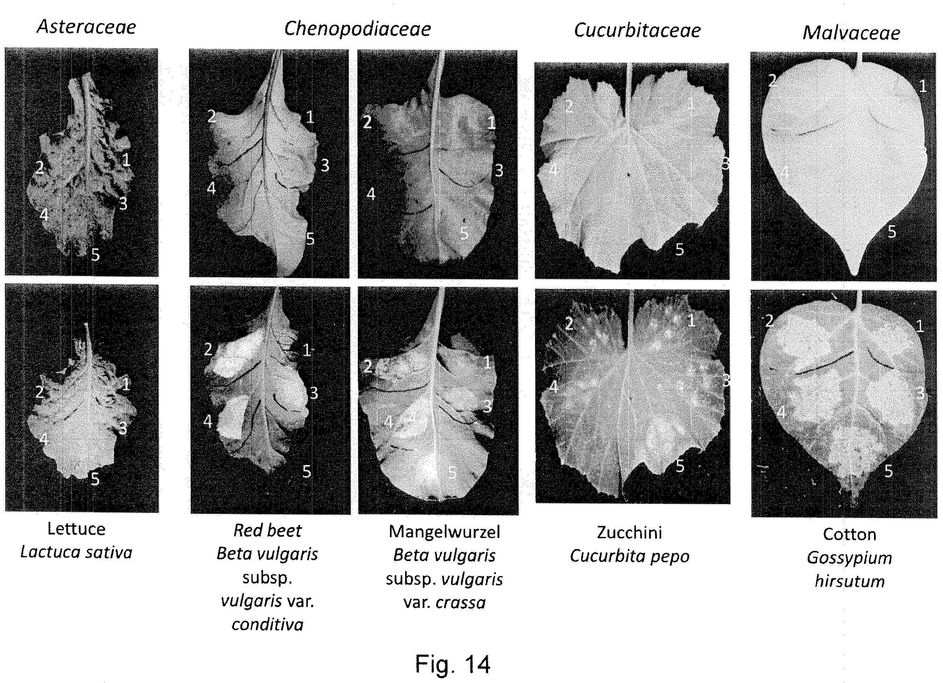

Fig. 14 Screening for optimal expression vector using syringe infiltration with family members of Asteraceae, Chenopodiaceae, Cucurbitaceae and Malvaceae. Numerals indicate the strains/vectors used as follows: 1 - TMV(fsMP)-GFP (pNMD570); 2 - TMV(MP)-GFP (pNMD560); 3 - PVX(ΔCP)-GFP pNMD620); 4 - PVX(CP)-GFP (pNMD630); 5 - 35S-GFP + P19 (pNMD293). Dilution factor of agrobacterial cultures (OD600=1.5-1.7): 10⁻²; photographs taken 8 dpi.

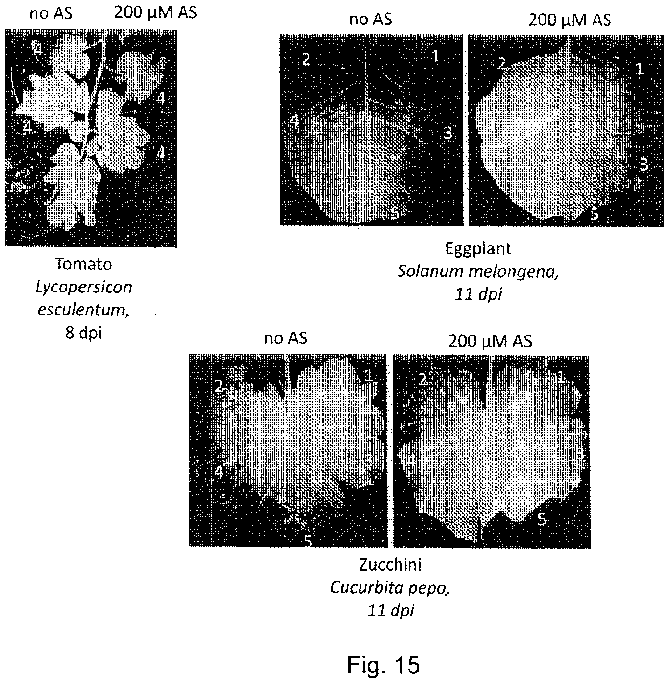

Fig. 15 shows factors enhancing agrobacterial transfection: acetosyringone (AS). Vectors: 1 - TMV(fsMP)-GFP (pNMD570); 2 - TMV(MP)-GFP (pNMD560); 3 - PVX(ΔCP)-GFP (pNMD620); 4 - PVX(CP)-GFP (pNMD630); 5 - 35S-GFP + P19 (pNMD293). Dilution of agrobacterial cultures (OD600=1.5-1.7): 10⁻². For AS treatment, 200 µM acetosyringone (AS) was added to agrobacterial suspensions 2 hours before transfection. For comparison, leaves transfected with suspensions not containing AS are also shown (no AS).

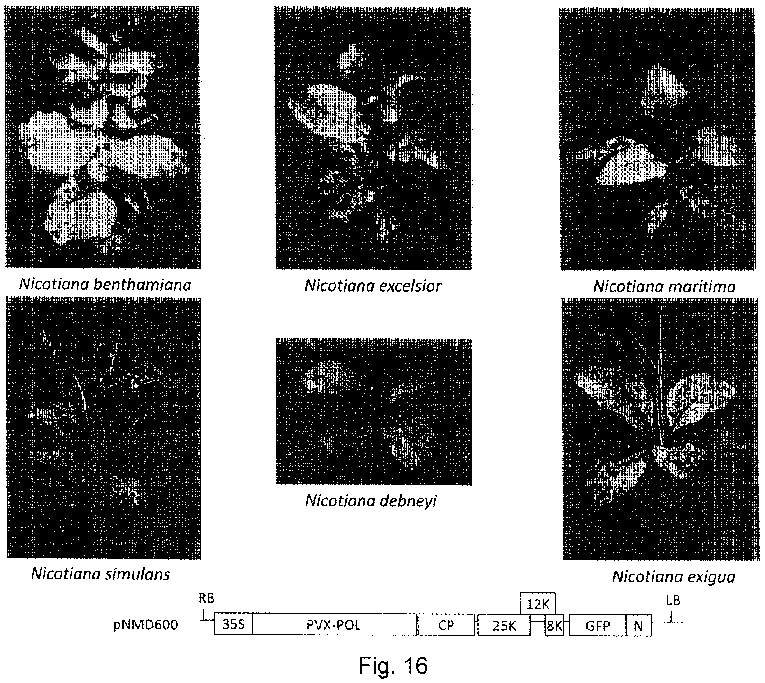

Fig. 16 Photographs showing GFP expression under uv light in various *Nicotiana* species after spraying entire plants with 1000-fold diluted agrobacterial suspensions of OD600=1.0. PVX-based viral vector with cell-to-cell and systemic movement ability were used (PVX(+CP)-GFP, pNMD600). Sprayed suspensions contained 0.1 weight % Silwet L-77. photographs taken 12 dpi.

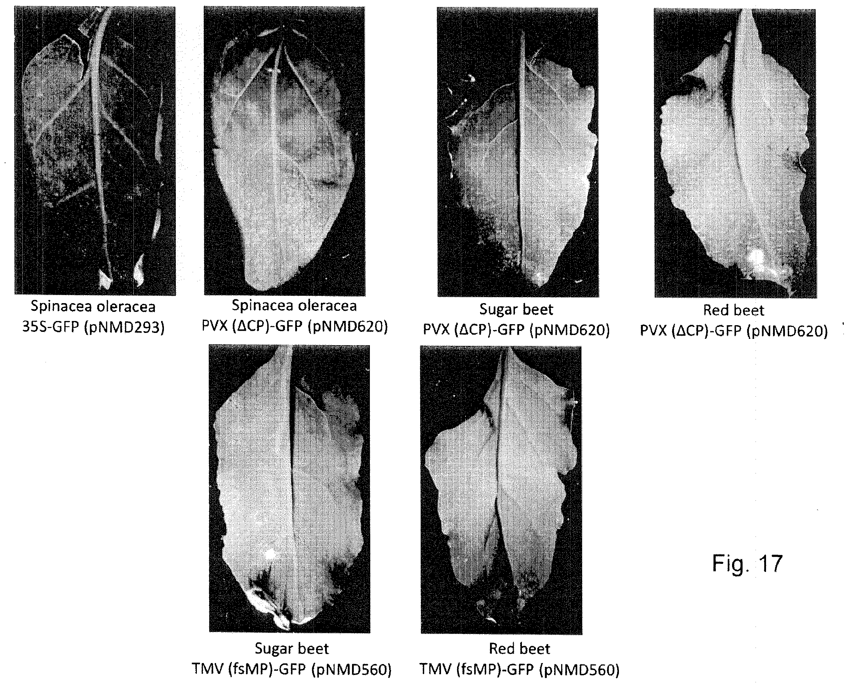

Fig. 17 shows GFP expression after delivery of diluted agrobacteria to spinach and beet plants by dipping with surfactant. A transcriptional vector as well as TMV- and PVX-based viral vectors without cell-to-cell movement ability were used. Agrobacterial cultures were grown to OD600=1.5 and diluted 1:100; 0.1 % Silwet L-77, dipping for 10 sec, photographs taken 12 dpi.

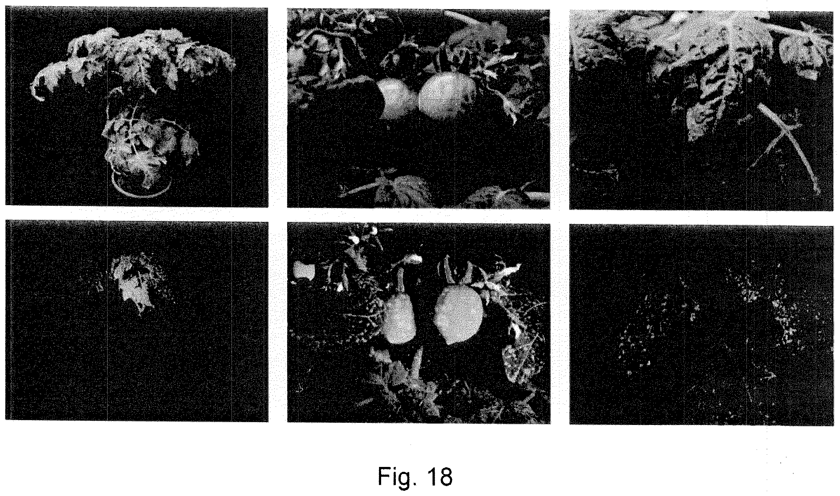

Fig. 18 shows GFP expression after delivery of diluted agrobacteria to tomato *Lycopersicon esculentum* plants by spraying in the presence of surfactant. The vector used was PVX(CP)-GFP (pNMD630). Dilution factor of agrobacterial culture (OD600=1.5): 10⁻², 0.1% Silwet L-77, 200 µM acetosyringone; photographs taken 14 dpi.

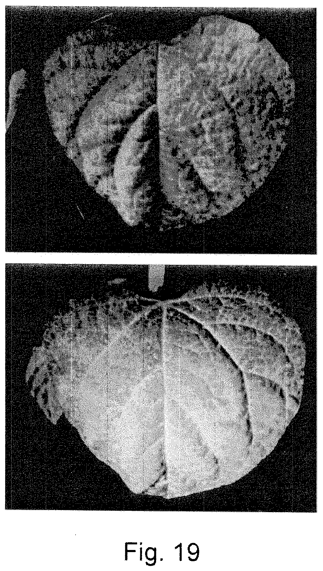

Fig. 19 shows GFP expression after delivery of diluted agrobacteria to Inca berry *Physalis peruviana* plants by spraying with surfactant. Vector PVX(CP)-GFP (pNMD630). Dilution of agrobacterial culture (OD600=1.5): 10⁻², 0.1% Silwet L-77, 200 µM acetosyringone; photographs taken 14 dpi.

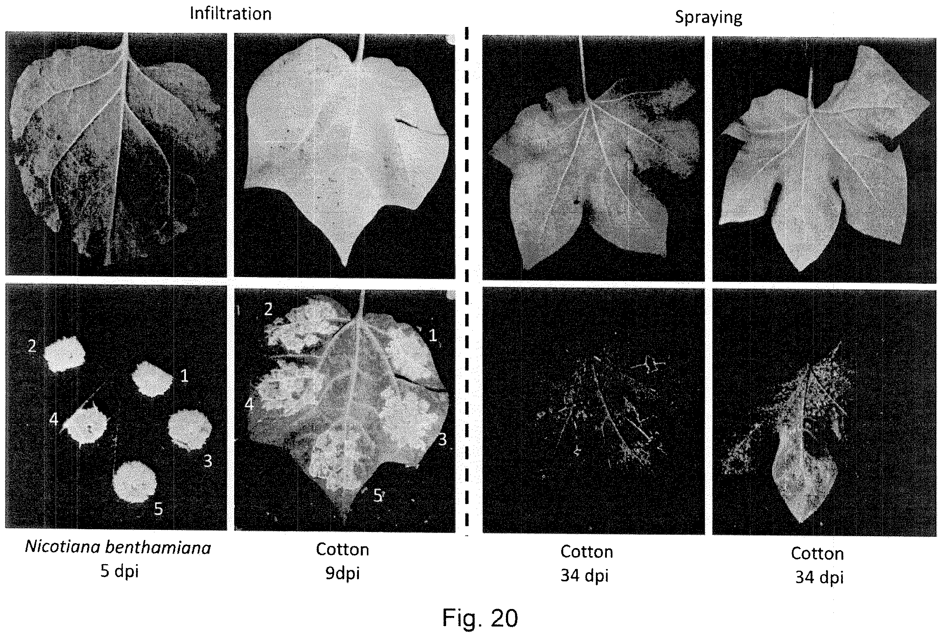

Fig. 20 shows a comparison between transfection by infiltration using a syringe and spraying. Numerals indicate the *Agrobacterium* strain/vector used. Delivery of diluted agrobacteria to cotton *Gossypium hirsutum* L. leaves by using syringe infiltration and spraying with agrobacterial suspensions. Overnight agrobacterial cultures (ICF320 strain) were grown to OD600=1.7-2.0, diluted by a factor of 10⁻² with buffer for infiltration, and incubated with 200µM acetosyringone for 2 hours before use. For spraying, agrobacterial suspensions were additionally supplemented with 0.1 % Silwet L-77.
   Infiltration: 1 - TMV(fsMP)-GFP (pNMD570); 2 - TMV(MP)-GFP (pNMD560); 3 - PVX(ΔCP)-GFP (pNMD620); 4 - PVX(CP)-GFP (pNMD630); 5 - 35S-GFP + P19 (pNMD293).
   Spraying: TMV(MP)-GFP (pNMD560).
   *Nicotiana benthamiana* plant was used as a positive control.

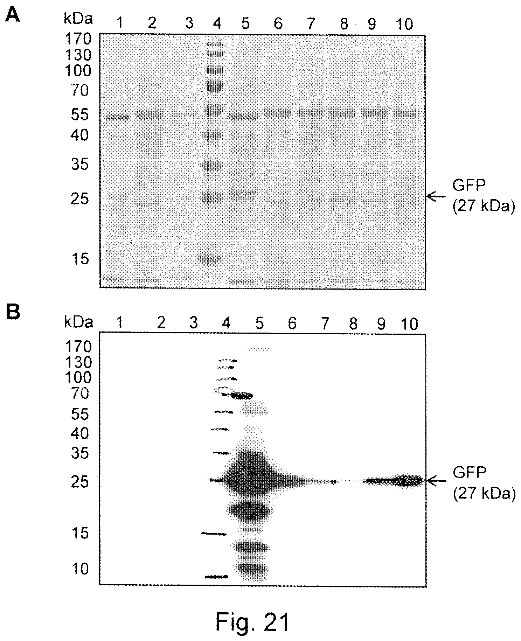

Fig. 21 Shows GFP expression in leaves of cotton *Gossipium* hirsutum L. infiltrated with suspension of agrobacteria carrying transcriptional and viral vectors.
   A) SDS-PAGE with Coomassie staining; B) Western blot probed with anti-GFP antibody (1:3000), second Antibody: anti mouse-HRP (1:5000). The lanes are as follows:
      1- N.benthamiana uninfected leaf;
      2- cotton uninfected leaf;
      3- red cabbage uninfected leaf;
      4- Protein ladder (Fermentas, #SM0671);
      5- TMV(fsMP)-GFP (pNMD570) in Nicotiana benthamiana;
      6- TMV(fsMP)-GFP (pNMD570) in cotton;
      7- TMV(MP)-GFP (pNMD560) in cotton;
      8- PVX(ΔCP)-GFP (pNMD620) in cotton;
      9- PVX(CP)-GFP (pNMD630) in cotton;
      10- 35S9-GFP+P19 (pNMD293) in cotton.
      100 mg of leaf material were boiled in 600 µl of 1 x Laemmli buffer containing beta-mercaptoethanol; 2.5 µl aliquots were loaded on the gel.
Fig. 22 shows GFP expression after delivery of agrobacteria to *Beta vulgaris vulgaris L.* leaves using spraying with surfactant; influence of acetosyringone and abrasive. Vector: PVX(CP)-GFP (pNMD630). Agrobacterial cells were incubated with 200 µM acetosyringone for 2 hours before spraying. For abrasive treatment, 0.3% carborundum (silicon carbide mixture of F800, F1000 and F1200 particles, Mineraliengrosshandel Hausen GmbH, Telfs, Austria) was added to agrobacterial suspension. Dilution factor of agrobacteria of OD600=1.4: 10⁻². GFP-expressing spots were leaves are indicated on the right.
Fig. 23 shows GFP expression after delivery of diluted agrobacteria to plants of different species by spraying with surfactant and abrasive. Vectors: TMV(MP)-GFP (pNM600) and PVX(CP)-GFP (pNMD630), dilution factor of agrobacterial culture (OD600=1.5): 10⁻², 0.1 % Silwet L-77, 0.3 % silicon carbide.
Fig. 24 shows expression after triple subsequent treatments of *Nicotiana benthamina* plants with agrobacteria harbouring viral vectors. Dipping of leaves was performed with 7-days interval in the order:
   A) PVX(ΔCP)-GFP, PVX (CP)-dsRED, TMV(MP)-GFP;
   B) TMV(fsMP)-GFP, PVX(CP)-dsRED, PVX(CP)-GFP;
   C) PVX(ΔCP)-GFP, TMV(MP)-dsRED, PVX(CP)-GFP;
   D) TMV(fsMP)-GFP, PVX(CP)-dsRED, TMV(MP)-GFP;
   E) TMV(fsMP)-GFP, TMV(MP)-dsRED, TMV(MP)-GFP;
   F) PVX(ΔCP)-GFP, TMV(MP)-dsRED, TMV(MP)-GFP;
   G) TMV(fsMP)-GFP, PVX(CP)-dsRED, TMV(MP)-GFP;
   H) PVX(ΔCP)-GFP, PVX(CP)-dsRED, TMV-GFP.
   Single treatments:
   I) TMV(fsMP)-GFP; J) PVX(ΔCP)-GFP; K) TMV(MP)-GFP; L) PVX(CP)-GFP; M) TMV(MP)-dsRED and N) PVX(CP)-dsRED.

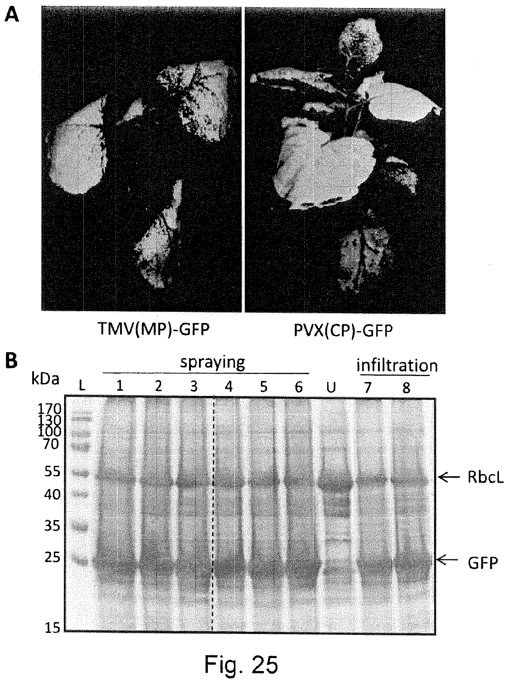

Fig. 25 Analysis of GFP expression in *Nicotiana benthamiana* plants sprayed with agrobacterial suspensions (10⁻² dilution factor) harbouring TMV(MP)-GFP (pNM600) and PVX(CP)-GFP (pNMD630) vectors.
   A) Spraying-transfected *Nicotiana benthamiana* plants, photograph taken 15 dpi.
   B) SDS-PAGE with Coomassie staining; 12% gel, reducing conditions. Leaves from spraying-transfected N.benthamiana plants expressing GFP) were harvested at 15 dpi. Plant material was extracted with 6 volumes of 1 x Laemmli buffer containing beta-mercaptoethanol. After heating at 95 C, 10 µl aliquots were loaded on the gel.
      L - Protein Ladder (Fermentas, #SM0671);
      1 - TMV(MP)-GFP, plant 1;
      2 - TMV(MP)-GFP, plant 2;
      3 - TMV(MP)-GFP, plant3;
      4 - PVX(CP)-GFP, plant 1;
      5 - PVX(CP)-GFP, plant 2;
      6 - PVX(CP)-GFP, plant3;
      U - uninfected *N. benthamiana* leaf tissue;
      7 - TMV(MP)-GFP, vacuum-infiltrated plant;
      8 - PVX(CP)-GFP, vacuum-infiltrated plant.
      RbcL-RUBISCO large subunit.

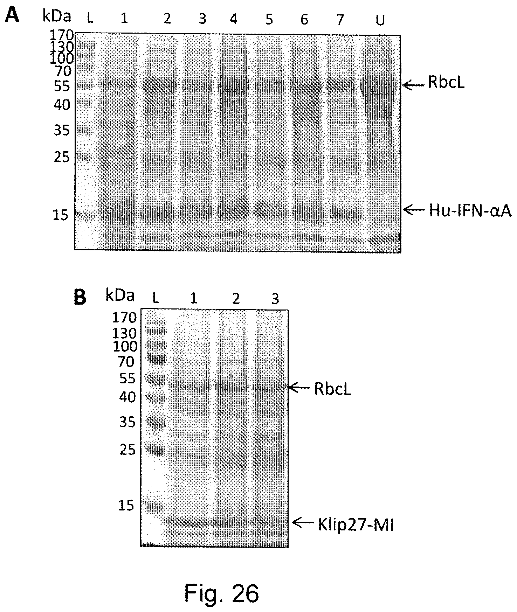

Fig. 26 SDS-PAGE with Coomassie staining for analysis of human alpha-a interferon (Hu-IFN-αA) and Klip27-Mini-Insulin expressed in *Nicotiana benthamiana* plants sprayed with agobacterial suspensions of TMV-based viral vectors capable for cell-to-cell movement.
   A) Hu-IFN-αA: pNMD38 and pNMD45 vectors, 10-2 dilution factor of agrobacterial culture, harvesting at 12 dpi.
      L - Protein Ladder (Fermentas, #SM0671);
      1 - pNMD38, syringe infiltration;
      2 - pNMD38, spraying, leaf1;
      3 - pNMD38, spraying, leaf2;
      4 - pNMD38, spraying, leaf3;
      5 - pNMD45, spraying, leaf1;
      6 - pNMD45, spraying, leaf2;
      7 - pNMD45, spraying, leaf3;
      U - uninfected N.benthamiana leaf tissue.
   B) Klip27-Mini-Insulin: pNMD331 vector, 10⁻² and 10⁻³ dilution factors of agrobacterial culture, harvesting at 12 dpi.
      L- Protein Ladder (Fermentas, #SM0671);
      1 - pNMD331, syringe infiltration, dilution 10⁻³;
      2 - pNMD331, spraying, dilution 10⁻³;
      3 - pNMD331, syringe infiltration, dilution 10⁻³.
         Plant material was extracted with 6 volumes of 1 x Laemmli buffer containing beta-mercaptoethanol. 10 µl aliquots were separated in 15% polyacrylamide gel under reducing conditions.

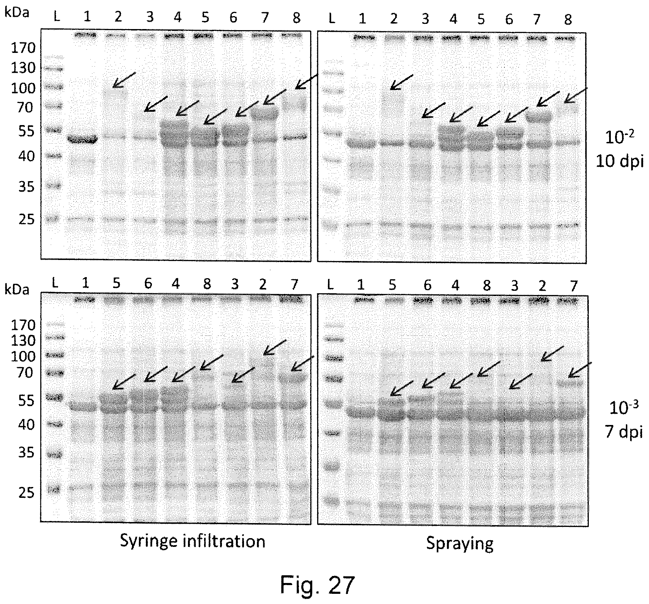

Fig. 27 Expression of cellulases in *N. benthamiana* plants achieved by spraying of diluted agrobacterial cultures harbouring TMV vectors capable for cell-to-cell movement (7 and 10 dpi). *N. benthamiana* plants were inoculated with 10⁻² (top panel) and 10⁻³ (bottom panel) dilutions of *Agrobacterium* cultures (OD600=1.3) either by needleless syringe or spraying with 0.1% Silwet L-77. Protein levels of cellulase fusions were analyzed in crude extracts using SDS-PAGE with Coomassie staining.
   For crude extracts, 50 mg plant material (pooled samples of 3 independant leaves) harvested 10 dpi was ground in liquid nitrogen, extracted with 5 vol. 2xLaemmli buffer and denatured at 95°c for 5 min. 7.5 µl of each sample were analyzed by 10% SDS-PAGE and Coomassie staining.
   L - Protein Ladder (Fermentas, #SM0671);
   1 - uninfected *N. benthamiana* leaf tissue;
   2 - exocellulase E3 from *Thermobifida fusca* targeted to apoplast;
   3 - exoglucanase 1 (CBH I) from *Trichoderma reesei* targeted to apoplast;
   4 - b-glucosidase BGL4 from *Humicola grisea* targeted to chloroplasts;
   5 - b-glucosidase BGL4 from *Humicola grisea* expressed in cytosol;
   6 - His-tagged b-glucosidase BGL4 from *Humicola grisea*;
   7 - exocellulase E3 from *Thermobifida fusca* targeted to chloroplasts;
   8 - endoglucanase E1 from *Acidothermus cellulolyticus* targeted to apoplast.

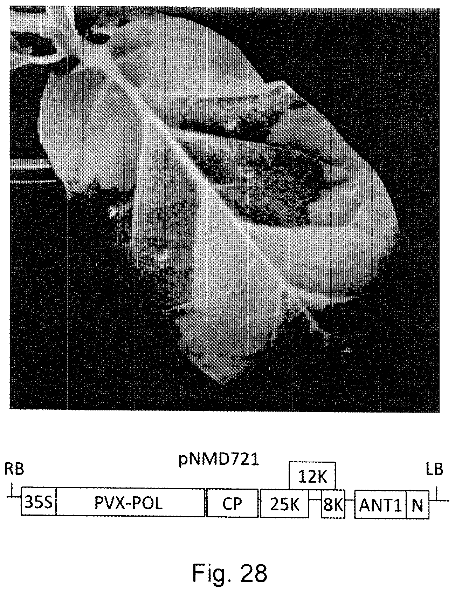

Fig. 28 Induction of anthocyanin biosynthesis in *Nicotiana tabacum* leaves via transient expression by infiltration of MYB transcription factor anthocyanin 1 (ANT1) from *Lycopersicon esculentum* (AAQ55181). 7 dpi, *Agrobacterium* culture (OD600=1.4) dilution factor 10⁻².

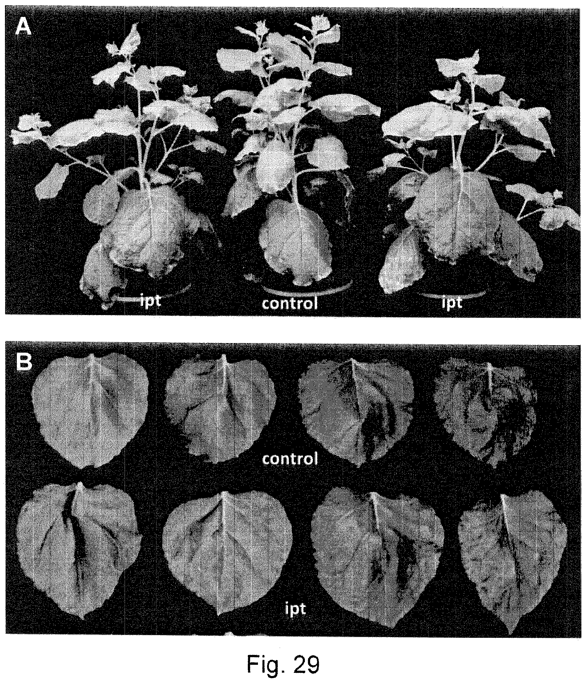

Fig. 29 Morphological changes in *Nicotiana benthamiana* plants caused by transient expression of isopentenyl transferase (ipt) gene delivered by spraying with diluted agrobacteria harbouring transcriptional vector containing ipt coding sequence under the control of 35S promoter. Agrobacterium culture (OD600=1.4) was diluted by a factor of 10⁻² and supplemented with 0.1% Silwet L-77; photograph taken 45 dpi.
   A) Habitus of transfected plants. ipt - Plants sprayed with diluted agrobacterial culture (pNMD460 construct); control - plant sprayed with buffer for transfection containing no agrobacteria.
   B) Leaves of transfected plants. Top: leaves of plants sprayed with buffer for transfection containing no agrobacteria. Bottom: leaves of plants sprayed with diluted agrobacterial culture (pNMD460 construct).

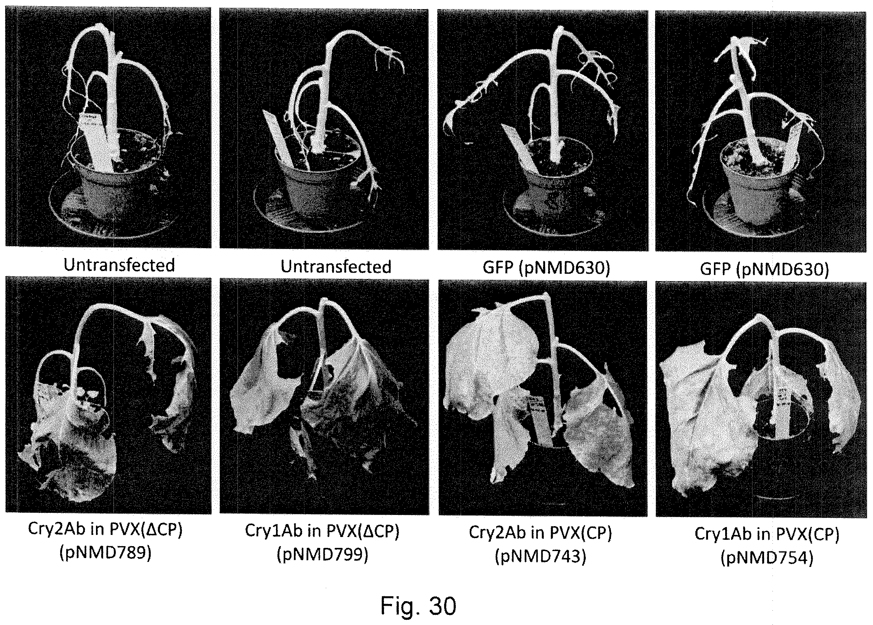

Fig. 30 Transient expression of *Bacillus thuringiensis* endotoxins after the spraying with diluted agrobacterial cultures harbouring corresponding PVX-based expression vectors protects *Nicotiana benthamiana* plants from feeding damage by larvae of the tobacco hornworm *Manduca sexta.* Plants were sprayed with agrobacterium cultures (OD600=1.4-1.7) diluted by factor 10⁻² and supplemented with 0.1% Silwet L-77. Two weeks later, 3 larvae of the 3rd instar were fed on each plant. Pictures were taken in 2 weeks after the beginning of feeding.

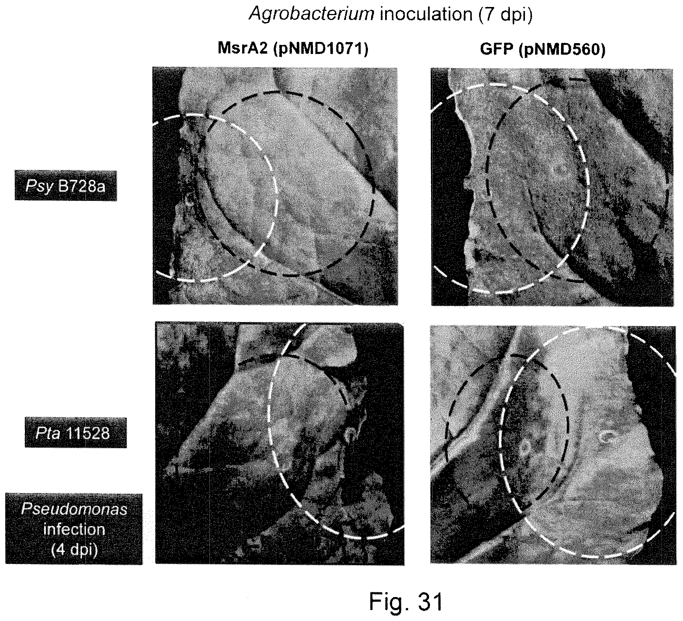

Fig. 31 Phenotypes of *N.benthamiana* transiently expressing defensin MsrA2 and GFP via TMV-based vectors with cell-to-cell movement ability (pNMD1071 and pNMD560, respectively). Inoculation with *Pseudomonas* was performed at 3 days post inoculation with Agrobacterium. Pictures were taken at 4 days post inoculation with *Pseudomonas.* Inoculation of leaves with both *Agrobacterium* and *Pseudomonas* was
   performed using needleless syringe.

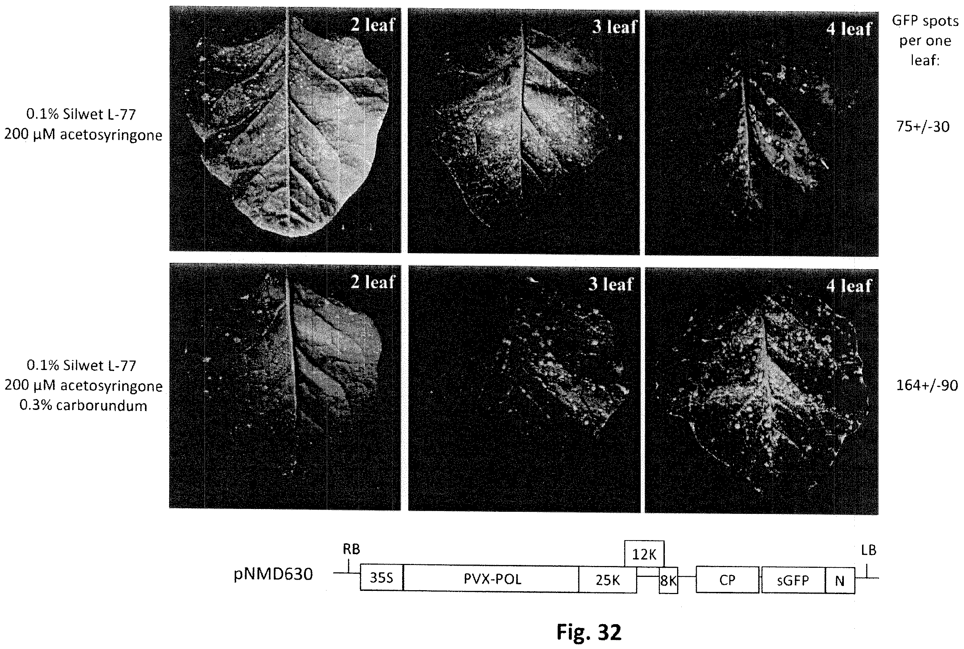

Fig. 32 shows GFP expression after delivery of agrobacteria to eggplant *Solanum melongena* L. leaves using spraying with surfactant (0.1% Silwet L-77); influence of abrasive. Vector: PVX(CP)-GFP (pNMD630). Agrobacterial cells (ICF320 strain) were incubated with 200 µM acetosyringone for 2 hours before spraying. For abrasive treatment, 0.3% carborundum (silicon carbide mixture of F800, F1000 and F1200 particles, Mineraliengrosshandel Hausen GmbH, Telfs, Austria) was added to agrobacterial suspension. Dilution factor of agrobacteria of OD600=1.3: 10⁻². Pictures were taken at 19 dpi. The number of GFP-expressing spots is given on the right.

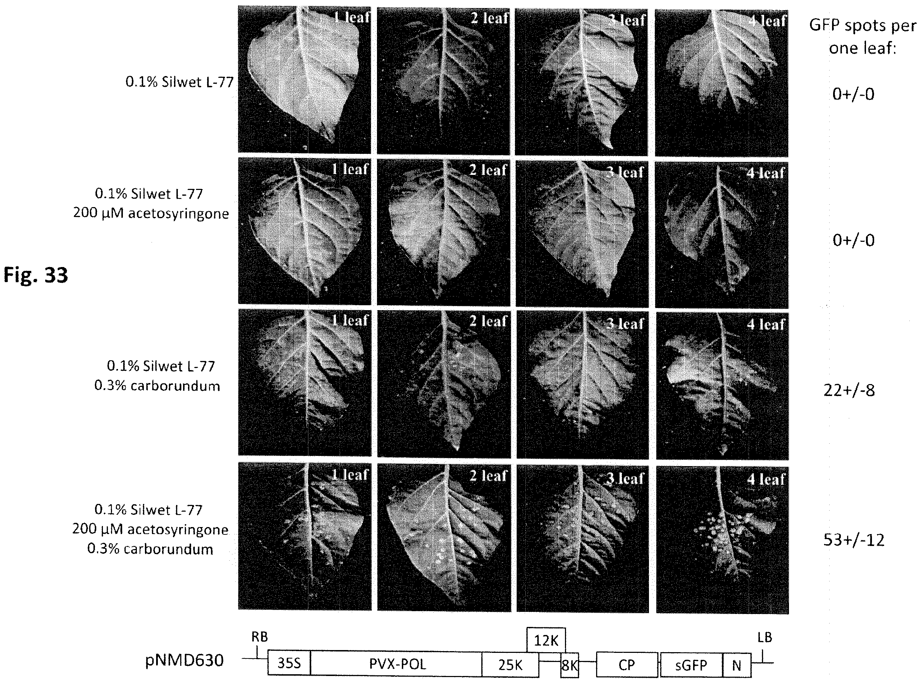

Fig. 33 shows GFP expression after delivery of agrobacteria to pepper *Capsicum annuum* L. cv Feher Gelb leaves using spraying with surfactant (0.1% Silwet L-77); synergistic action of acetosyringone and abrasive. Vector: PVX(CP)-GFP (pNMD630). Agrobacterial cells (ICF320 strain) were incubated with 200 µM acetosyringone for 2 hours before spraying. For abrasive treatment, 0.3% carborundum (silicon carbide mixture of F800, F1000 and F1200 particles, Mineraliengrosshandel Hausen GmbH, Telfs, Austria) was added to agrobacterial suspension. Dilution factor of agrobacteria of OD600=1.4: 10⁻². Pictures were taken at 18 dpi. The number of GFP-expressing spots is given on the right.

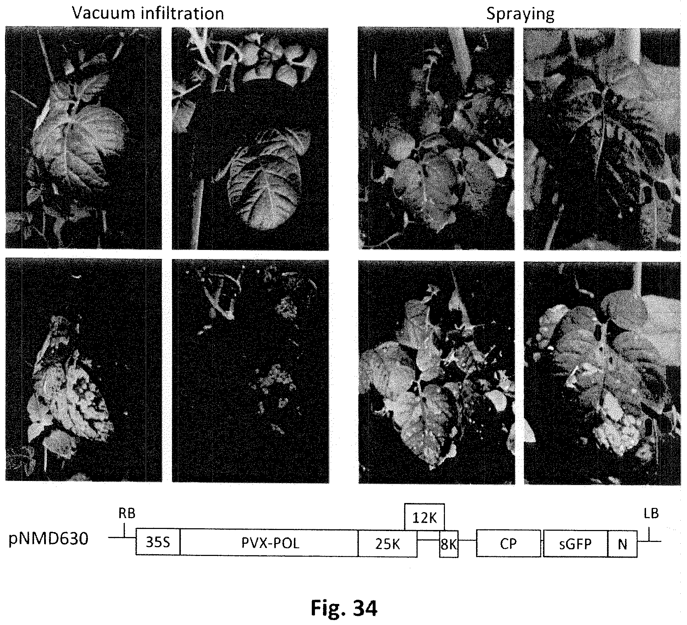

Fig. 34 depicts GFP expression after delivery of agrobacteria to potato *Solanum tuberosum* L. cv Mirage leaves using vacuum infiltration and spraying with surfactant (0.1% Silwet L-77); comparison of vacuum infiltration and spraying. Vector: PVX(CP)-GFP (pNMD630). Agrobacterial cells (ICF320 strain) were incubated with 200 µM acetosyringone for 2 hours before spraying. Dilution factor of agrobacteria of OD600=1.5: 10⁻². Pictures were taken at 14 dpi.

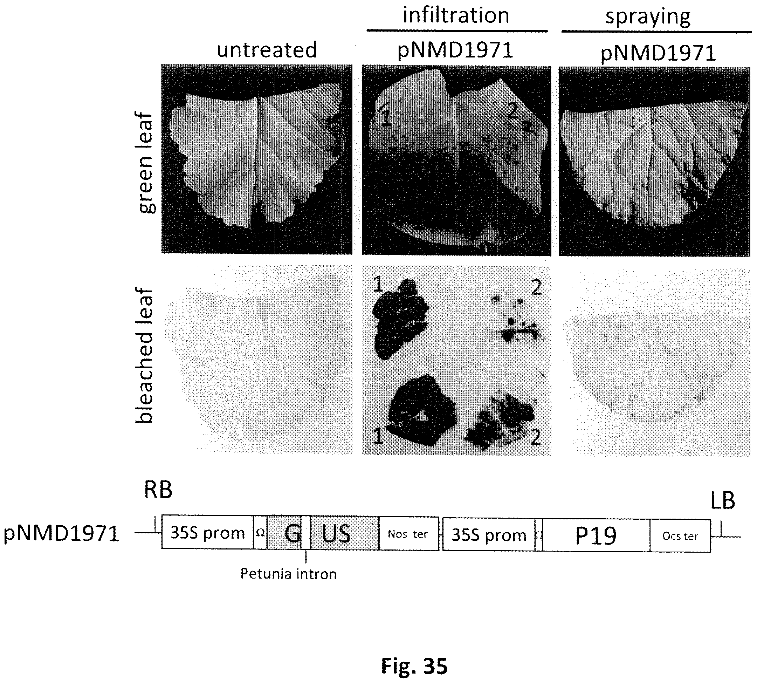

Fig. 35 shows GUS expression after delivery of agrobacteria to rapeseed *Brassica napus* L. leaves using syringe infiltration and spraying with surfactant (0.1% Silwet L-77). Vector: 35S-GUS+35S-P19 (pNMD1971). Agrobacterial cells (EHA105 strain) were incubated with 200 µM acetosyringone for 2 hours before spraying. Dilution factor of agrobacteria of OD600=1.3: 10⁻¹ and 10⁻². Syringe infiltration: 1: 10⁻¹ dilution of agrobacterial culture; 2: 10⁻² dilution of agrobacterial culture. For spraying, 10⁻¹ dilution of agrobacterial culture was used. Pictures for infiltrated and for sprayed leaves were taken at 5 and 13 dpi, respectively.

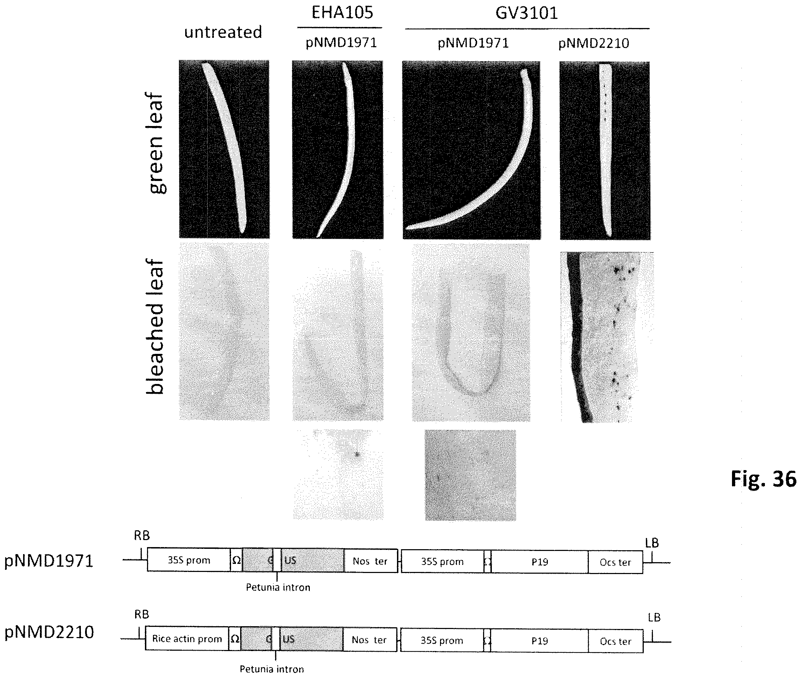

Fig. 36 depicts GUS expression after delivery of agrobacteria to onion *Allium cepa* cv Stuttgarter Riesen leaves sprayed with agrobacteria using surfactant (0.1% Silwet L-77). Agrobacterial cells (EHA105 and GV3101 strains) were incubated with 200 µM acetosyringone for 2 hours before spraying. Vectors: 35S-GUS+35S-P19 (pNMD1971) and rice actin promoter-GUS+35S-P19 (pNMD2210). Dilution factor of agrobacteria of OD600=1.3: 10⁻¹. Pictures were taken at 11 dpi.

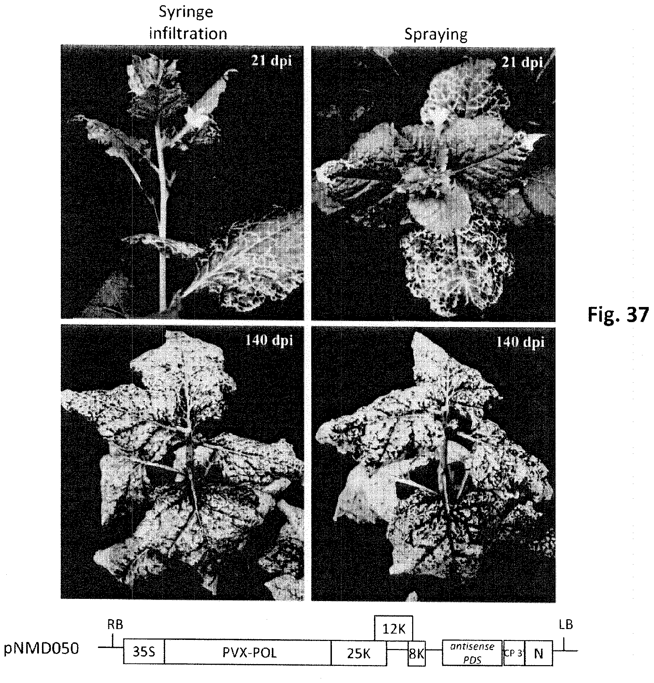

Fig. 37 shows the photobleaching by gene silencing of phytoene desaturase (PDS) in *Nicotiana benthamina* leaves after the agrobacterium-mediated delivery of PVX constructs carrying the fragment of PDS coding sequence in an anti-sense orientation; comparison of syringe infiltration and spraying with surfactant (0.1% Silwet L-77). Vector: PVX(CP)-antiPDS (pNMD050); *Agrobacterium tumefaciens* strain GV3101. Dilution factor of agrobacteria of OD600=1.5: 10⁻². Pictures were taken at 21 dpi and 140 dpi.

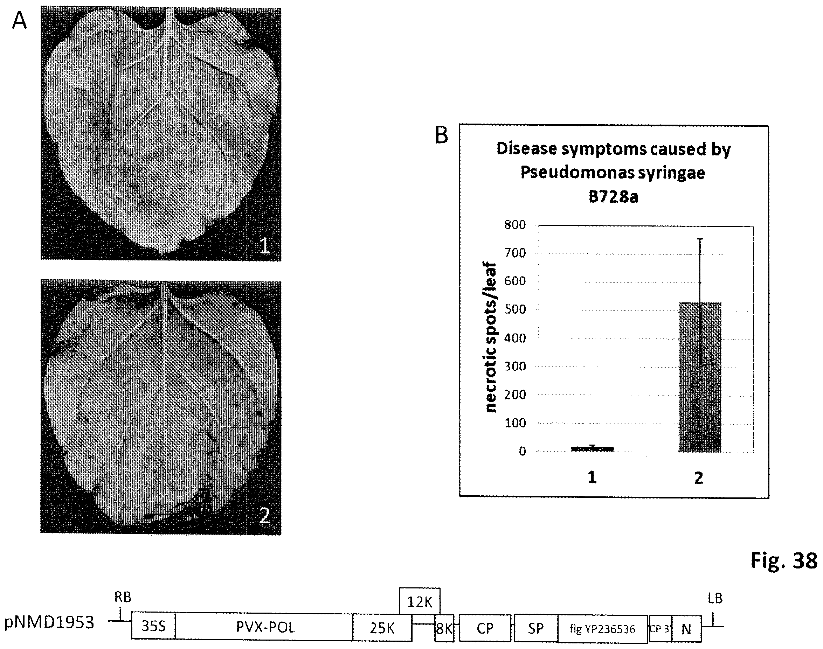

Fig. 38 shows effect of transient flagellin expression on infection of *Nicotiana benthamiana* by *Pseudomonas.* A) Leaves of *Nicotiana benthamiana* plants infected with *Pseudomonas syringae* pv. *syringae B728a.* B) Disease symptoms counted as a number of necrotic lesions (see as dark spots) per one leaf. 1- plant preliminary sprayed with agrobacterial suspension (ICF320 strain) bearing PVX(CP) vector providing the expression of translational fusion of full-length flagellin (YP236536) from *Pseudomonas syringae* pv. *syringae B728a* with barley a-amylase apoplast signal peptide (pNMD1953); 2 - plant preliminary sprayed with agrobacterial cells (ICF320 strain) without any T-DNA-containing vector.

### DETAILED DESCRIPTION OF THE INVENTION

In the invention, agrobacteria are used for transfecting plants with a sequence or construct of interest by spraying with aqueous suspensions containing cells of an *Agrobacterium* strain. The *Agrobacterium* strain may belong to the species *Agrobacterium tumefaciens* or *Agrobacterium rhizogenes* that are commonly used for plant transformation and transfection and which is known to the skilled person from general knowledge. The *Agrobacterium* strain comprises a DNA molecule comprising a nucleic constructs containing a DNA sequence of interest. The DNA sequence of interest encodes a protein or an RNA to be expressed in plants. The nucleic construct is typically present in T-DNA of Ti-plasmids for introduction of the nucleic construct into plant cells by the secretory system of the *Agrobacterium* strain. On at least one side or on both sides, the nucleic acid construct is flanked by a T-DNA border sequence for allowing transfection of said plant(s) and introduction of cells of said plant with said DNA sequence of interest. In the nucleic acid construct, the DNA sequence of interest is present such as to be expressible in plant cells. For this purpose, the DNA sequence of interest is, in said nucleic acid construct, typically under the control of a promoter active in plant cells. Examples of the DNA sequence of interest are a DNA sequence encoding a DNA viral replicon or an RNA viral replicon or a gene to be expressed. The gene may encode an RNA of interest or a protein of interest to be expressed in cells of the plant(s). Also the viral replicons typically encode an RNA or a protein of interest to be expressed in plants. The DNA construct may comprise, in addition to the DNA sequence of interest, other sequences such as regulatory sequences for expression of the DNA sequence of interest. *Agrobacterium*-mediated gene transfer and vectors therefor are known to the skilled person, e.g. from the references cited in the introduction or from text books on plant biotechnology such as Slater, Scott and Fowler, Plant Biotechnology, second edition, Oxford University Press, 2008.

In embodiments wherein strong expression of a protein or RNA is desired or wherein accumulation of viral nucleic acids to high amounts in plant cells and possible negative effects on plant health is not a concern, the nucleic acid construct may encode a replicating viral vector that can replicate in plant cells. In order to be replicating, the viral vector contains an origin of replication that can be recognized by a nucleic acid polymerase present in plant cells, such as by the viral polymerase expressed from the replicon. In case of RNA viral vectors, the viral replicons may be formed by transcription, under the control of a plant promoter, from the DNA construct after the latter has been introduced into plant cell nucleic. In case of DNA viral replicons, the viral replicons may be formed by recombination between two recombination sites flanking the sequence encoding the viral relicon in the DNA construct, e.g. as described in WO00/17365 and WO 99/22003. If viral replicons are encoded by the DNA construct, RNA viral replicons are preferred. Use of DNA and RNA viral replicons has been extensively described in the literature at least over the last 15 years. Some examples are the following patent publications by Icon Genetics: WO2008028661, WO2007137788, WO 2006003018, WO2005071090, WO2005049839, WO02097080, WO02088369, WO02068664. An example of DNA viral vectors are those based on geminiviruses. For the present invention, viral vectors or replicons based on plant RNA viruses, notably based on plus-sense single-stranded RNA viruses are preferred. Examples of such viral vectors are tobacco mosaic virus (TMV) and potex virus X (PVX) used in the examples. Potexvirus-based viral vectors and expression systems are described in EP2061890. Many other plant viral replicons are described in the patent publications mentioned above.

The aqueous suspension used for spraying in the processes of the invention may have a concentration of *Agrobacterium* cells of at most 1.1·10⁹ cfu/ml, which corresponds approximately to an *Agrobacterium* culture in LB-medium of an optical density at 600 nm of 1. Due to the high transfection efficiency achieved in the invention, much lower concentrations may, however, be used, which allows treatment of many plants such as entire farm fields without the need for huge fermenters for *Agrobacterium* production. Thus, the concentration is preferably at most 2.2·10⁷ cfu/ml, more preferably at most 1.1·10⁷ cfu/ml, more preferably at most 4.4·10⁶ cfu/ml. In one embodiment, the concentration is at most 1.1·10⁶ cfu/ml of the suspension. For avoiding determination of cell concentrations in terms of cfu/ml, concentrations of agrobacterial suspensions are frequently assessed by measuring the apparent optical density at 600 nm using a spectrophotometer. Herein, the concentration of 1.1·10⁷ cfu/ml corresponds to a calculated optical density at 600 nm of 0.01, whereby the calculated optical density is defined by a 100-fold dilution with water or buffer of a suspension having an optical density of 1.0 at 600 nm. Similarly, the concentrations of 4.4·10⁶ cfu/ml and 1.1·10⁶ cfu/ml correspond to a calculated optical density at 600 nm of 0.004 and 0.001, respectively, whereby the calculated optical densities are defined by a 250-fold or 1000-fold, respectively, dilution with water or buffer of a suspension having an optical density of 1.0 at 600 nm.

The abrasive used in the invention is a particulate material that is essentially insoluble in the aqueous suspension of *Agrobacterium* cells. The abrasive is believed to weaken, notably if used together with a wetting agent, the surface of plant tissue such as leaves, and thereby facilitates penetration of *Agrobacterium* cells into the intercellular space of plant tissue. As a result, the transfection efficiency increases.

The particulate material to be used as the abrasive of the invention may be carrier material as commonly used as carriers in wettable powder (WP) of pesticide formulations. In the context of wettable powders, these carriers are also referred to in the field of pesticide formulations as "fillers" or "inert fillers". Wettable powder formulations are part of the general knowledge in the field of plant protection. Reference is made to the handbook PESTICIDE SPECIFICATIONS, "Manual for Development and Use of FAO and WHO Specifications for Pesticides", edited by the World Health Organisation (WHO) and the FOOD and Agriculture Organization of the United States, Rome, 2002, ISBN 92-5-104857-6. Wettable powder formulations for plant protection are for example described in EP 1810569, EP1488697, EP1908348 and EP0789510. The abrasive may be a mineral material, typically an inorganic material. Examples of such carrier materials are diatomaceous earth, talc, clay, calcium carbonate, bentonite, acid clay, attapulgite, zeolite, sericite, sepiolite or calcium silicate. It is also possible to use quartz powder such as the highly pure quartz powder described in WO02/087324. Preferred examples are silica, such as precipitated and fumed hydrophilic silica, and carborundum. The abrasive properties of diluents or fillers such as silica used in wettable powders are known (see "Pesticide Application Methods" by G.A. Matthews, third edition, Blackwell Science, 2000, on page 52 thereof).

As commercial products of particulate inorganic materials for use as abrasives in the invention, the hydrophilic silica Sipernat™ 22S and Sipernat™ 50 S, manufactured by Evonic Degussa may be mentioned. Other products are "Hi-Sil™ 257", a synthetic, amorphous, hydrated silica produced by PPG Industries Taiwan Ltd. or "Hubersorb 600™", a synthetic calcium silicate, manufactured by Huber Corporation. A commercial sub-micron sized silica is Hi-Sil™ 233 (PPG Industries) having an average particle size of around 0.02 µm.

The abrasive may have a median particle size between 0.01 and 40, preferably between 0.015 and 30, more preferably between 0.05 and 30, even more preferably between 0.1 and 30, even more preferably between 0.1 and 20, even more preferably between 0.5 and 20, and most preferably between 1.0 and 16 µm. In one embodiment, the median particle size is between 0.015 and 1 or between 0.02 and 0.5 µm. The median particle size is the volume median particle size that can be measured by laser diffraction using a Mastersizer™ from Malvern Instruments, Ltd. In order to avoid clogging of spraying nozzles, the maximum particle size of the largest particles contained in the abrasive should be at most 45 µm, preferably at most 40 µm, which may be determined by sieving. This condition is considered fulfilled, if the sieve residue is below 1.5 % by weight (following ISO 3262-19). The abrasive may have a D90 value of at most 40 µm, preferably of at most 30 µm, measured by laser diffraction as described above. Typically, the particle sizes above relate to primary particle sizes.

The content of the abrasive in the aqueous suspension of the invention may be between 0.01 and 3, preferably between 0.02 and 2, more preferably between 0.05 and 1 and even more preferably between 0.1 and 0.5 % by weight of said suspension.

The aqueous suspension used in the invention contains an agricultural spray adjuvant. The spray adjuvant is a surfactant. The surfactant has multiple advantages in the present invention. It reduces the surface tension of the water of the aqueous suspension and makes the waxy surface of plant leaves more permeable for agrobacteria. It further improves the stability of the suspension and reduces settling of the abrasive in the suspension. Surfactants used in the present invention are non-ionic surfactants and examples of the surfactants include the following (A). These may be used singly or in combination.
(A) Nonionic surfactants: A measurement frequently used to describe surfactants is the HLB (hydrophilic/lipophilic balance). The HLB describes the ability of the surfactant to associate with hydrophilic and lipophilic compounds. Surfactants with a high HLB balance associate better with water soluble compounds than with oil soluble compounds. Herein, the HLB value should be 12 or greater, preferably at least 13. As noninionic surfactants, organo-silicone surfactants such as polyalkyleneoxide-modified heptamethyltrisiloxane are most preferred in the present invention. A commercial product is Silwet L77™ spray adjuvant from GE Advanced Materials.
(A-1) Polyethylene glycol type surfactants: examples of polyethylene glycol type surfactants include polyoxyethylene alkyl (C12-18) ether, ethylene oxide adduct of alkylnaphthol, polyoxyethylene (mono or di) alkyl (C8-12) phenyl ether, formaldehyde condensation product of polyoxyethylene (mono or di) alkyl (C8-12) phenyl ether, polyoxyethylene (mono, di, or tri) phenyl phenyl ether, polyoxyethylene (mono, di, or tri) benzyl phenyl ether, polyoxypropylene (mono, di, or tri) benzyl phenyl ether, polyoxyethylene (mono, di, or tri) styryl phenyl ether, polyoxypropylene (mono, di or tri) styryl phenyl ether, a polymer of polyoxyethylene (mono, di, or tri) styryl phenyl ether, a polyoxyethylene polyoxypropylene block polymer, an alkyl (C12-18) polyoxyethylene polyoxypropylene block polymer ether, an alkyl (C8-12) phenyl polyoxyethylene polyoxypropylene block polymer ether, polyoxyethylene bisphenyl ether, polyoxyethylene resin acid ester, polyoxyethylene fatty acid (C12-18) monoester, polyoxyethylene fatty acid (C12-18) diester, polyoxyethylene sorbitan fatty acid (C12-18) ester, ethylene oxide adduct of glycerol fatty acid ester, ethylene oxide adduct of castor oil, ethylene oxide adduct of hardened caster oil, ethylene oxide adduct of alkyl (C12-8) amine and ethylene oxide adduct of fatty acid (C12-18) amide;
(A-2) Polyvalent alcohol type surfactants: examples of polyvalent alcohol type surfactants include glycerol fatty acid ester, polyglycerin fatty acid ester, pentaerythritol fatty acid ester, sorbitol fatty acid (C12-18) ester, sorbitan fatty acid (C12-8) ester, sucrose fatty acid ester, polyvalent alcohol alkyl ether, and fatty acid alkanol amide;
(A-3) Acetylene-type surfactants: examples of acetylene type surfactants include acetylene glycol, acetylene alcohol, ethylene oxide adduct of acetylene glycol and ethylene oxide adduct of acetylene alcohol.

The above surfactants may be used singly or in combination of two or more surfactants. Notably, the preferred organo-silicone surfactants may be combined with other surfactants. The total concentration of surfactants in the aqueous suspension of the invention may be easily tested by conducting comparative spraying experiments, similarly as done in the examples. However, in general, the total concentration of surfactants may be between 0.025 and 0.5 volume-%, preferably between 0.025 and 0.2 volume-% of said suspension. Since the density of surfactants is generally close to 1.0 g/ml, the total concentration of surfactants is between 0.25 and 5.0 g, preferably between 0.25 and 2.0 g per liter of said suspension (including abrasive). If the above organo-silicone surfactants such as polyalkyleneoxide-modified heptamethyltrisiloxane are used, the concentration of the organo-silicone surfactant in the agrobacterial suspension used for spraying may be between 0.05 and 0.5 volume-%, preferably between 0.05 and 0.2 volume-%. Alternatively, the concentration of the organo-silicone surfactant in the agrobacterial suspension used for spraying may be defined as being between 0.5 and 5.0 g, preferably between 0.5 and 2.0 g per liter of said suspension.

In order to improve the physical properties of the aqueous suspension, it is possible to add highly dispersed sub-micron size silicic acid (silica) or porous polymers such as urea/formaldehyde condensate (Pergopak™). Notably, where the median particle size of the abrasive is between 0.1 and 30 µm, or in one of the preferred sub-ranges of this range given above, it is possible to add a highly dispersed sub-micron size silica to the suspension. Herein, sub-micron size silica is silica having a median particle size between 0.01 and 0.5 µm, preferably between 0.02 and 0.5 µm, more preferably between 0.02 and 0.1 µm. Highly dispersed silicic acid such as Hi-Sil™ 233 (PPG Industries) can contribute to the abrasive properties of the aqueous suspension (see Jensen et al., Bull. Org. mond. Sante, Bull. Wld Hlth Org. 41 (1969) 937-940). These agents may be incorporated in an amount of from 1 to 10 g per liter of the suspension of the invention.

Further possible additives to the agrobacterial suspension are buffer substances to keep maintain the pH of the suspension used for spraying at a desired pH, typically between 7.0 and 7.5. Further, inorganic soluble salts such as sodium chloride by be added to adjust the ionic strength of the suspension. Nutrient broth such as LB medium may also be contained in the suspension.

The aqueous suspension may be produced as follows. In one method, the *Agrobacterium* stain to be used in the process of the invention is inoculated into culture medium and grown to a high cell concentration. Larger cultures may be inoculated with small volumes of a highly concentrated culture medium for obtaining large amounts of the culture medium. Agrobacteria are generally grown up to a cell concentration corresponding to an OD at 600 nm of at least 1, typically of about 1.5. Such highly concentrated agrobacterial suspensions are then diluted to achieve the desired cell concentration. For diluting the highly concentrated agrobacterial suspensions, water is used. The water may contain a buffer. The water may further contain the surfactant of the invention. Alternatively, the concentrated agrobacterial suspensions may be diluted with water, and any additives such as the surfactant and the optional buffer substances are added after or during the dilution process. The abrasive may be added before, during or after dilution. It is however preferred to agitate the suspension during addition of the abrasive to uniformly disperse the abrasive in the agrobacterial suspension. The step of diluting the concentrated agrobacterial suspension may be carried out in the spray tank of the sprayer used for spraying the diluted suspensions.

The sprayer to be used in the process of the invention mainly depends on the number of plants or the area to be sprayed. For one or a small number of plants to be sprayed, pump sprayers as widely used in household and gardening can be used. These may have volumes of the spray tank of between 0.5 and 2 liters. For applications on a medium scale, manually operated hydraulic sprayers such as lever-operated knapsack sprayers or manually operated compression sprayers may be used. However, the high transfection efficiency achieved in the invention has its full potential in the transfection of many plants such as plants growing on a farm field or in a greenhouse. For this purpose, power-operated hydraulic sprayers such as tractor-mounted hydraulic sprayers equipped with spray booms can be used. Aerial application techniques using helicopters or airplanes are also possible for large fields. All these types of sprayers are known in the art and are described for example in the book "Pesticide Application Methods" by G.A. Matthews, third edition, Blackwell Science, 2000. In order to ensure a homogeneous suspension in the spray tanks of the sprayers, small or medium size sprayers may be shaken at regular intervals or continuously during spraying. Large sprayers such as the tractor-mounted sprayers should be equipped with an agitator in the spray tank.

Considering the presence of agrobacterial cells and abrasive in the suspensions to be sprayed, sprayers used in the invention should produce spray of a droplet size at least of fine spray. Also, medium spray or coarse spray in the classification of sprays used in the above-mentioned book by G.A. Matthews, page 74, may be used. The main purpose of the spraying in the invention is wetting of plant tissue with the suspension. Thus, the exact droplet size is not critical. However, the transfection efficiency may be further improved by providing the spray to plant surfaces with increased pressure.

In the process of the invention, at least parts of plants are sprayed. In an important embodiment, plants growing in soil on a field are sprayed, i.e. plants not growing in movable pots or containers. Such plants cannot be turned upside down and dipped into agrobacterial suspension for vacuum infiltration. At least parts of plants are sprayed such as leaves. Preferably, most leaves are sprayed or entire plants.

The present invention is used for transient transfection of plants with a DNA sequence of interest. The term "transient" means that the no selection methods are used for selecting cells or plants transfected with the DNA sequence of interest in the background of non-transfected cells or plants using, e.g. selectable agents and selectable marker genes capable of detoxifying the selectable agents. As a result, the transfected DNA is generally not stably introduced into plant chromosomal DNA. Instead, transient methods make use of the effect of transfection in the very plants transfected.

The invention is generally used for transfecting multi-cellular plants, notably, higher plants. Both monocot and dicot plants can be transfected, whereby dicot plants are preferred. Plants for the use in this invention include any plant species with preference given to agronomically and horticulturally important crop species. Common crop plants for the use in present invention include alfalfa, barley, beans, canola, cowpeas, cotton, corn, clover, lotus, lentils, lupine, millet, oats, peas, peanuts, rice, rye, sweet clover, sunflower, sweetpea, soybean, sorghum triticale, yam beans, velvet beans, vetch, wheat, wisteria, and nut plants. The plant species preferred for practicing this invention include, but not restricted to, representatives of Gramineae, Compositeae, Solanaceae and Rosaceae.

Further preferred species for the use in this invention are plants from the following genera: Arabidopsis, Agrostis, Allium, Antirrhinum, Apium, Arachis, Asparagus, Atropa, Avena, Bambusa, Brassica, Bromus, Browaalia, Camellia, Cannabis, Capsicum, Cicer, Chenopodium, Chichorium, Citrus, Coffea, Coix, Cucumis, Curcubita, Cynodon, Dactylis, Datura, Daucus, Digitalis, Dioscorea, Elaeis, Eleusine, Festuca, Fragaria, Geranium, Glycine, Helianthus, Heterocallis, Hevea, Hordeum, Hyoscyamus, Ipomoea, Lactuca, Lens, Lilium, Linum, Lolium, Lotus, Lycopersicon, Majorana, Malus, Mangifera, Manihot, Medicago, Nemesia, Nicotiana, Onobrychis, Oryza, Panicum, Pelargonium, Pennisetum, Petunia, Pisum, Phaseolus, Phleum, Poa, Prunus, Ranunculus, Raphanus, Ribes, Ricinus, Rubus, Saccharum, Salpiglossis, Secale, Senecio, Setaria, Sinapis, Solanum, Sorghum, Stenotaphrum, Theobroma, Trifolium, Trigonella, Triticum, Vicia, Vigna, Vitis, Zea, and the Olyreae, the Pharoideae and others.

In one embodiment, the process of the invention can be used for producing a protein of interest in a plant or in many plants growing on a field. For this purpose, the plants may be sprayed with the agrobacterial suspension at a desired growth state of the plants. If the main aim is to achieve the highest possible expression levels followed by harvesting plants for obtaining plant material containing high amounts of the protein, viral vectors may be used, since they generally give the highest expression levels.

In another embodiment, the process of the invention is used for generating or altering a trait in a plant such as an input trait. In this embodiment, excessive expression of a protein or RNA of interest may not be desired for avoiding deleterious effects on plant health. For such embodiments, non-replicating vectors (also referred to herein as "transcriptional vectors"), i.e. vectors lacking a functional origin of replication recognised by a nucleic acid polymerase present in the plant cells are preferred. An example of such embodiment is the expression of hormonal molecules as secondary messengers in plant cells. In the example of Fig. 29, we demonstrate the delivery of isopenthenyl transferase, key enzyme of cytokinin biosynthesis, into *Nicotiana benthamiana* cells by spraying with diluted agrobacteria carrying a transcriptional vector containing the ipt coding sequence under the control of a 35S promoter. Morphological changes of transfected plants caused by cytokinin overproduction were observed (Fig. 29). Another application of the invention is RNA expression, e.g. for RNA interference, wherein the interference signal can spread in the plant from cells having expressed the signal to other cells. An example is the targeting of undesired viral DNA in plants as described by Pooggin in Nat. Biotech. 21 (2003) 131. An example of oncogene silencing that can be adapted to a transient system is described by Escobar et al. Proc. Natl. Acad. Sci. USA 98 (2001) 13437-13442. Fig. 37 shows photobleaching by gene silencing of phytoene desaturase (PDS) in *Nicotiana benthamina* leaves. A further example is the control of coleopteran insect pests through RNA interference similar as described by Baum et al., Nat. Biotech. 25 (2007) 1322-1326 that can be adapted to the transient process of the invention by transiently transfecting pest-infested plants with DNA of interest encoding and expressing the dsRNA. Further methods applicable to the transient process of the invention are those described by Huang et al., Proc. Natl. Acad. Sci. USA 103 (2006) 14302-14306; Chuang et al., Proc. Natl. Acad. Sci. USA 97 (2000) 4985-4990. In the experiment the results of which are shown in Fig. 38, flagellin expression protects a plant from disease symptoms caused by *Pseudomonas syringai.*

Further, the process of the invention allows altering at a desired point in time traits relating to the regulation of flowering time or fruit formation such as tuborisation in potato (Martinez-Garcia et al., Proc. Natl. Acad. Sci. USA 99 (2002) 15211-15216) or the regulation of the flavonoid pathway using a transcription factor (Deluc et al., Plant Physiol. 147 (2008) 2041-2053). Flowering may be induced by transiently expressing the movable florigen protein FT (Zeevaart, Current Opinion in Plant Biology 11 (2008) 541-547; Corbesier et al., Science 316 (2007) 1030-1033). Parthenocarpic fruits in tomatoes may by produced on a large scale using the invention and the method described by Pandolfini et al., BMC Biotechnology 2 (2002). Further applications of the invention are in the context of altering cotton fiber development by way of MYB transcription factors as described by Lee et al., Annals of Botany 100 (2007) 1391-1401 or activation of plant defensive genes (Bergey et al., Proc. Natl. Acad. Sci. USA 93 (1996) 12053-12058. We have demonstrated that transient expression of defensin MsrA2 in *Nicotiana benthamiana* leaves significantly decreases the Pseudomonas infection symptoms (Fig. 31).

The invention also provides a process of protecting crop plants on a field from a pest. In such process, infestation of at least one of the plants from a plurality of plants growing in a lot or farm field may be determined. Due to the rapidness of the process of the invention expression of a protein or RNA detrimental to the pest needs to be caused only if infestation by the pest is determined. Thus, strong and constitutive expression of pest toxins or dsRNA for RNAi even in the absence of a risk of infestation is not necessary. Transient expression of *Bacillus thuringiensis* endotoxins after the spraying with diluted agrobacterial cultures harbouring corresponding PVX-based expression vectors protected *Nicotiana benthamiana* plants from feeding damage by larvae of the tobacco hornworm Manduca sexta (Fig. 30).

### EXAMPLES

The invention is further described in the following by way of examples. The invention is however not limited to these examples. Examples 1, 2, and 5 to 9 are reference examples.

### Reference Example 1: Determination of Agrobacterium cell concentration in liquid culture in terms of colony forming units (cfu)

The concentration of *Agrobacterium* cells in liquid suspension in terms of colony forming units per ml (cfu/ml) of liquid suspensions can be determined using the following protocol. Cells of *Agrobacterium tumefaciens* strain ICF 320 transformed with construct pNMD620 were grown in 7.5 ml of liquid LBS medium containing 25 mg/L kanamycin (AppliChem, A1493) and 50 mg/L rifampicin (Carl Roth, 4163.2). The bacterial culture was incubated at 28°C with continuous shaking. Absorbance or optical density of bacterial culture expressed in absorbance units (AU) was monitored in 1-ml aliquots of the culture using a spectrophotometer at 600 nm wavelength (OD600). The cell concentration estimated as a number of colony-forming units per milliliter of liquid culture (cfu/ml) can be analyzed at OD600 values 1; 1.3; 1.5; 1.7 and 1.8. For this purpose 250-µl aliquots of liquid culture were diluted with LBS-medium to achieve a final volume of 25 ml (dilution 1:100). 2.5 ml of such 1:100 dilution were mixed with 22.5 ml of LBS to achieve the dilution 1:1000. Liquid culture dilutions 1:100; 1:1,000; 1:10,000; 1:100,000; 1:1,000,000; 1:10,000,000 and 1:100,000,000 were prepared similarly. Aliquots of last three dilutions were spread on agar-solidified LBS medium supplemented with 25 mg/L kanamycin and 50 mg/L rifampicin (250 µl of bacterial culture per plate of 90 mm diameter). Plating of aliquots for each dilution was performed in triplicate. After 2 days incubation at 28°C, bacterial colonies were counted for each plate. Plating of 1: 1,000,000 and 1:10,000,000 dilutions resulted in few hundred and few dozen colonies per plate, respectively. So far as dilution 1:100,000,000 provided just few colonies per plate, this dilution was not used for calculation of cell concentration. The cell concentration was estimated according to the formula: cfu/ml = 4 x number of colonies per plate x dilution factor.

For transforming cell concentrations as measured by absorbance measurements at 600 nm (in LB medium) and in terms of cell-forming units, the following relation is used herein: an OD600 of 1.0 corresponds to 1.1 x10⁹ cfu/ml.

### LBS medium (liquid)

1% soya peptone (papaic hydrolysate of soybean meal; Duchefa, S1330)
0.5% yeast extract (Duchefa, Y1333)
1% sodium chloride (Carl Roth, 9265.2)
dissolved in water, and the is adjusted to pH 7.5 with 1M NaOH (Carl Roth, 6771.2)

To prepare the solid LBS medium, liquid LBS medium was supplemented with 1.5% agar (Carl Roth, 2266.2). Media were autoclaved at 121°C for 20 min.

### Example 1: Vectors used in the following examples

In this study we used standard transcriptional vectors based on 35S CaMV promoter as well as TMV- and PVX-based viral replicons with or without cell-to-cell movement ability.

All transcriptional vectors were created on the basis of pICBV10, a pBIN19-derived binary vector (Marillonnet et al., 2004, 2006). They contained two expression cassettes inserted within right and left borders of same T-DNA region (Fig. 1). For cloning of pNMD293 expression vector, two intermediate constructs (pNMD280 and pNMD033) were created. pNMD280 contained the expression cassette comprising, in sequential order, the Cauliflower mosaic virus (CAMV) 35S promoter, omega translational enhancer from Tobacco Mosaic Virus, coding sequence of P19 suppressor of silencing from Tomato Bushy Stunt Virus (TBSV) (GenBank accession no. CAB56483.1) and terminator from octopine synthase gene of *Agrobacterium tumefaciens* inserted between T-DNA right and left borders. To enable the next cloning step, two restriction sites, EcoRI and Sphl, were introduced between the T-DNA right border and the 35S promoter sequence. pNMD033 construct contained between left and right T-DNA borders the expression cassette flanked with EcoRI and SpHI restriction sites and comprized of 35S promoter, omega translational enhancer, coding sequence of jellyfish green fluorescent protein and terminator from octopin synthase gene of *Agrobacterium tumefaciens,* listed in sequential order. For cloning of pNMD293 construct, GFP expression cassette was excised from pNMD033 construct using EcoRI and Sphl restriction enzymes and transferred into pNMD280 vector linearized with same restrictases. Resulting pNMD293 construct contained two expression cassettes inserted between T-DNA right and left borders. An expression cassette adjacent to the right border comprised CAMV 35S promoter, omega translational enhancer, coding sequences of green fluorescent protein and the nos terminator (listed in sequential order). Expression cassette adjacent to the left border contained 35S promoter followed by omega translational enhancer, coding sequence of P19 suppressor of silencing and ocs terminator. All other construct were created on the basis pNMD293 vector, by replacing the GFP coding sequence with PCR-amplified coding sequences of another genes of interest using for cloning Ncol and BamHI restriction sites. Genes of interest introduced in transcriptional vector constructs encoded sGFP, modified green fluorescent protein (GFP) from jelly fish *Aequorea victoria* (GeneBank accession no. EF030489) (pNMD293); DsRED, red fluorescent protein from a *Discosoma* sp. reef coral (GeneBank accession no. AF168419.2) (pNMD1380); SP3D flowering factor from tomato (GeneBank accession no. AY186735) (pNMD421); Flowering Locus T (FT) from Arabidopsis (GeneBank accession no. BAA77839) (pNMD655); brassinosteroid regulator DWARF4 from Arabidopsis (NM_114926) (pNMD440); isopentenyl transferase (IPT), key enzyme of cytokinin biosynthesis from *Agrobacterium tumefaciens* strain C58/ATCC33970 (GeneBank accession no. AE007871.2) (pNMD460).

TMV-based vectors with cell-to cell movement ability (Fig. 2) were created on the basis of vectors described in Marillonnet et al. (2006). pNMD035 construct was employed as a cloning vector for consequent insertion of coding sequences of genes of interest using Bsal cloning sites. Resulting constructs contained, in sequential order, a fragment from the *Arabidopsis* actin 2 (*ACT2*) promoter (GenBank accession no. AB026654); the 5' end of TVCV (GenBank accession no. BRU03387, base pairs 1-5455) and a fragment of cr-TMV [GenBank accession no. Z29370, base pairs 5457-5677, both together containing 16 intron insertions]; a gene of interest; cr-TMV 3' nontranslated region (3' NTR; GenBank accession no. Z29370), and the nopaline synthase (Nos) terminator. The entire fragment was cloned between the T-DNA left and right borders of binary vector. Genes of interest used in these constructs encoded GFP (pNMD 560), dsRED (pNMD580), human interferon alpha-a with rice amylase apoplast-targeting signal (pNMD38), klip27-mini-insulin with rice amylase apoplast-targeting signal (pNMD330), thaumatin 2 from Taumatococcus danielii (pNMD700), β-glucosidase BGL4 from *Humicola grisea* (pNMD1200), exocellulase E3 from *Thermobifida fusca* (pNMD1160), exoglucanase 1 (CBH I) from *Trichoderma reesei* (pNMD1180), defensin Rs-AFP2 from *Rafanus sativus* (pNMD1061), defensin MsrA2 (a synthetic derivative of dermaseptin B1 from frog *Phyllomedusa bicolor*) (pNMD1071), defensin MB39 (modified cecropin from the Cecropia Moth *Hyalophora cecropia*) (pNMD1280), defensin plectasin from fungus *Pseudoplectania nigrella* (pNMDxxxx).

TMV-based vectors lacking cell-to cell movement ability were identical to corresponding TMV-based vectors capable of cell-to-cell movement with an exception of point mutation in MP-coding sequence leading to the open reading frame shift that distorted the MP translation (Fig. 3). Cloning of these constructs was performed using pNMD661 as a cloning vector.

For cloning of most of PVX-based vectors with cell- to-cell and systemic movement ability, pNMD670 cloning vector was used. Resulting constructs contained, in sequential order, 35S CaMV promoter, coding sequences of RNA-dependent RNA polymerase, coat protein, triple gene block modules comprising 25kDa, 12 kDa and 8 kDa proteins, gene of interest and 3'untranslated region. The entire fragment was cloned between the T-DNA left and right borders of binary vector (Fig. 4). Another group of PVX-based constructs had similar structure with difference in CP position, which was inserted between PVX polymerase and triple gene block (e. g., pNMD600).

PVX-based vectors with deletion of coat protein coding sequence were disabled for both systemic and cell-to cell movement. Cloning of these constructs was performed using pNMD694 as a cloning vector. This type of vectors contained, in sequential order, 35S CaMV promoter, coding sequences of RNA-dependent RNA polymerase, triple gene block module, gene of interest and 3'untranslated region inserted between the T-DNA left and right borders of binary vector (Fig.5).

### Example 2: Diluted agrobacteria can be delivered to Nicotiana benthamina using surfactant by spraying

We have shown that *Nicotiana benthamiana* plants can be transfected by spraying of plants with diluted agrobacterial cultures containing surfactant (Fig. 6). To evaluate the parameters influencing the transfection and optimize the transfection efficiency, we used dipping of *Nicotiana benthamiana* leaves in agrobacterial suspension. This approach allows exact measurements and easy testing of multiple experiment versions. Overnight agrobacterial cultures (OD600=1.5) were diluted 1:100 and 1:1000 (dilution factors 10⁻² and 10⁻², respectively) in 10 mM MES buffer (pH 5.5) containing 10 mM magnesium sulfate and supplemented with surfactant Silwet L-77. Three types of constructs providing GFP expression were tested: 1) transcriptional vectors, 2) TMV-based viral replicons and 3) PVX-based viral replicons (Fig. 6). Viral vectors used in these experiments were disabled for both systemic and cell-to-cell movement. They provided the expression of the reporter gene only in cells transfected with T-DNA. Percent of GFP-expressing cells was counted after the isolation of leaf protoplasts (Fig. 7). Depending of agrobacterial suspension concentration and regardless the type of vector, 2-8% of total leaf cells were transfected as a result of *Agrobacterium-*mediated T-DNA transfer when 0.1% per volume Silwet-L77 and 1 min dipping time were used.

To find the optimal surfactant concentration, we tested 0.1% and 0.05% Silwet L-77 in dipping experiments. For all three types of vectors, the transfection efficiency provided by using of 0.1% Silwet was significantly higher if compared to 0.05 % concentration (Fig. 8-10).

10 sec dipping of *Nicotiana benthamiana* leaves in diluted agrobacterial suspension supplemented with 0.1 % Silwet L-77 provided transfection rates close to the efficiency of spraying with same suspension (Fig. 11). In both cases the transfection efficiency was higher for older developed leaves. The transfection rate varied from 1.4 to 3.7 % for dipping, and from 1.1 to 1.7 % for spraying at 1:100 dilution of agrobacterial culture. At 1:1000 dilution the variation was 0.2 -1.1% for dipping and 0.1-0.6% for spraying.

The Silwet L-77 used in all examples herein was purchased from Kurt Obermeier GmbH & Co. KG (Bad Berleburg, Germany). The supplier is GE Silicones, Inc., USA. The Silwet L-77 used is an organosilicone product composed of 84.0 % of polyalkyleneoxide modified heptamethyltrisiloxane (CAS-No. 27306-78-1) and 16 % of allyloxypolyethylene-glycol methyl ether (CAS-No. 27252-80-8). All concentrations of Silwet L-77 content given in the examples or figures relate to this commercial product.

### Example 3: Diluted agrobacteria can be delivered to other species by spraying using surfactant and abrasive

We tested the number of plant species using agobacterial transfection with spraying and surfactant. First, we screened each species for optimal expression vector. For this purpose plant leaves were infiltrated using needleless syringe with 1:100 dilutions of OD=1.5 of five agrobacterial cultures harboring GFP expression transcriptional vectors: 1) transcriptional vector 35S-GFP + P19 (pNMD293), 2) TMV-based viral vector capable of cell-to-cell movement TMV(MP)-GFP (pNMD560), 3) TMV-based viral vector disabled for cell-to-cell movement TMV(fsMP)-GFP (pNMD570), 4) PXV-based viral vector capable of both systemic and cell-to-cell movement PVX(CP)-GFP (pNMD630) and 5) PVX-based viral vector disabled for both systemic and cell-to-cell movement PVX(ΔCP)-GFP (pNMD620). In some cases vacuum infiltration was performed.

We demonstrated the efficient *Agrobacterium*-mediated transfection for several Solanaceae species including Nicotiana benthamiana (all five vectors), tobacco *Nicotiana tabacum* (all five vectors), tomato *Lycopersicon esculentum* (PVX-based and transcriptional vectors), pepper *Capsicum annuum,* Inca berry *Physalis peruviana,* eggplant *Solanum melongena,* potato *Solanum tuberosum* (all with PVX-based vectors) (Fig. 13).

Agrobacterium-mediated transfection was denonstrated for the lettuce *Lactuca sativa* from Asteraceae family (transcriptional vector), beet *Beta vulgaris* from Chenopodiaceae family (all five vectors), zucchini *Cucurbita pepo* from Cucurbitaceae family (transcriptional vector), and cotton *Gossypium hirsutum* from Malvaceae family (all five vectors (Fig. 14).

Treatment of agrobacterial cells with acetosyringone (200 µM, 2 hours) significantly increased the transfection efficiency for several plant species including tomato, eggplant and zucchini (Fig. 15).

Based on infiltration data, spraying with diluted agrobacterial suspensions was tested for the number of plant species. The efficient delivery of diluted agrobacteria (10⁻³) by spraying with suspensions containing 0.1% Silwet was demonstrated for several Nicotiana species (*Nicotiana benthamiana, Nicotiana debne, Nicotiana excelsior, Nicotiana exigua, Nicotiana maritima* and *Nicotiana simulans*) as it is shown using PVX with cell-to cell and systemic movement ability in Fig. 16.

Delivery of agrobacteria to other species including Solanaceae, Chenopodiaceae, Amarantaceae and Aizoaceae families was demonstrated by both dipping in agrobacterial suspension and spraying with and without abrasive using transcriptional vectors as well as TMV and PVX vectors with and without cell-to-cell movement ability (Fig. 17-21). The list of species successfully transfected includes spinach *Spinacea oleracea* from Amaranthaceae family (transcriptional and PVX-based vectors), beet *Beta vulgaris* varieties from Chenopodiaceae family (TMV-based and PVX-based viral vectors) (Fig. 17), tomato *Lycopersicon esculentum* (PVX-based vector) (Fig. 18), Inca berry *Physalis peruviana* and potato *Solanum tuberosum* (Fig. 34) (PVX-based vector) (Fig. 19) from Solanaceae family, cotton *Gossypium hirsutum* from Malvaceae family (TMV-based vector) (Fig. 20). The expression of GFP in cotton tissue after agrobacterial transfection was confirmed using Western blot probed with GFP-specific antibodies (Fig. 21).

Using the GUS gene as a reporter, the successful transfection by spraying with agrobacterial suspension was achieved for rapeseed *Brassica napus* from *Brassicaceae* family (Fig. 35). The pNMD1971 construct was created on the basis of pNMD293 plasmid by replacing the GFP coding sequence with sequence of beta-glucuronidase (GUS) from *Escherichia coli* (P05804) containing the 7th intron from *Petunia hybrida* PSK7 gene (AJ224165).

The efficient transfection of plants using the spraying with diluted agrobacterial suspension was demonstrated also for monocot species. Fig. 36 shows the transfection of onion *Allium cepa* plants after the spraying with agrobacterial suspension supplemented with 0.1% Silwet L-77. The pNMD2210 construct was created on the basis of pNMD1971 plasmid by replacing the 35S promoter in the GUS expression cassette with actin 2 (Act2) promoter from rice Oryza sativa (EU155408).

In all examples described herein, spraying was performed either with a pump spray flasks with nominal volume or 500 or 1000 ml (Carl Roth, # 0499.1 and # 0500.1) based on direct manual pumping or with a pressure sprayer with 1.25 L volume (Gardena, # 00864-20) exploiting the increased pressure for pumping. Plants were sprayed so as to wet completely leaves. Sprayers were shaken from time to time to ensure homogeneity of the suspensions to be sprayed, notably if the suspensions contained an abrasive.

### Example 4: Transfection of plants using Agrobacterum suspensions containing abrasive

The carborundum used in these experiments was a mixture of carborundum (silicon carbide) F800, F1000 and F1200 particles from Mineraliengrosshandel Hausen GmbH, Telfs, Austria. According to the provider, F800, F1000 and F1200 have surface median diameters of 6.5, 4.5 and 3 µm, respectively. 97 mass-% of the particles of F800, F1000 and F1200 have a surface diameter smaller than 14, 10 and 7 µm, respectively. 94 mass-% of the particles have a surface diameter larger than 2, 1, and 1 µm, respectively. F800, F1000 and F1200 were mixed in equal amounts by weight. 0.3% (w/v) of the mixed carborundum was added into the agrobacterial suspensions supplemented with 0.1% Silwet L-77 and used for the spraying of plants using the sprayers described in example 3.

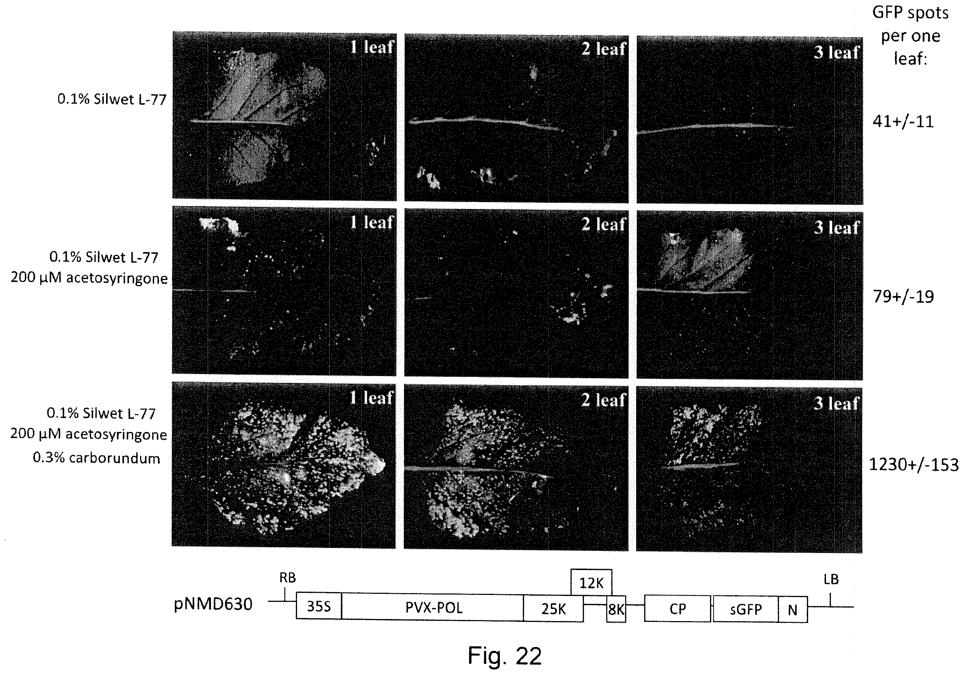

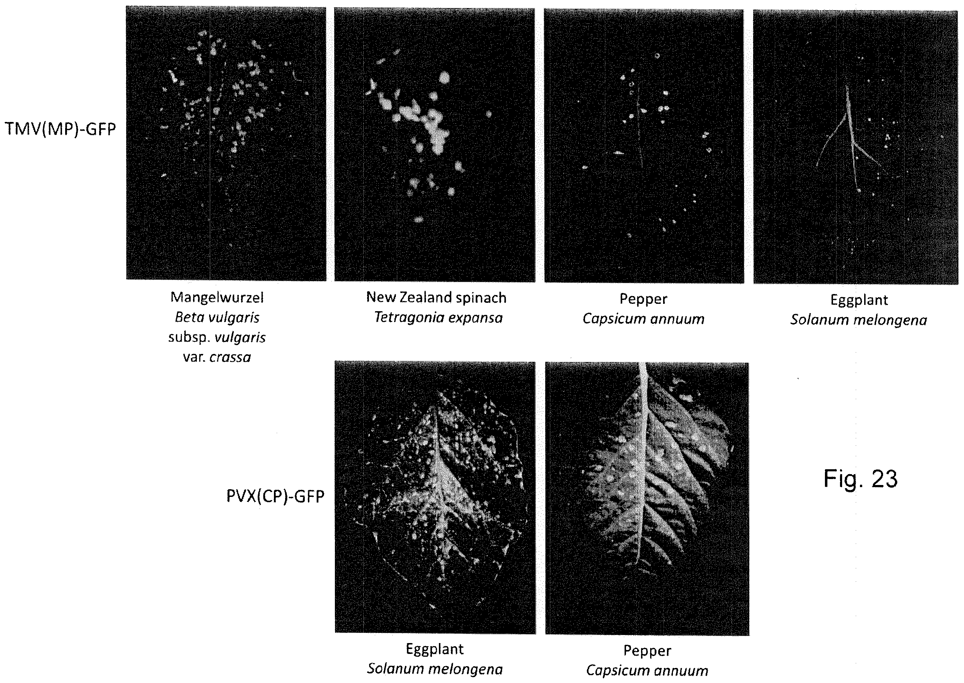

The results shown in Fig. 32, 22 and 33 demonstrated that use of the abrasive significantly increases the transfection efficiency. Spraying of eggplant *Solanum melongena* plants with with agrobacterial suspension containing 0.3 % of carborundum (silicon carbide SiC) provided a 2-fold increase of transfection efficiency (Fig. 32). In case of red beets, same abrasive treatment resulted in a 15-fold increase of transfection efficiency (Fig. 22). Remarkably, the use of an abrasive was a decisive factor allowed the transfection of pepper plants by spraying with agrobacterial suspension; combination of an abrasive treatment with acetosyringone activation of agrobacterial cells further increased the transfection effciency (Fig. 32). List of species transfected using spraying with surfactant and abrasive includes also Mangelwurzel, another variety of *Beta vulgaris,* New Zealand spinach *Tetragonia expansa* from Aizoaceae family, pepper *Capsicum annuum* and eggplant *Solanum melongena* from Solanaceae (Fig. 23).

### Example 5: Treatment with agrobacteria can be repeated: multiple subsequent treatments

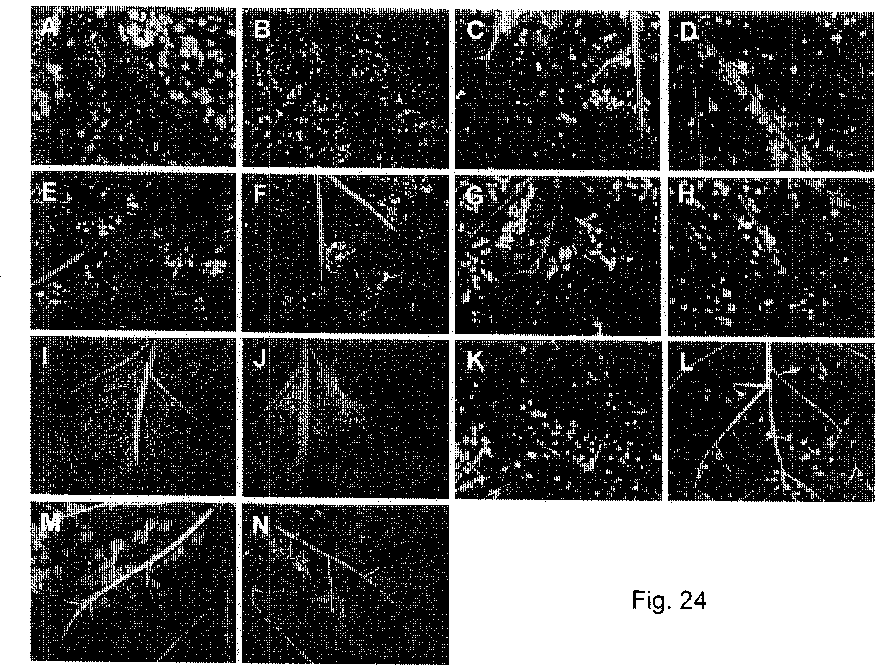

We performed multiple subsequent treatments of Nicotiana benthamiana plants with diluted agrobacteria. For this purpose leaves were dipped in diluted suspensions of Agrobacterium carrying next constructs: pNMD570 (TMV(fsMP)-GFP without cell-to-cell movement ability), pNMD560 (TMV(MP)-GFP with cell-to-cell movement ability), pNMD580 (TMV(MP)-DsRED with cell-to-cell movement ability), pNMD620 (PVX(ΔCP)-GFP without cell-to-cell movement ability, pNMD600 (PVX(CP)-GFP with cell-to-cell and systemic movement ability) and pNMD610 (PVX(CP)-dsRED with cell-to-cell and systemic movement ability). After the transfection, these vectors form fluorescing spots differing in colour and size (Fig. 24). We performed the dipping transfection of each tested leaf with 3 different cultures and with 7 days intervals between transfections. For each of eight tested vector combinations all transfections with agrobacterium were successful, no particular silencing effect was observed (Fig. 24).

### Example 6: Spraying with agrobacteria can deliver viral replicons capable of cell-to-cell movement

We demonstrated that spraying of *Nicotiana benthamiana* plants with 1:100 and 1:1000 dilutions of agrobacterial suspension provides efficient delivery of viral replicons capable of cell to cell-movement, which results in high expression of genes of interest, comparable with expression achieved using infiltration of agrobacteria. This was demonstrated for GFP (Fig. 25), human alpha-a interferon and klip27-mini-insulin (Fig. 26), and several cellulases including exocellulase E3 from *Thermobifida fusca,* exoglucanase 1 (CBH I) from *Trichoderma reesei,* β-glucosidase BGL4 from *Humicola grisea* and exocellulase E3 from *Thermobifida fusca* (Fig. 27).

### Example 7: Agrobacteria can be used to deliver transcription factors as secondary messengers

We have demonstrated the induction of anthocyanin biosynthesis in *Nicotiana tabacum* leaves infiltrated with agrobacterial suspension harbouring the PVX-based viral vector providing the expression of MYB transcription factor anthocyanin 1 (ANT1) from *Lycopersicon esculentum* (Fig. 28).

### Example 8: Agrobacteria can be used to deliver RNAi as secondary messengers

We have demonstrated the photobleaching of *Nicotiana benthamiana* leaves caused by silencing of phytoene desaturase (PDS) gene after the spraying of leaves with agrobacterial suspension bearing the PVX-based viral vector containing the fragment of PDS coding sequence in an anti-sense orientation (Fig. 37). To generate this construct (pNMD050), a 298-624 bp fragment of *Nicotiana benthamiana* phytoene desaturase (PDS) cDNA (EU165355) was inserted into pNMD640 cloning vector in an anti-sense orientation using Bsal sites.

### Example 9: Agrobacteria can be used to deliver MAMPs (Microbe-Associated Molecular Patterns) as secondary messengers

We demonstrated the reduction in number of necrotic lesions caused by *Pseudomonas syringae* pv. *syringae* infection in *Nicotiana benthamiana* leaves after the preliminary spraying of plants with agrobacterial suspension bearing the PVX-based vector providing the expression of flagellin gene from *Pseudomonas* (pNMD1953) (Fig. 38). To create the pNMD1953 plasmid, the GFP coding sequence was replaced in pNMD630 construct with sequence comprising the fragment encoding apoplast signal peptide from barley (Hordeum vulgare) alpha-amylase (AMY3) gene (FN179391) fused in frame with sequence encoding the flagellin from *Pseudomonas syringae* pv. *syringae* (YP236536). Four *Nicotiana benthamiana* plants were inoculated with 1:1000 dilutions of *Agrobacterium* cultures (OD₆₀₀=1.3) by spraying. 6 dpi of *Agrobacterium* cultures, all plants were inoculated with *Pseudomonas syringae* pv. *syringae* B728 at 1x10⁵ cfu/ml by sprayng. 7 dpi of Pseudomonas, disease symptoms were scored by counting necrotic spots on two leaves of each plant. The number of necrotic spots caused by Pseudomonas-infection per leaf is given as average of each 2 leaves of 4 plants.

The sequence listing below contains the following nucleotide sequences:
SEQ ID NO: 1: TNA region of T-DNA region of pNMD280
SEQ ID NO: 2: TNA region of T-DNA region of pNMD033
SEQ ID NO: 3: TNA region of T-DNA region of pNMD035
SEQ ID NO: 4: TNA region of T-DNA region of pNMD661
SEQ ID NO: 5: TNA region of T-DNA region of pNMD670
SEQ ID NO: 6: TNA region of T-DNA region of pNMD694
SEQ ID NO: 7: TNA region of T-DNA region of pNMD1971
SEQ ID NO: 8: TNA region of T-DNA region of pNMD2210
SEQ ID NO: 9: TNA region of T-DNA region of pNMD050
SEQ ID NO: 10: TNA region of T-DNA region of pNMD1953

### References

1. Andrews, L. B. & Curtis, W. R. (2005). Comparison of transient protein expression in tobacco leaves and plant suspension culture. Biotechnol Prog 21, 946-52.
2. Arguelles-Arias, A., Ongena, M., Halimi, B., Lara, Y., Brans, A., Joris, B. & Fickers, P. (2009). Bacillus amyloliquefaciens GA1 as a source of potent antibiotics and other secondary metabolites for biocontrol of plant pathogens. Microb Cell Fact 8, 63.
3. Azhakanandam, K., Weissinger, S. M., Nicholson, J. S., Qu, R. & Weissinger, A. K. (2007). Amplicon-plus targeting technology (APTT) for rapid production of a highly unstable vaccine protein in tobacco plants. Plant Mol Biol 63, 393-404.
4. Barton, K. A., Binns, A. N., Matzke, A. J. & Chilton, M. D. (1983). Regeneration of intact tobacco plants containing full length copies of genetically engineered T-DNA, and transmission of T-DNA to R1 progeny. Cell 32, 1033-43.
5. Baum, J. A., Bogaert, T., Clinton, W., Heck, G. R., Feldmann, P., Ilagan, O., Johnson, S., Plaetinck, G., Munyikwa, T., Pleau, M., Vaughn, T. & Roberts, J. (2007). Control of coleopteran insect pests through RNA interference. Nat Biotechnol 25, 1322-6.
6. Bergey, D. R., Howe, G. A. & Ryan, C. A. (1996). Polypeptide signaling for plant defensive genes exhibits analogies to defense signaling in animals. Proc Natl Acad Sci U S A 93, 12053-8.
7. Broothaerts, W., Mitchell, H. J., Weir, B., Kaines, S., Smith, L. M., Yang, W., Mayer, J. E., Roa-Rodriguez, C. & Jefferson, R. A. (2005). Gene transfer to plants by diverse species of bacteria. Nature 433, 629-33.
8. Chuang, C. F. & Meyerowitz, E. M. (2000). Specific and heritable genetic interference by double-stranded RNA in Arabidopsis thaliana. Proc Natl Acad Sci U S A 97, 4985-90.
9. Chung, M. H., Chen, M. K. & Pan, S. M. (2000). Floral spray transformation can efficiently generate Arabidopsis transgenic plants. Transgenic Res 9, 471-6.
10. Clough, S. J. & Bent, A. F. (1998). Floral dip: a simplified method for Agrobacterium-mediated transformation of Arabidopsis thaliana. Plant J 16, 735-43.
11. Corbesier, L., Vincent, C., Jang, S., Fornara, F., Fan, Q., Searle, I., Giakountis, A., Farrona, S., Gissot, L., Turnbull, C. & Coupland, G. (2007). FT protein movement contributes to long-distance signaling in floral induction of Arabidopsis. Science 316, 1030-3.
12. D'Aoust, M. A., Lavoie, P. O., Belles-Isles, J., Bechtold, N., Martel, M. & Vezina, L. P. (2009). Transient expression of antibodies in plants using syringe agroinfiltration. Methods Mol Biol 483, 41-50.
13. D'Aoust, M. A., Lavoie, P. O., Couture, M. M., Trepanier, S., Guay, J. M., Dargis, M., Mongrand, S., Landry, N., Ward, B. J. & Vezina, L. P. (2008). Influenza virus-like particles produced by transient expression in Nicotiana benthamiana induce a protective immune response against a lethal viral challenge in mice. Plant Biotechnol J 6, 930-40.
14. De Buck, S., De Wilde, C., Van Montagu, M. & Depicker, A. (2000). Determination of the T-DNA transfer and the T-DNA integration frequencies upon cocultivation of Arabidopsis thaliana root explants. Mol Plant Microbe Interact 13, 658-65.
15. De Buck, S., Jacobs, A., Van Montagu, M. & Depicker, A. (1998). Agrobacterium tumefaciens transformation and cotransformation frequencies of Arabidopsis thaliana root explants and tobacco protoplasts. Mol Plant Microbe Interact 11, 449-57.
16. de Felippes, F. F. & Weigel, D. (2010). Transient assays for the analysis of miRNA processing and function. Methods Mol Biol 592, 255-64.
17. Deluc, L., Bogs, J., Walker, A. R., Ferrier, T., Decendit, A., Merillon, J. M., Robinson, S. P. & Barrieu, F. (2008). The transcription factor VvMYB5b contributes to the regulation of anthocyanin and proanthocyanidin biosynthesis in developing grape berries. Plant Physiol 147, 2041-53.
18. Escobar, M. A., Civerolo, E. L., Summerfelt, K. R. & Dandekar, A. M. (2001). RNAi-mediated oncogene silencing confers resistance to crown gall tumorigenesis. Proc Natl Acad Sci U S A 98, 13437-42.
19. Fraley, R. T., Rogers, S. G., Horsch, R. B., Sanders, P. R., Flick, J. S., Adams, S. P., Bittner, M. L., Brand, L. A., Fink, C. L., Fry, J. S., Galluppi, G. R., Goldberg, S. B., Hoffmann, N. L. & Woo, S. C. (1983). Expression of bacterial genes in plant cells. Proc Natl Acad Sci U S A 80, 4803-7.
20. Giritch, A., Marillonnet, S., Engler, C., van Eldik, G., Botterman, J., Klimyuk, V. & Gleba, Y. (2006). Rapid high-yield expression of full-size IgG antibodies in plants coinfected with noncompeting viral vectors. Proc Natl Acad Sci U S A 103, 14701-6.
21. Gleba, Y., Klimyuk, V. & Marillonnet, S. (2007). Viral vectors for the expression of proteins in plants. Curr Opin Biotechnol 18, 134-41.
22. Gleba, Y., Marillonnet, S. & Klimyuk, V. (2004). Engineering viral expression vectors for plants: the 'full virus' and the 'deconstructed virus' strategies. Curr Opin Plant Biol 7, 182-8.
23. Gleba, Y., Marillonnet, S. & Klimyuk, V. (2008). Plant virus vectors (gene expression systems). In Encyclopedia of Virology, Third Edition. M.H.V. van Regenmortel, Mahy, B.W..J., eds. (San Diego, CA: Elsevier Academic Press), vol. 4, pp. 229-237.
24. Green, B. J., Fujiki, M., Mett, V., Kaczmarczyk, J., Shamloul, M., Musiychuk, K., Underkoffler, S., Yusibov, V. & Mett, V. (2009). Transient protein expression in three Pisum sativum (green pea) varieties. Biotechnol J 4, 230-7.
25. Grimsley, N., Hohn, B., Hohn, T. & Walden, R. (1986). "Agroinfection," an alternative route for viral infection of plants by using the Ti plasmid. Proc Natl Acad Sci U S A 83, 3282-3286.
26. Huang, G., Allen, R., Davis, E. L., Baum, T. J. & Hussey, R. S. (2006). Engineering broad root-knot resistance in transgenic plants by RNAi silencing of a conserved and essential root-knot nematode parasitism gene. Proc Natl Acad Sci U S A 103, 14302-6.
27. Huang, Z., Santi, L., LePore, K., Kilbourne, J., Arntzen, C. J. & Mason, H. S. (2006). Rapid, high-level production of hepatitis B core antigen in plant leaf and its immunogenicity in mice. Vaccine 24, 2506-13.
28. Joh, L. D., McDonald, K. A. & VanderGheynst, J. S. (2006). Evaluating extraction and storage of a recombinant protein produced in agroinfiltrated lettuce. Biotechnol Prog 22, 723-30.
29. Joh, L. D., Wroblewski, T., Ewing, N. N. & VanderGheynst, J. S. (2005). High-level transient expression of recombinant protein in lettuce. Biotechnol Bioeng 91, 861-71.
30. Johnson, K. B. (2009). Optimization of Biocontrol of Plant Diseases. Annu Rev Phytopathol.
31. Jones, H. D., Doherty, A. & Sparks, C. A. (2009). Transient transformation of plants. Methods Mol Biol 513, 131-52.
32. Kerr, A. & Tate, M. E. (1984). Agrocins and the biological control of crown gall. Microbiol Sci 1, 1-4.
33. Kim, J. G., Park, B. K., Kim, S. U., Choi, D., Nahm, B. H., Moon, J. S., Reader, J. S., Farrand, S. K. & Hwang, I. (2006). Bases of biocontrol: sequence predicts synthesis and mode of action of agrocin 84, the Trojan horse antibiotic that controls crown gall. Proc Natl Acad Sci U S A 103, 8846-51.
34. Lee, J. J., Woodward, A. W. & Chen, Z. J. (2007). Gene expression changes and early events in cotton fibre development. Ann Bot 100, 1391-401.
35. Lee, M. W. & Yang, Y. (2006). Transient expression assay by agroinfiltration of leaves. Methods Mol Biol 323, 225-9.
36. Li, J. F., Park, E., von Arnim, A. G. & Nebenfuhr, A. (2009). The FAST technique: a simplified Agrobacterium-based transformation method for transient gene expression analysis in seedlings of Arabidopsis and other plant species. Plant Methods 5, 6.
37. Li, X. Q., Liu, C. N., Ritchie, S. W., Peng, J. Y., Gelvin, S. B. & Hodges, T. K. (1992). Factors influencing Agrobacterium-mediated transient expression of gusA in rice. Plant Mol Biol 20, 1037-48.
38. Lico, C., Chen, Q. & Santi, L. (2008). Viral vectors for production of recombinant proteins in plants. J Cell Physiol 216, 366-77.
39. Lindbo, J. A. (2007). TRBO: a high-efficiency tobacco mosaic virus RNA-based overexpression vector. Plant Physiol 145, 1232-40.
40. Lindbo, J. A. (2007). High-efficiency protein expression in plants from agroinfection-compatible Tobacco mosaic virus expression vectors. BMC Biotechnol 7, 52.
41. Liu, C. N., Li, X. Q. & Gelvin, S. B. (1992). Multiple copies of virG enhance the transient transformation of celery, carrot and rice tissues by Agrobacterium tumefaciens. Plant Mol Biol 20, 1071-87.
42. Marillonnet, S., Giritch, A., Gils, M., Kandzia, R., Klimyuk, V. & Gleba, Y. (2004). In planta engineering of viral RNA replicons: efficient assembly by recombination of DNA modules delivered by Agrobacterium. Proc Natl Acad Sci U S A 101, 6852-7.
43. Marillonnet, S., Thoeringer, C., Kandzia, R., Klimyuk, V. & Gleba, Y. (2005). Systemic Agrobacterium tumefaciens-mediated transfection of viral replicons for efficient transient expression in plants. Nat Biotechnol 23, 718-23.
44. Martinez-Garcia, J. F., Virgos-Soler, A. & Prat, S. (2002). Control of photoperiod-regulated tuberization in potato by the Arabidopsis flowering-time gene CONSTANS. Proc Natl Acad Sci U S A 99, 15211-6.
45. Mett, V., Lyons, J., Musiychuk, K., Chichester, J. A., Brasil, T., Couch, R., Sherwood, R., Palmer, G. A., Streatfield, S. J. & Yusibov, V. (2007). A plant-produced plague vaccine candidate confers protection to monkeys. Vaccine 25, 3014-7.
46. Moore, L. W. (1988). Use of Agrobacterium radiobacter in agricultural ecosystems. Microbiol Sci 5, 92-5.
47. Nam, M. H., Park, M. S., Kim, H. G. & Yoo, S. J. (2009). Biological control of strawberry Fusarium wilt caused by Fusarium oxysporum f. sp. fragariae using Bacillus velezensis BS87 and RK1 formulation. J Microbiol Biotechnol 19, 520-4.
48. Pandolfini, T., Rotino, G. L., Camerini, S., Defez, R. & Spena, A. (2002). Optimisation of transgene action at the post-transcriptional level: high quality parthenocarpic fruits in industrial tomatoes. BMC Biotechnol 2, 1.
49. Plesha, M. A., Huang, T. K., Dandekar, A. M., Falk, B. W. & McDonald, K. A. (2007). High-level transient production of a heterologous protein in plants by optimizing induction of a chemically inducible viral amplicon expression system. Biotechnol Prog 23, 1277-85.
50. Plesha, M. A., Huang, T. K., Dandekar, A. M., Falk, B. W. & McDonald, K. A. (2009). Optimization of the bioprocessing conditions for scale-up of transient production of a heterologous protein in plants using a chemically inducible viral amplicon expression system. Biotechnol Prog 25, 722-34.
51. Pooggin, M., Shivaprasad, P. V., Veluthambi, K. & Hohn, T. (2003). RNAi targeting of DNA virus in plants. Nat Biotechnol 21, 131-2.
52. Reader, J. S., Ordoukhanian, P. T., Kim, J. G., de Crecy-Lagard, V., Hwang, I., Farrand, S. & Schimmel, P. (2005). Major biocontrol of plant tumors targets tRNA synthetase. Science 309, 1533.
53. Regnard, G. L., Halley-Stott, R. P., Tanzer, F. L., Hitzeroth, II & Rybicki, E. P. (2010). High level protein expression in plants through the use of a novel autonomously replicating geminivirus shuttle vector. Plant Biotechnol J 8, 38-46.
54. Ryu, C. M., Anand, A., Kang, L. & Mysore, K. S. (2004). Agrodrench: a novel and effective agroinoculation method for virus-induced gene silencing in roots and diverse Solanaceous species. Plant J 40, 322-31.
55. Santi, L., Giritch, A., Roy, C. J., Marillonnet, S., Klimyuk, V., Gleba, Y., Webb, R., Arntzen, C. J. & Mason, H. S. (2006). Protection conferred by recombinant Yersinia pestis antigens produced by a rapid and highly scalable plant expression system. Proc Natl Acad Sci U S A 103, 861-6.
56. Shang, Y., Schwinn, K. E., Bennett, M. J., Hunter, D. A., Waugh, T. L., Pathirana, N. N., Brummell, D. A., Jameson, P. E. & Davies, K. M. (2007). Methods for transient assay of gene function in floral tissues. Plant Methods 3, 1.
57. Shiboleth, Y. M., Arazi, T., Wang, Y. & Gal-On, A. (2001). A new approach for weed control in a cucurbit field employing an attenuated potyvirus-vector for herbicide resistance. J Biotechnol 92, 37-46.
58. Shoji, Y., Farrance, C. E., Bi, H., Shamloul, M., Green, B., Manceva, S., Rhee, A., Ugulava, N., Roy, G., Musiychuk, K., Chichester, J. A., Mett, V. & Yusibov, V. (2009). Immunogenicity of hemagglutinin from A/Bar-headed Goose/Qinghai/1A/05 and A/Anhui/1/05 strains of H5N1 influenza viruses produced in Nicotiana benthamiana plants. Vaccine 27, 3467-70.
59. Simmons, C. W., VanderGheynst, J. S. & Upadhyaya, S. K. (2009). A model of Agrobacterium tumefaciens vacuum infiltration into harvested leaf tissue and subsequent in planta transgene transient expression. Biotechnol Bioeng 102, 965-70.
60. Sudarshana, M. R., Plesha, M. A., Uratsu, S. L., Falk, B. W., Dandekar, A. M., Huang, T. K. & McDonald, K. A. (2006). A chemically inducible cucumber mosaic virus amplicon system for expression of heterologous proteins in plant tissues. Plant Biotechnol J 4, 551-9.
61. Turpen, T. H., Turpen, A. M., Weinzettl, N., Kumagai, M. H. & Dawson, W. O. (1993). Transfection of whole plants from wounds inoculated with Agrobacterium tumefaciens containing cDNA of tobacco mosaic virus. J Virol Methods 42, 227-39.
62. Vaquero, C., Sack, M., Chandler, J., Drossard, J., Schuster, F., Monecke, M., Schillberg, S. & Fischer, R. (1999). Transient expression of a tumor-specific single-chain fragment and a chimeric antibody in tobacco leaves. Proc Natl Acad Sci U S A 96, 11128-33.
63. Vezina, L. P., Faye, L., Lerouge, P., D'Aoust, M. A., Marquet-Blouin, E., Burel, C., Lavoie, P. O., Bardor, M. & Gomord, V. (2009). Transient co-expression for fast and high-yield production of antibodies with human-like N-glycans in plants. Plant Biotechnol J 7, 442-55.
64. Vicedo, B., Penalver, R., Asins, M. J. & Lopez, M. M. (1993). Biological Control of Agrobacterium tumefaciens, Colonization, and pAgK84 Transfer with Agrobacterium radiobacter K84 and the Tra Mutant Strain K1026. Appl Environ Microbiol 59, 309-315.
65. Voinnet, O., Rivas, S., Mestre, P. & Baulcombe, D. (2003). An enhanced transient expression system in plants based on suppression of gene silencing by the p19 protein of tomato bushy stunt virus. Plant J 33, 949-56.
66. Wroblewski, T., Tomczak, A. & Michelmore, R. (2005). Optimization of Agrobacterium-mediated transient assays of gene expression in lettuce, tomato and Arabidopsis. Plant Biotechnol J 3, 259-73.
67. Yang, L., Wang, H., Liu, J., Li, L., Fan, Y., Wang, X., Song, Y., Sun, S., Wang, L., Zhu, X. & Wang, X. (2008). A simple and effective system for foreign gene expression in plants via root absorption of agrobacterial suspension. J Biotechnol 134, 320-4.
68. Yang, Y., Li, R. & Qi, M. (2000). In vivo analysis of plant promoters and transcription factors by agroinfiltration of tobacco leaves. Plant J 22, 543-51.
69. Zambre, M., Terryn, N., De Clercq, J., De Buck, S., Dillen, W., Van Montagu, M., Van Der Straeten, D. & Angenon, G. (2003). Light strongly promotes gene transfer from Agrobacterium tumefaciens to plant cells. Planta 216, 580-6.
70. Zeevaart, J. A. (2008). Leaf-produced floral signals. Curr Opin Plant Biol 11, 541-7.
71. Zhang, C. & Ghabrial, S. A. (2006). Development of Bean pod mottle virus-based vectors for stable protein expression and sequence-specific virus-induced gene silencing in soybean. Virology 344, 401-11.
72. Zhao, M. M., An, D. R., Zhao, J., Huang, G. H., He, Z. H. & Chen, J. Y. (2006). Transiently expressed short hairpin RNA targeting 126 kDa protein of tobacco mosaic virus interferes with virus infection. Acta Biochim Biophys Sin (Shanghai) 38, 22-8.

### Annex: Sequences of T-DNA regions for basic constructs used as cloning vectors

pNMD280 (Fig. 1) 
cctgtggttggcacatacaaatggacgaacggataaaccttttcacgcccttttaaatatccgattattctaataaacgctcttttctcttaggtttacccgc
caatatatcctgtcaaacactgatagtttaaactgaaggcgggaaacgacaatctgatctaagctaggcatggaattcccagggggtggcatgc
caattccaatcccacaaaaatctgagcttaacagcacagttgctcctctcagagcagaatcgggtattcaacaccctcatatcaactactacgttgtg
tataacggtccacatgccggtatatacgatgactggggttgtacaaaggcggcaacaaacggcgttcccggagttgcacacaagaaatttgccac
tattacagaggcaagagcagcagctgacgcgtacacaacaagtcagcaaacagacaggttgaacttcatccccaaaggagaagctcaactca
agcccaagagctttgctaaggccctaacaagcccaccaaagcaaaaagcccactggctcacgctaggaaccaaaaggcccagcagtgatcc
agccccaaaagagatctcctttgccccggagattacaatggacgatttcctctatctttacgatctaggaaggaagttcgaaggtgaaggtgacgac
actatgttcaccactgataatgagaaggttagcctcttcaatttcagaaagaatgctgacccacagatggttagagaggcctacgcagcaggtctca
tcaagacgatctacccgagtaacaatctccaggagatcaaataccttcccaagaaggttaaagatgcagtcaaaagattcaggactaattgcatc
aagaacacagagaaagacatatttctcaagatcagaagtactattccagtatggacgattcaaggcttgcttcataaaccaaggcaagtaataga
gattggagtctctaaaaaggtagttcctactgaatctaaggccatgcatggagtctaagattcaaatcgaggatctaacagaactcgccgtgaagac
tggcgaacagttcatacagagtcttttacgactcaatgacaagaagaaaatcttcgtcaacatggtggagcacgacactctggtctactccaaaaat
gtcaaagatacagtctcagaagaccaaagggctattgagacttttcaacaaaggataatttcgggaaacctcctcggattccattgcccagctatct
gtcacttcatcgaaaggacagtagaaaaggaaggtggctcctacaaatgccatcattgcgataaaggaaaggctatcattcaagatctctctgccg
acagtggtcccaaagatggacccccacccacgaggagcatcgtggaaaaagaagacgttccaaccacgtcttcaaagcaagtggattgatgtg
acatctccactgacgtaagggatgacgcacaatcccactatccttcgcaagacccttcctctatataaggaagttcatttcatttggagaggacacgc
tcgagtataagagctctatttttacaacaattaccaacaacaacaaacaacaaacaacattacaattacatttacaattaccatggaacgagctata
caaggaaacgatgctagggaacaagcttatggtgaacgttggaatggaggatcaggaagttccacttctcccttcaaacttcctgacgaaagtccg
agttggactgagtggcggctacataacgatgagacgatttcgaatcaagataatccccttggtttcaaggaaagctggggtttcgggaaagttgtattt
aagagatatctcagatacgacggacggaaacttcactgcacagagtccttggatcttggacgggagattcggttaactatgcagcatctcgatttct
cggtttcgaccagatcggatgtacctatagtattcggtttcgaggagttagtgtcaccatttctggagggtcgcgaactcttcagcatctcagtgaaatg
gcaattcggtctaagcaagaactgctacagcttaccccagtcaaagtggaaagtgatgtatcaagaggatgccctgaaggtgttgaaaccttcgaa
gaagaaagcgagtaaggatcctctagagtcctgctttaatgagatatgcgagacgcctatgatcgcatgatatttgctttcaattctgttgtgcacgttgt
aaaaaacctgagcatgtgtagctcagatccttaccgccggtttcggttcattctaatgaatatatcacccgttactatcgtatttttatgaataatattctcc
gttcaatttactgattgtaccctactacttatatgtacaatattaaaatgaaaacaatatattgtgctgaataggtttatagcgacatctatgatagagcgc
cacaataacaaacaattgcgttttattattacaaatccaattttaaaaaaagcggcagaaccggtcaaacctaaaagactgattacataaatcttattc
aaatttcaaaagtgccccaggggctagtatctacgacacaccgagcggcgaactaataacgctcactgaagggaactccggttccccgccggc
gcgcatgggtgagattccttgaagttgagtattggccgtccgctctaccgaaagttacgggcaccattcaacccggtccagcacggcggccgggta
accgacttgctgccccgagaattatgcagcatttttttggtgtatgtgggccccaaatgaagtgcaggtcaaaccttgacagtgacgacaaatcgttg
ggcgggtccagggcgaatttgcgacaacatgtcgaggctcagcaggacctgcataagctcttctgtcagcgggcccactgcatccaccccagta
cattaaaaacgtccgcaatgtgttattaagttgtctaagcgtcaatttgtttacaccacaatatatcctgccaccagccagccaacagctccccgaccg
gcagctcggcacaaaatcaccactcgatacaggcagcc
pNMD033 (Fig. 1) 
tgatgggctgcctgtatcgagtggtgattttgtgccgagctgccggtcggggagctgttggctggctggtggcaggatatattgtggtgtaaacaaattg
acgcttagacaacttaataacacattgcggacgtttttaatgtactggggtggatgcagnnnnnnctgctgagcctcgacatgttgtcgcaaaattcg
ccctggacccgcccaacgatttgtcgtcactgtcaaggtttgacctgcacttcatttggggcccacatacaccaaaaaaatgctgcataattctcggg
gcagcaagtcggttacccggccgccgtgctggaccgggttgaatggtgcccgtaactttcggtagagcggacggccaatactcaacttcaaggaa
tctcacccatgcgcgccggcggggaaccggagttcccttcagtgaacgttattagttcgccgctcggtgtgtcgtagatactagcccctggggcctttt
gaaatttgaataagatttatgtaatcagtcttttaggtttgaccggttctgccgctttttttaaaattggatttgtaataataaaacgcaattgtttgttattgtgg
cgctctatcatagatgtcgctataaacctattcagcacaatatattgttttcattttaatattgtacatataagtagtagggtacaatcagtaaattgaacgg
agaatattattcataaaaatacgatagtaacgggtgatatattcattagaatgaaccgaaaccggcggtaaggatctgagctacacatgctcaggttt
tttacaacgtgcacaacagaattgaaagcaaatatcatgcgatcataggcgtctcgcatatctcattaaagcaggactctaggatcgatcccccgg
gtcatcaaatctcggtgacgggcaggaccggacggggcggtaccggcaggctgaagtccagctgccagaaacccacgtcatgccagttcccgt
gcttgaagccggccgcccgcagcatgccgcgggggggcatatccgagcgcctcgtgcatgcgcacgctcgggtcgttgggcagcccgatgacag
cgaccacgctcttgaagccctgtgcctccagggacttcagcaggtgggtgtagagcgtggagcccagtcccgtccgctggtggcgggggggagac
gtacacggttgactcggccgtccagtcgtaggcgttgcgtgccttccaggggcccgcgtaggcgatgccggcgacctcgccgtccacctcggcga
cgagccagggatagcgctcccgcagacggacgaggtcgtccgtccactcctgcggttcctgcggctcggtacggaagttgaccgtgcttgtctcga
tgtagtggttgacgatggtgcagaccgccggcatgtccgcctcggtggcacggcggatgtcggccgggcgtcgttctgggctcatggtaattgtaaa
tagtaattgtaatgttgtttgttgtttgttgttgttggtaattgttgtaaaaatagtcgagttgagagtgaatatgagactctaattggataccgaggggaattt
atggaacgtcagtggagcatttttgacaagaaatatttgctagctgatagtgaccttaggcgacttttgaacgcgcaataatggtttctgacgtatgtgct
tagctcattaaactccagaaacccgcggctcagtggctccttcaacgttgcggttctgtcagttccaaacgtaaaacggcttgtcccgcgtcatcggc
gggggtcataacgtgactcccttaattctccgctcatgatccgaattccaatcccacaaaaatctgagcttaacagcacagttgctcctctcagagca
gaatcgggtattcaacaccctcatatcaactactacgttgtgtataacggtccacatgccggtatatacgatgactggggttgtacaaaggcggcaa
caaacggcgttcccggagttgcacacaagaaatttgccactattacagaggcaagagcagcagctgacgcgtacacaacaagtcagcaaaca
 
gacaggttgaacttcatccccaaaggagaagctcaactcaagcccaagagctttgctaaggccctaacaagcccaccaaagcaaaaagccca
ctggctcacgctaggaaccaaaaggcccagcagtgatccagccccaaaagagatctcctttgccccggagattacaatggacgatttcctctatctt
tacgatctaggaaggaagttcgaaggtgaaggtgacgacactatgttcaccactgataatgagaaggttagcctcttcaatttcagaaagaatgctg
acccacagatggttagagaggcctacgcagcaggtctcatcaagacgatctacccgagtaacaatctccaggagatcaaataccttcccaagaa
ggttaaagatgcagtcaaaagattcaggactaattgcatcaagaacacagagaaagacatatttctcaagatcagaagtactattccagtatggac
gattcaaggcttgcttcataaaccaaggcaagtaatagagattggagtctctaaaaaggtagttcctactgaatctaaggccatgcatggagtctaa
gattcaaatcgaggatctaacagaactcgccgtgaagactggcgaacagttcatacagagtcttttacgactcaatgacaagaagaaaatcttcgt
caacatggtggagcacgacactctggtctactccaaaaatgtcaaagatacagtctcagaagaccaaagggctattgagacttttcaacaaagga
taatttcgggaaacctcctcggattccattgcccagctatctgtcacttcatcgaaaggacagtagaaaaggaaggtggctcctacaaatgccatcat
tgcgataaaggaaaggctatcattcaagatctctctgccgacagtggtcccaaagatggacccccacccacgaggagcatcgtggaaaaagaa
gacgttccaaccacgtcttcaaagcaagtggattgatgtgacatctccactgacgtaagggatgacgcacaatcccactatccttcgcaagaccctt
cctctatataaggaagttcatttcatttggagaggacacgctcgagtataagagctcattttacaacaattaccaacaacaacaaacaacaaacaa
cattacaattacatttacaattatcgataccatggtgagcaagggcgaggagctgttcaccggggtggtgcccatcctggtcgagctggacggcga
cgtgaacggccacaagttcagcgtgtccggcgagggcgagggcgatgccacctacggcaagctgaccctgaagttcatctgcaccaccggcaa
gctgcccgtgccctggcccaccctcgtgaccaccttcagctacggcgtgcagtgcttcagccgctaccccgaccacatgaagcagcacgacttctt
caagtccgccatgcccgaaggctacgtccaggagcgcaccatcttcttcaaggacgacggcaactacaagacccgcgccgaggtgaagttcga
gggcgacaccctggtgaaccgcatcgagctgaagggcatcgacttcaaggaggacggcaacatcctggggcacaagctggagtacaactaca
acagccacaacgtctatatcatggccgacaagcagaagaacggcatcaaggtgaacttcaagatccgccacaacatcgaggacggcagcgtg
cagctcgccgaccactaccagcagaacacccccatcggcgacggccccgtgctgctgcccgacaaccactacctgagcacccagtccgccct
gagcaaagaccccaacgagaagcgcgatcacatggtcctgctggagttcgtgaccgccgccgggatcactcacggcatggacgagctgtaca
agtaaagcggatcctctagagtcaagcagatcgttcaaacatttggcaataaagtttcttaagattgaatcctgttgccggtcttgcgatgattatcatat
aatttctgttgaattacgttaagcatgtaataattaacatgtaatgcatgacgttatttatgagatgggtttttatgattagagtcccgcaattatacatttaat
acgcgatagaaaacaaaatatagcgcgcaaactaggataaattatcgcgcgcggtgtcatctatgttactagatcgacctgcaggcatgcaagctt
agatcagattgtcgtttcccgccttcagtttaaactatcagtgtttgacaggatatattggcgggtaaacctaagagaaaagagcgtttattagaataat
cggatatttaaaagggcgtgaaaaggtttatccgttcgtccatttgtatgtgcatgccaaccacagggttcccc
pNMD035 (Fig. 2) 
cctgtggttggcacatacaaatggacgaacggataaacctttcacgcccttttaaatatccgattattctaataaacgctcttttctcttaggtttacccgc
caatatatcctgtcaaacactgatagtttaaactgaaggcgggaaacgacaatctgatctaagctagcttggaattggtaccacgcgtttcgacaaa
atttagaacgaacttaattatgatctcaaatacattgatacatatctcatctagatctaggttatcattatgtaagaaagttttgacgaatatggcacgaca
aaatggctagactcgatgtaattggtatctcaactcaacattatacttataccaaacattagttagacaaaatttaaacaactatttttatgtatgcaaga
gtcagcatatgtataattgattcagaatcgttttgacgagttcggatgtagtagtagccattatttaatgtacatactaatcgtgaatagtgaatatgatga
aacattgtatcttattgtataaatatccataaacacatcatgaaagacactttctttcacggtctgaattaattatgatacaattctaatagaaaacgaatt
aaattacgttgaattgtatgaaatctaattgaacaagccaaccacgacgacgactaacgttgcctggattgactcggtttaagttaaccactaaaaaa
acggagctgtcatgtaacacgcggatcgagcaggtcacagtcatgaagccatcaaagcaaaagaactaatccaagggctgagatgattaattag
tttaaaaattagttaacacgagggaaaaggctgtctgacagccaggtcacgttatctttacctgtggtcgaaatgattcgtgtctgtcgattttaattatttt
ttgaaaggccgaaaataaagttgtaagagataaacccgcctatataaattcatatttcctctccgctttgaagttttagttttattgcaacaacaaca
acaaattacaataacaacaaacaaaatacaaacaacaacaacatggcacaatttcaacaaacaattgacatgcaaactctccaagccgctgcg
ggacgcaacagcttggtgaatgatttggcatctcgtcgcgtttacgataatgcagtcgaggagctgaatgctcgttccagacgtcccaaggtaatag
gaactttctggatctactttatttgctggatctcgatcttgtttctcaatttccttgagatctggaattcgtttaattggatctgtgaacctccactaaatctttg
gtttactagaatcgatctaagttgaccgatcagttagctcgattatagctaccagaatttggcttgaccttgatggagagatccatgttcatgttacctgg
gaaatgatttgtatatgtgaattgaaatctgaactgttgaagttagattgaatctgaacactgtcaatgttagattgaatctgaacactgtttaaggttagat
gaagtttgtgtatagattcttcgaaactttaggatttgtagtgtcgtacgttgaacagaaagctatttctgattcaatcagggtttattgactgtattgaactct
tttgtgtgtttgcaggtccacttctccaaggcagtgtctacggaacagaccctgattgcaacaaacgcatatccggagttcgagatttcctttactcata
cgcaatccgctgtgcactccttggccggaggccttcggtcacttgagttggagtatctcatgatgcaagttccgttcggttctctgacgtacgacatcgg
cggtaacttttccgcgcaccttttcaaagggcgcgattacgttcactgctgcatgcctaatctggatgtacgtgacattgctcgccatgaaggacacaa
ggaagctatttacagttatgtgaatcgtttgaaaaggcagcagcgtcctgtgcctgaataccagagggcagctttcaacaactacgctgagaaccc
gcacttcgtccattgcgacaaacctttccaacagtgtgaattgacgacagcgtatggcactgacacctacgctgtagctctccatagcatttatgatat
ccctgttgaggagttcggttctgcgctactcaggaagaatgtgaaaacttgtttcgcggcctttcatttccatgagaatatgcttctagattgtgatacagt
cacactcgatgagattggagctacgttccagaaatcaggtaacattccttagttacctttcttttctttttccatcataagtttatagattgtacatgctttgag
atttttctttgcaaacaatctcaggtgataacctgagcttcttcttccataatgagagcactctcaattacacccacagcttcagcaacatcatcaagtac
gtgtgcaagacgttcttccctgctagtcaacgcttcgtgtaccacaaggagttcctggtcactagagtcaacacttggtactgcaagttcacgagagtg
gatacgttcactctgttccgtggtgtgtaccacaacaatgtggattgcgaagagttttacaaggctatggacgatgcgtggcactacaaaaagacgtt
agcaatgcttaatgccgagaggaccatcttcaaggataacgctgcgttaaacttctggttcccgaaggtgctcttgaaattggaagtcttcttttgttgtct
aaacctatcaatttcttgcggaaatttatttgaagctgtagagttaaaattgagtcttttaaacttttgtaggtgagagacatggttatcgtccctctctttga
cgcttctatcacaactggtaggatgtctaggagagaggttatggtgaacaaggacttcgtctacacggtcctaaatcacatcaagacctatcaagct
aaggcactgacgtacgcaaacgtgctgagcttcgtggagtctattaggtctagagtgataattaacggtgtcactgccaggtaagttgttacttatgatt
gttttcctctctgctacatgtatttgttgttcattctgtaagatataagaattgagtttcctctgatgatattattaggtctgaatgggacacagacaaggca
attctaggtccattagcaatgacattcttcctgatcacgaagctgggtcatgtgcaagatgaaataatcctgaaaaagttccagaagttcgacagaac
caccaatgagctgatttggacaagtctctgcgatgccctgatggggggttattccctcggtcaaggagacgcttgtgcgcggtggttttgtgaaagtagc
agaacaagccttagagatcaaggttagtatcatatgaagaaatacctagtttcagttgatgaatgctattttctgacctcagttgttctcttttgagaattatt
 
tcttttctaatttgcctgatttttctattaattcattaggttcccgagctatactgtaccttcgccgaccgattggtactacagtacaagaaggcggaggagtt
ccaatcgtgtgatctttccaaacctctagaagagtcagagaagtactacaacgcattatccgagctatcagtgcttgagaatctcgactcttttgactta
gaggcgtttaagactttatgtcagcagaagaatgtggacccggatatggcagcaaaggtaaatcctggtccacacttttacgataaaaacacaag
atttaaactatgaactgatcaataatcattcctaaaagaccacactttgtttgttctaaagtaatttttactgttataacaggtggtcgtagcaatcatga
agtcagaattgacgttgcctttcaagaaacctacagaagaggaaatctcggagtcgctaaaaccaggagaggggtcgtgtgcagagcataagg
aagtgttgagcttacaaaatgatgctccgttcccgtgtgtgaaaaatctagttgaaggttccgtgccggcgtatggaatgtgtcctaagggtggtggttt
cgacaaattggatgtggacattgctgatttccatctcaagagtgtagatgcagttaaaaaggaactatgatgtctgcggtgtacacagggtctatca
aagttcaacaaatgaagaactacatagattacttaagtgcgtcgctggcagctacagtctcaaacctctgcaaggtaagaggtcaaaaggtttccg
caatgatccctctttttttgtttctctagtttcaagaatttgggtatatgactaacttctgagtgttccttgatgcatatttgtgatgagacaaatgtttgttctatgtt
ttaggtgcttagagatgttcacggcgttgacccagagtcacaggagaaatctggagtgtgggatgttaggagaggacgttggttacttaaacctaatg
cgaaaagtcacgcgtggggtgtggcagaagacgccaaccacaagttggttattgtgttactcaactgggatgacggaaagccggtttgtgatgag
acatggttcagggtggcggtgtcaagcgattccttgatatattcggatatgggaaacttaagacgctcacgtcttgcagtccaaatggtgagccacc
ggagcctaacgccaaagtaatttggtcgatggtgttcccggttgtggaaaaacgaaggagattatcgaaaaggtaagttctgcatttggttatgctcc
ttgcatttaggtgttcgtcgctcttccatttccatgaatagctaagatttttttctctgcattcattcttcttgcctcagttctaactgtttgtggtattttttgttttaatt
attgctacaggtaaacttctctgaagacttgatttagtccctgggaaggaagcttctaagatgatcatccggagggccaaccaagctggtgtgataa
gagcggataaggacaatgttagaacggtggattccttcttgatgcatccttctagaagggtgtttaagaggttgtttatcgatgaaggactaatgctgca
tacaggttgtgtaaatttcctactgctgctatctcaatgtgacgtcgcatatgtgtatggggacacaaagcaaattccgttcattgcagagtcgcgaact
ttccgtatccagcgcattttgcaaaactcgtcgctgatgagaaggaagtcagaagagttacgctcaggtaaagcaactgtgtttaatcaatttcttgtc
aggatatatggattataacttaattttgagaaatctgtagtatttggcgtgaaatgagtttgctttttggtttctcccgtgttataggtgcccggctgatgttac
gtatttccttaacaagaagtatgacggggcggtgatgtgtaccagcgcggtagagagatccgtgaaggcagaagtggtgagaggaaagggtgca
ttgaacccaataaccttaccgttggagggtaaaatttgaccttcacacaagctgacaagttcgagttactggagaagggttacaaggtaaagtttcc
aactttcctttaccatatcaaactaaagttcgaaacttttattgatcaacttcaaggccacccgatctttctattcctgattaatttgtgatgaatccatattg
acttttgatggttacgcaggatgtgaacactgtgcacgaggtgcaaggggagacgtacgagaagactgctattgtgcgcttgacatcaactccgtta
gagatcatatcgagtgcgtcacctcatgttttggtggcgctgacaagacacacaacgtgttgtaaatattacaccgttgtgttggacccgatggtgaat
gtgatttcagaaatggagaagttgtccaatttccttcttgacatgtatagagttgaagcaggtctgtctttcctatttcatatgtttaatcctaggaatttgatc
aattgattgtatgtatgtcgatcccaagactttcttgttcacttatatcttaactctctctttgctgtttcttgcaggtgtccaatagcaattacaaatcgatgca
gtattcaggggacagaacttgtttgttcagacgcccaagtcaggagattggcgagatatgcaattttactatgacgctcttcttcccggaaacagtact
attctcaatgaatttgatgctgttacgatgaattgagggatattccttaaacgtcaaagattgcagaatcgacttctccaaatccgtgcaacttcctaaa
gaacaacctatttcctcaagcctaaaataagaactgcggcagaaatgccgagaactgcaggtaaaatattggatgccagacgatattctttctttg
atttgtaactttttcctgtcaaggtcgataaattttatttttttggtaaaaggtcgataattttttttggagccattatgtaatttcctaattaactgaaccaaaat
tatacaaaccaggtttgctggaaaattggttgcaatgatcaaaagaaacatgaatgcgccggatttgacagggacaattgacattgaggatactgc
atctctggtggttgaaaagttttgggattcgtatgttgacaaggaatttagtggaacgaacgaaatgaccatgacaagggagagcttctccaggtaag
gacttctcatgaatattagtggcagattagtgttgttaaagtctttggttagataatcgatgcctcctaattgtccatgttttactggttttctacaattaaaggt
ggctttcgaaacaagagtcatctacagttggtcagttagcggactttaactttgtggatttgccggcagtagatgagtacaagcatatgatcaagagtc
aaccaaagcaaaagttagacttgagtattcaagacgaatatcctgcattgcagacgatagtctaccattcgaaaaagatcaatgcgattttcggtcc
aatgttttcagaacttacgaggatgttactcgaaaggattgactcttcgaagtttctgttctacaccagaaagacacctgcacaaatagaggacttctttt
ctgacctagactcaacccaggcgatggaaattctggaactcgacatttcgaagtacgataagtcacaaaacgagttccattgtgctgtagagtaca
agatctgggaaaagttaggaattgatgagtggctagctgaggtctggaaacaaggtgagttcctaagttccatttttttgtaatccttcaatgttattttaac
ttttcagatcaacatcaaaattaggttcaatttcatcaaccaaataatatttttcatgtatatataggtcacagaaaaacgaccttgaaagattatacgg
ccggaatcaaaacatgtctttggtatcaaaggaaaagtggtgatgtgacaacctttattggtaataccatcatcattgccgcatgtttgagctcaatgat
ccccatggacaaagtgataaaggcagctttttgtggagacgatagcctgatttacattcctaaaggtttagacttgcctgatattcaggcgggcgcga
acctcatgtggaacttcgaggccaaactcttcaggaagaagtatggttacttctgtggtcgttatgttattcaccatgatagaggagccattgtgtattac
gatccgcttaaactaatatctaagttaggttgtaaacatattagagatgttgttcacttagaagagttacgcgagtctttgtgtgatgtagctagtaacttaa
ataattgtgcgtatttttcacagttagatgaggccgttgccgaggttcataagaccgcggtaggcggttcgtttgctttttgtagtataattaagtatttgtca
gataagagattgtttagagattgttctttgtttgataatgtcgatagtctcgtacgaacctaaggtgagtgatttcctcaatctttcgaagaaggaagaga
tcttgccgaaggctctaacgaggttaaaaaccgtgtctattagtactaaagatattatatctgtcaaggagtcggagactttgtgtgatatagattgttaa
tcaatgtgccattagataagtatagatatgtgggtatcctaggagccgtttttaccggagagtggctagtgccagacttcgttaaaggtggagtgacga
taagtgtgatagataagcgtctggtgaactcaaaggagtgcgtgattggtacgtacagagccgcagccaagagtaagaggttccagttcaaattgg
ttccaaattactttgtgtccaccgtggacgcaaagaggaagccgtggcaggtaaggattttatgatatagtatgcttatgtatttgtactgaaagcatat
cctgcttcattgggatattactgaaagcatttaactacatgtaaactcacttgatgatcaataaacttgattttgcaggttcatgttcgtatacaagacttga
agattgaggcgggttggcagccgttagctctggaagtagtttcagttgctatggtcaccaataacgttgtcatgaagggtttgagggaaaaggtcgtc
gcaataaatgatccggacgtcgaaggtttcgaaggtaagccatcttcctgcttatttttataatgaacatagaaataggaagttgtgcagagaaacta
attaacctgactcaaaatctaccctcataattgttgtttgatattggtcttgtatttgcaggtgtggttgacgaattcgtcgattcggttgcagcatttaaagc
ggttgacaactttaaaagaaggaaaaagaaggttgaagaaaagggtgtagtaagtaagtataagtacagaccggagaagtacgccggtcctga
ttcgtttaatttgaaagaagaaaacgtcttacaacattacaaacccgaatcagtaccagtatttcgataagaaacaagaaaccatgagagacctga
tatccacaaccgtggtctcgagcttactagagcgtggtgcgcacgatagcgcatagtgttttctctccacttgaatcgaagagatagacttacggtgt
aaatccgtaggggtggcgtaaaccaaattacgcaatgttttgggttccatttaaatcgaaaccccttatttcctggatcacctgttaacgcacgtttgac
gtgtattacagtgggaataagtaaaagtgagaggttcgaatcctccctaaccccgggtaggggcccagcggccgctctagctagagtcaagcag
atcgttcaaacatttggcaataaagtttcttaagattgaatcctgttgccggtcttgcgatgattatcatataatttctgttgaattacgttaagcatgtaataa
ttaacatgtaatgcatgacgttatttatgagatgggtttttatgattagagtcccgcaattatacatttaatacgcgatagaaaacaaaatatagcgcgca
aactaggataaattatcgcgcgcggtgtcatctatgttactagatcgacctgcatccaccccagtacattaaaaacgtccgcaatgtgttattaagttgt
 
ctaagcgtcaatttgtttacaccacaatatatcctgccaccagccagccaacagctccccgaccggcagctcggcacaaaatcaccactcgatac
aggcagcccatcagtcagatcaggatctcctttgcgacgctcacc
pNMD661 (Fig. 3) 
cctgtggttggcacatacaaatggacgaacggataaaccttttcacgcccttttaaatatccgattattctaataaacgctcttttctcttaggtttacccgc
caatatatcctgtcaaacactgatagtttaaactgaaggcgggaaacgacaatctgatctaagctagcttggaattggtaccacgcgtttcgacaaa
atttagaacgaacttaattatgatctcaaatacattgatacatatctcatctagatctaggttatcattatgtaagaaagttttgacgaatatggcacgaca
aaatggctagactcgatgtaattggtatctcaactcaacattatacttataccaaacattagttagacaaaatttaaacaactatttttatgtatgcaaga
gtcagcatatgtataattgattcagaatcgttttgacgagttcggatgtagtagtagccattatttaatgtacatactaatcgtgaatagtgaatatgatga
aacattgtatcttattgtataaatatccataaacacatcatgaaagacactttcttcacggtctgaattaattatgatacaattctaatagaaaacgaatt
aaattacgttgaattgtatgaaatctaattgaacaagccaaccacgacgacgactaacgttgcctggattgactcggtttaagttaaccactaaaaa
acggagctgtcatgtaacacgcggatcgagcaggtcacagtcatgaagccatcaaagcaaaagaactaatccaagggctgagatgattaattag
tttaaaaattagttaacacgagggaaaaggctgtctgacagccaggtcacgttatctttacctgtggtcgaaatgattcgtgtctgtcgattttaattattttt
ttgaaaggccgaaaataaagttgtaagagataaacccgcctatataaattcatatatttcctctccgctttgaagttttagttttattgcaacaacaaca
acaaattacaataacaacaaacaaaatacaaacaacaacaacatggcacaatttcaacaaacaattgacatgcaaactctccaagccgctgcg
ggacgcaacagcttggtgaatgattggcatctcgtcgcgtttacgataatgcagtcgaggagctgaatgctcgttccagacgtcccaaggtaatag
gaactttctggatctactttattgctggatctcgatcttgttttctcaatttccttgagatctggaattcgtttaatttggatctgtgaacctccactaaatcttttg
gttttactagaatcgatctaagttgaccgatcagttagctcgattatagctaccagaatttggcttgaccttgatggagagatccatgttcatgttacctgg
gaaatgatttgtatatgtgaattgaaatctgaactgttgaagttagattgaatctgaacactgtcaatgttagattgaatctgaacactgtttaaggttagat
gaagtttgtgtatagattcttcgaaactttaggatttgtagtgtcgtacgttgaacagaaagctatttctgattcaatcagggtttatttgactgtattgaactct
tttgtgtgtttgcaggtccacttctccaaggcagtgtctacggaacagaccctgattgcaacaaacgcatatccggagttcgagatttcctttactcata
cgcaatccgctgtgcactccttggccggaggccttcggtcacttgagttggagtatctcatgatgcaagttccgttcggttctctgacgtacgacatcgg
cggtaacttttccgcgcacctttcaaagggcgcgattacgttcactgctgcatgcctaatctggatgtacgtgacattgctcgccatgaaggacacaa
ggaagctatttacagttatgtgaatcgtttgaaaaggcagcagcgtcctgtgcctgaataccagagggcagctttcaacaactacgctgagaaccc
gcacttcgtccattgcgacaaacctttccaacagtgtgaattgacgacagcgtatggcactgacacctacgctgtagctctccatagcatttatgatat
ccctgttgaggagttcggttctgcgctactcaggaagaatgtgaaaacttgttcgcggcctttcatttccatgagaatatgcttctagattgtgatacagt
cacactcgatgagattggagctacgttccagaaatcaggtaacattccttagttacctttcttttctttttccatcataagtttatagattgtacatgctttgag
atttttcttgcaaacaatctcaggtgataacctgagcttcttcttccataatgagagcactctcaattacacccacagcttcagcaacatcatcaagtac
gtgtgcaagacgttcttccctgctagtcaacgcttcgtgtaccacaaggagttcctggtcactagagtcaacacttggtactgcaagttcacgagagtg
gatacgttcactctgttccgtggtgtgtaccacaacaatgtggattgcgaagagtttacaaggctatggacgatgcgtggcactacaaaaagacgtt
agcaatgcttaatgccgagaggaccatcttcaaggataacgctgcgttaaacttctggttcccgaaggtgctcttgaaattggaagtcttcttttgttgtct
aaacctatcaatttctttgcggaaatttatttgaagctgtagagttaaaattgagtcttttaaacttttgtaggtgagagacatggttatcgtccctctctttga
cgcttctatcacaactggtaggatgtctaggagagaggttatggtgaacaaggacttcgtctacacggtcctaaatcacatcaagacctatcaagct
aaggcactgacgtacgcaaacgtgctgagcttcgtggagtctattaggtctagagtgataattaacggtgtcactgccaggtaagttgttacttatgatt
gtttcctctctgctacatgtatttgttgttcatttctgtaagatataagaattgagtttcctctgatgatattattaggtctgaatgggacacagacaaggca
attctaggtccattagcaatgacattcttcctgatcacgaagctgggtcatgtgcaagatgaaataatcctgaaaaagttccagaagttcgacagaac
caccaatgagctgatttggacaagtctctgcgatgccctgatggggttattccctcggtcaaggagacgcttgtgcgcggtggttttgtgaaagtagc
agaacaagccttagagatcaaggttagtatcatatgaagaaatacctagtttcagttgatgaatgctattttctgacctcagttgttctcttttgagaattatt
tcttttctaatttgcctgattttctattaattcattaggttcccgagctatactgtaccttcgccgaccgattggtactacagtacaagaaggcggaggagtt
ccaatcgtgtgatctttccaaacctctagaagagtcagagaagtactacaacgcattatccgagctatcagtgcttgagaatctcgactcttttgactta
gaggcgtttaagactttatgtcagcagaagaatgtggacccggatatggcagcaaaggtaaatcctggtccacacttttacgataaaaacacaag
attttaaactatgaactgatcaataatcattcctaaaagaccacacttttgttttgtttctaaagtaatttttactgttataacaggtggtcgtagcaatcatga
agtcagaattgacgttgcctttcaagaaacctacagaagaggaaatctcggagtcgctaaaaccaggagaggggtcgtgtgcagagcataagg
aagtgttgagcttacaaaatgatgctccgttcccgtgtgtgaaaaatctagttgaaggttccgtgccggcgtatggaatgtgtcctaagggtggtggttt
cgacaaattggatgtggacattgctgatttccatctcaagagtgtagatgcagttaaaaaggaactatgatgtctgcggtgtacacagggtctatca
aagttcaacaaatgaagaactacatagattacttaagtgcgtcgctggcagctacagtctcaaacctctgcaaggtaagaggtcaaaaggtttccg
caatgatccctctttttttgtttctctagtttcaagaatttgggtatatgactaacttctgagtgttccttgatgcatatttgtgatgagacaaatgttgttctatgtt
ttaggtgcttagagatgttcacggcgttgacccagagtcacaggagaaatctggagtgtgggatgttaggagaggacgttggttacttaaacctaatg
cgaaaagtcacgcgtggggtgtggcagaagacgccaaccacaagttggttattgtgttactcaactgggatgacggaaagccggtttgtgatgag
acatggttcagggtggcggtgtcaagcgattccttgatatattcggatatgggaaacttaagacgctcacgtcttgcagtccaaatggtgagccacc
ggagcctaacgccaaagtaattttggtcgatggtgttcccggttgtggaaaaacgaaggagattatcgaaaaggtaagttctgcattggttatgctcc
ttgcattttaggtgttcgtcgctcttccatttccatgaatagctaagatttttttctctgcattcattcttcttgcctcagttctaactgtttgtggtatttttgttttaatt
attgctacaggtaaacttctctgaagacttgattttagtccctgggaaggaagcttctaagatgatcatccggagggccaaccaagctggtgtgataa
gagcggataaggacaatgttagaacggtggattccttcttgatgcatccttctagaagggtgtttaagaggttgtttatcgatgaaggactaatgctgca
tacaggttgtgtaaatttcctactgctgctatctcaatgtgacgtcgcatatgtgtatggggacacaaagcaaattccgttcattgcagagtcgcgaact
ttccgtatccagcgcatttgcaaaactcgtcgctgatgagaaggaagtcagaagagttacgctcaggtaaagcaactgtgtttaatcaatttcttgtc
aggatatatggattataacttaattttgagaaatctgtagtatttggcgtgaaatgagtttgctttttggtttctcccgtgttataggtgcccggctgatgttac
gtatttccttaacaagaagtatgacggggcggtgatgtgtaccagcgcggtagagagatccgtgaaggcagaagtggtgagaggaaagggtgca
ttgaacccaataaccttaccgttggagggtaaaattttgaccttcacacaagctgacaagttcgagttactggagaagggttacaaggtaaagtttcc
aactttcctttaccatatcaaactaaagttcgaaactttttattgatcaacttcaaggccacccgatctttctattcctgattaattgtgatgaatccatattg
actttgatggttacgcaggatgtgaacactgtgcacgaggtgcaaggggagacgtacgagaagactgctattgtgcgcttgacatcaactccgtta
 
gagatcatatcgagtgcgtcacctcatgttttggtggcgctgacaagacacacaacgtgttgtaaatattacaccgttgtgttggacccgatggtgaat
gtgatttcagaaatggagaagttgtccaatttccttcttgacatgtatagagttgaagcaggtctgtctttcctatttcatatgtttaatcctaggaatttgatc
aattgattgtatgtatgtcgatcccaagactttcttgttcacttatatcttaactctctctttgctgtttcttgcaggtgtccaatagcaattacaaatcgatgca
gtattcaggggacagaacttgtttgttcagacgcccaagtcaggagattggcgagatatgcaatttactatgacgctcttcttcccggaaacagtact
attctcaatgaatttgatgctgttacgatgaatttgagggatatttccttaaacgtcaaagattgcagaatcgacttctccaaatccgtgcaacttcctaaa
gaacaacctatttcctcaagcctaaaataagaactgcggcagaaatgccgagaactgcaggtaaaatattggatgccagacgatattctttctttg
atttgtaacttttcctgtcaaggtcgataaatttatttttttggtaaaaggtcgataatttttttttggagccattatgtaatttcctaattaactgaaccaaaat
tatacaaaccaggtttgctggaaaattggttgcaatgatcaaaagaaacatgaatgcgccggatttgacagggacaattgacattgaggatactgc
atctctggtggttgaaaagttttgggattcgtatgttgacaaggaatttagtggaacgaacgaaatgaccatgacaagggagagcttctccaggtaag
gacttctcatgaatattagtggcagattagtgttgttaaagtctttggttagataatcgatgcctcctaattgtccatgttttactggttttctacaattaaaggt
ggctttcgaaacaagagtcatctacagttggtcagttagcggactttaactttgtggattgccggcagtagatgagtacaagcatatgatcaagagtc
aaccaaagcaaaagttagacttgagtattcaagacgaatatcctgcattgcagacgatagtctaccattcgaaaaagatcaatgcgatttcggtcc
aatgttttcagaacttacgaggatgttactcgaaaggattgactcttcgaagtttctgttctacaccagaaagacacctgcacaaatagaggacttctttt
ctgacctagactcaacccaggcgatggaaattctggaactcgacatttcgaagtacgataagtcacaaaacgagttccattgtgctgtagagtaca
agatctgggaaaagttaggaattgatgagtggctagctgaggtctggaaacaaggtgagttcctaagttccatttttttgtaatccttcaatgttattttaac
ttttcagatcaacatcaaaattaggttcaattttcatcaaccaaataatatttttcatgtatatataggtcacagaaaaacgaccttgaaagattatacgg
ccggaatcaaaacatgtctttggtatcaaaggaaaagtggtgatgtgacaacctttattggtaataccatcatcattgccgcatgtttgagctcaatgat
ccccatggacaaagtgataaaggcagcttttgtggagacgatagcctgatttacattcctaaaggtttagacttgcctgatattcaggcgggcgcga
acctcatgtggaacttcgaggccaaactcttcaggaagaagtatggttacttctgtggtcgttatgttattcaccatgatagaggagccattgtgtattac
gatccgcttaaactaatatctaagttaggttgtaaacatattagagatgttgttcacttagaagagttacgcgagtctttgtgtgatgtagctagtaacttaa
ataattgtgcgtatttttcacagttagatgaggccgttgccgaggttcataagaccgcggtaggcggttcgtttgctttttgtagtataattaagtatttgtca
gataagagattgtttagagatttgttctttgtttgataatgtcgatagtctcgtacgaacctaaggtgagtgatttcctcaatctttcgaagaaggaagaga
tcttgccgaaggctctaacgaggttaaaaaccgtgtctattagtactaaagatattatatctgtcaaggagtcggagactttgtgtgatatagattgttaa
tcaatgtgccattagataagtatagatatgtgggtatcctagctaggagccgtttttaccggagagtggctagtgccagacttcgttaaaggtggagtg
acgataagtgtgatagataagcgtctggtgaactcaaaggagtgcgtgattggtacgtacagagccgcagccaagagtaagaggttccagttcaa
attggttccaaattactttgtgtccaccgtggacgcaaagaggaagccgtggcaggtaaggattttatgatatagtatgcttatgtatttgtactgaaag
catatcctgcttcattgggatattactgaaagcatttaactacatgtaaactcacttgatgatcaataaacttgattttgcaggttcatgttcgtatacaaga
cttgaagattgaggcgggttggcagccgttagctctggaagtagtttcagttgctatggtcaccaataacgttgtcatgaagggtttgagggaaaaggt
cgtcgcaataaatgatccggacgtcgaaggtttcgaaggtaagccatcttcctgcttatttttataatgaacatagaaataggaagttgtgcagagaa
actaattaacctgactcaaaatctaccctcataattgttgtttgatattggtcttgtatttgcaggtgtggttgacgaattcgtcgattcggttgcagcattta
aagcggttgacaactttaaaagaaggaaaaagaaggttgaagaaaagggtgtagtaagtaagtataagtacagaccggagaagtacgccggt
cctgattcgtttaatttgaaagaagaaaacgtcttacaacattacaaacccgaataatcgataactcgagtatttttacaacaattaccaacaacaac
aaacaacaaacaacattacaattacatttacaattatccatgtgagaccccacaaccgtggggtctcagatcagcggcccctagagcgtggtgcg
cacgatagcgcatagtgtttttctctccacttgaatcgaagagatagacttacggtgtaaatccgtaggggtggcgtaaaccaaattacgcaatgttttg
ggttccatttaaatcgaaaccccttatttcctggatcacctgttaacgcacgtttgacgtgtattacagtgggaataagtaaaagtgagaggttcgaatc
ctccctaaccccgggtaggggcccagcggccgctctagctagagtcaagcagatcgttcaaacatttggcaataaagtttcttaagattgaatcctgt
tgccggtcttgcgatgattatcatataatttctgttgaattacgttaagcatgtaataattaacatgtaatgcatgacgttatttatgagatgggtttttatgatt
agagtcccgcaattatacatttaatacgcgatagaaaacaaaatatagcgcgcaaactaggataaattatcgcgcgcggtgtcatctatgttactag
atcgacctgcatccaccccagtacattaaaaacgtccgcaatgtgttattaagttgtctaagcgtcaatttgtttacaccacaatatatcctgccaccag
ccagccaacagctccccgaccggcagctcggcacaaaatcaccactcgatacaggcagcccatcagtcagatcaggatctcctttgcga
pNMD670 (Fig. 4) 
cctgtggttggcacatacaaatggacgaacggataaaccttttcacgcccttttaaatatccgattattctaataaacgctcttttctcttaggtttacccgc
caatatatcctgtcaaacactgatagtttaaactgaaggcgggaaacgacaatctgatctaagctaggcatgcctgcaggtcaacatggtggagca
cgacacgcttgtctactccaaaaatatcaaagatacagtctcagaagaccaaagggcaattgagacttttcaacaaagggtaatatccggaaacc
tcctcggattccattgcccagctatctgtcactttattgtgaagatagtggaaaaggaaggtggctcctacaaatgccatcattgcgataaaggaaag
gccatcgttgaagatgcctctgccgacagtggtcccaaagatggacccccacccacgaggagcatcgtggaaaaagaagacgttccaaccac
gtcttcaaagcaagtggattgatgtgatatctccactgacgtaagggatgacgcacaatcccactatccttcgcaagacccttcctctatataaggaa
gttcatttcatttggagaggagaaaactaaaccatacaccaccaacacaaccaaacccaccacgcccaattgttacacacccgcttgaaaaaga
aagtttaacaaatggccaaggtgcgcgaggtttaccaatcttttacagactccaccacaaaaactctcatccaagatgaggcttatagaaacattcg
ccccatcatggaaaaacacaaactagctaacccttacgctcaaacggttgaagcggctaatgatctagaggggttcggcatagccaccaatccct
atagcattgaattgcatacacatgcagccgctaagaccatagagaataaacttctagaggtgcttggttccatcctaccacaagaacctgttacattt
atgtttcttaaacccagaaagctaaactacatgagaagaaacccgcggatcaaggacattttccaaaatgttgccattgaaccaagagacgtagc
caggtaccccaaggaaacaataattgacaaactcacagagatcacaacggaaacagcatacattagtgacactctgcacttcttggatccgagct
acatagtggagacattccaaaactgcccaaaattgcaaacattgtatgcgaccttagttctccccgttgaggcagcctttaaaatggaaagcactca
cccgaacatatacagcctcaaatacttcggagatggtttccagtatataccaggcaaccatggtggcggggcataccatcatgaattcgctcatcta
caatggctcaaagtgggaaagatcaagtggaggaccccaaggatagctttctcggacatctcaattacacgactgagcaggttgagatgcaca
cagtgacagtacagttgcaggaatcgttcgcggcaaaccacttgtactgcatcaggagaggagacttgctcacaccggaggtgcgcactttcggc
caacctgacaggtacgtgattccaccacagatcttcctcccaaaagttcacaactgcaagaagccgattctcaagaaaactatgatgcagctcttct
tgtatgttaggacagtcaaggtcgcaaaaaattgtgacattttgccaaagtcagacaattaattaaatcatctgacttggacaaatactctgctgtgga
actggtttacttagtaagctacatggagttccttgccgatttacaagctaccacctgcttctcagacacactttctggtggcttgctaacaaagacccttg
 
caccggtgagggcttggatacaagagaaaaagatgcagctgtttggtcttgaggactacgcgaagttagtcaaagcagttgatttccacccggtgg
atttttctttcaaagtggaaacttgggacttcagattccaccccttgcaagcgtggaaagccttccgaccaagggaagtgtcggatgtagaggaaatg
gaaagtttgttctcagatggggacctgcttgattgcttcacaagaatgccagcttatgcggtaaacgcagaggaagatttagctgcaatcaggaaaa
cgcccgagatggatgtcggtcaagaagttaaagagcctgcaggagacagaaatcaatactcaaaccctgcagaaactttcctcaacaagctcca
caggaaacacagtagggaggtgaaacaccaggccgcaaagaaagctaaacgcctagctgaaatccaggagtcaatgagagctgaaggtga
tgccgaaccaaatgaaataagcgggacgatgggggcaatacccagcaacgccgaacttcctggcacgaatgatgccagacaagaactcaca
ctcccaaccactaaacctgtccctgcaaggtgggaagatgcttcattcacagattctagtgtggaagaggagcaggttaaactccttggaaaagaa
accgttgaaacagcgacgcaacaagtcatcgaaggacttccttggaaacactggattcctcaattaaatgctgttggattcaaggcgctggaaattc
agagggataggagtggaacaatgatcatgcccatcacagaaatggtgtccgggctggaaaaagaggacttccctgaaggaactccaaaagag
ttggcacgagaattgttcgctatgaacagaagccctgccaccatccctttggacctgcttagagccagagactacggcagtgatgtaaagaacaa
gagaattggtgccatcacaaagacacaggcaacgagttggggcgaatacttgacaggaaagatagaaagcttaactgagaggaaagttgcga
cttgtgtcattcatggagctggaggttctggaaaaagtcatgccatccagaaggcattgagagaaattggcaagggctcggacatcactgtagtcct
gccgaccaatgaactgcggctagattggagtaagaaagtgcctaacactgagccctatatgttcaagacctctgaaaaggcgttaattgggggaa
caggcagcatagtcatctttgacgattactcaaaacttcctcccggttacatagaagccttagtctgtttctactctaaaatcaagctaatcattctaaca
ggagatagcagacaaagcgtctaccatgaaactgctgaggacgcctccatcaggcatttgggaccagcaacagagtacttctcaaaatactgcc
gatactatctcaatgccacacaccgcaacaagaaagatcttgcgaacatgcttggtgtctacagtgagagaacgggagtcaccgaaatcagcat
gagcgccgagttcttagaaggaatcccaactttggtaccctcggatgagaagagaaagctgtacatgggcaccgggaggaatgacacgttcaca
tacgctggatgccaggggctaactaagccgaaggtacaaatagtgttggaccacaacacccaagtgtgtagcgcgaatgtgatgtacacggcac
tttctagagccaccgataggattcacttcgtgaacacaagtgcaaattcctctgccttctgggaaaagttggacagcaccccttacctcaagactttcc
tatcagtggtgagagaacaagcactcagggagtacgagccggcagaggcagagccaattcaagagcctgagccccagacacacatgtgtgtc
gagaatgaggagtccgtgctagaagagtacaaagaggaactcttggaaaagtttgacagagagatccactctgaatcccatggtcattcaaactg
tgtccaaactgaagacacaaccattcagttgttttcgcatcaacaagcaaaagatgagactctcctctgggcgactatagatgcgcggctcaagac
cagcaatcaagaaacaaacttccgagaattcctgagcaagaaggacattggggacgttctgtttttaaactaccaaaaagctatgggtttacccaa
agagcgtattcctttttcccaagaggtctgggaagcttgtgcccacgaagtacaaagcaagtacctcagcaagtcaaagtgcaacttgatcaatgg
gactgtgagacagagcccagacttcgatgaaaataagattatggtattcctcaagtcgcagtgggtcacaaaggtggaaaaactaggtctaccca
agattaagccaggtcaaaccatagcagccttttaccagcagactgtgatgctttttggaactatggctaggtacatgcgatggttcagacaggctttcc
agccaaaagaagtcttcataaactgtgagacgacgccagatgacatgtctgcatgggccttgaacaactggaatttcagcagacctagcttggcta
atgactacacagctttcgaccagtctcaggatggagccatgttgcaatttgaggtgctcaaagccaaacaccactgcataccagaggaaatcattc
aggcatacatagatattaagactaatgcacagattttcctaggcacgttatcaattatgcgcctgactggtgaaggtcccacttttgatgcaaacactg
agtgcaacatagcttacacccatacaaagtttgacatcccagccggaactgctcaagtttatgcaggagacgactccgcactggactgtgttccag
aagtgaagcatagtttccacaggcttgaggacaaattactcctaaagtcaaagcctgtaatcacgcagcaaaagaagggcagttggcctgagtttt
gtggttggctgatcacaccaaaaggggtgatgaaagacccaattaagctccatgttagcttaaaattggctgaagctaagggtgaactcaagaaat
gtcaagattcctatgaaattgatctgagttatgcctatgaccacaaggactctctgcatgacttgttcgatgagaaacagtgtcaggcacacacactc
acttgcagaacactaatcaagtcagggagaggcactgtctcactttcccgcctcagaaactttctttaaccgttaagttaccttagagatttgaataag
atgtcagcaccagctagtacaacacagcccatagggtcaactacctcaactaccacaaaaactgcaggcgcaactcctgccacagcttcaggc
ctgttcactatcccggatggggatttctttagtacagcccgtgccatagtagccagcaatgctgtcgcaacaaatgaggacctcagcaagattgagg
ctatttggaaggacatgaaggtgcccacagacactatggcacaggctgcttgggacttagtcagacactgtgctgatgtaggatcatccgctcaaa
cagaaatgatagatacaggtccctattccaacggcatcagcagagctagactggcagcagcaattaaagaggtgtgcacacttaggcaattttgc
atgaagtatgccccagtggtatggaactggatgttaactaacaacagtccacctgctaactggcaagcacaaggtttcaagcctgagcacaaattc
gctgcattcgacttcttcaatggagtcaccaacccagctgccatcatgcccaaagaggggctcatccggccaccgtctgaagctgaaatgaatgct
gcccaaactgctgcctttgtgaagattacaaaggccagggcacaatccaacgactttgccagcctagatgcagctgtcactcgaggaaggatcac
cggaacgaccacagcagaggcagtcgttactctgcctcctccataacagaaactttctttaaccgttaagttaccttagagatttgaataagatggat
attctcatcagtagtttgaaaagtttaggttattctaggacttccaaatctttagattcaggacctttggtagtacatgcagtagccggagccggtaagtcc
acagccctaaggaagttgatcctcagacacccaacattcaccgtgcatacactcggtgtccctgacaaggtgagtatcagaactagaggcataca
gaagccaggacctattcctgagggcaacttcgcaatcctcgatgagtatactttggacaacaccacaaggaactcataccaggcactttttgctgac
ccttatcaggcaccggagtttagcctagagccccacttctacttggaaacatcatttcgagttccgaggaaagtggcagatttgatagctggctgtgg
cttcgatttcgagacgaactcaccggaagaagggcacttagagatcactggcatattcaaagggcccctactcggaaaggtgatagccattgatg
aggagtctgagacaacactgtccaggcatggtgttgagtttgttaagccctgccaagtgacgggacttgagttcaaagtagtcactattgtgtctgccg
caccaatagaggaaattggccagtccacagctttctacaacgctatcaccaggtcaaagggattgacatatgtccgcgcagggccataggctgac
cgctccggtcaattctgaaaaagtgtacatagtattaggtctatcatttgctttagtttcaattacctttctgctttctagaaatagcttaccccacgtcggtg
acaacattcacagcttgccacacggaggagcttacagagacggcaccaaagcaatcttgtacaactccccaaatctagggtcacgagtgagtct
acacaacggaaagaacgcagcatttgctgccgtttgctactgactttgctgatctatggaagtaaatacatatctcaacgcaatcatacttgtgcttgt
ggtaacaatcatagcagtcattagcacttccttagtgaggactgaaccttgtgtcatcaagattactggggaatcaatcacagtgttggcttgcaaact
agatgcagaaaccataagggccattgccgatctcaagccactctccgttgaacggttaagtttccattgatactcgaaagaggtcagcaccagcta
gcaacaaacaagaacatgagagacctcgcgatttaaatcgatggtctcagatcggtcgtatcactggaacaacaaccgctgaggctgttgtcact
ctaccaccaccataactacgtctacataaccgacgcctaccccagtttcatagtattttctggtttgattgtatgaataatataaataaaaaaaaaaaa
aaaaaaaaaaaactagtgagctcttctgtcagcgggcccactgcatccaccccagtacattaaaaacgtccgcaatgtgttattaagttgtctaagc
gtcaatttgtttacacccacaatatatcctgccaccagccagccaacagctccccgaccggcagctcggcacaaaatcaccactcgatacaggca
gcccatcagtc
pNMD694 (Fig. 5) 
cctgtggttggcacatacaaatggacgaacggataaaccttttcacgcccttttaaatatccgattattctaataaacgctcttttctcttaggtttacccgc
caatatatcctgtcaaacactgatagtttaaactgaaggcgggaaacgacaatctgatctaagctaggcatgcctgcaggtcaacatggtggagca
cgacacgcttgtctactccaaaaatatcaaagatacagtctcagaagaccaaagggcaattgagacttttcaacaaagggtaatatccggaaacc
tcctcggattccattgcccagctatctgtcactttattgtgaagatagtggaaaaggaaggtggctcctacaaatgccatcattgcgataaaggaaag
gccatcgttgaagatgcctctgccgacagtggtcccaaagatggacccccacccacgaggagcatcgtggaaaaagaagacgttccaaccac
gtcttcaaagcaagtggattgatgtgatatctccactgacgtaagggatgacgcacaatcccactatccttcgcaagacccttcctctatataaggaa
gttcatttcatttggagaggagaaaactaaaccatacaccaccaacacaaccaaacccaccacgcccaattgttacacacccgcttgaaaaaga
aagtttaacaaatggccaaggtgcgcgaggtttaccaatcttttacagactccaccacaaaaactctcatccaagatgaggcttatagaaacattcg
ccccatcatggaaaaacacaaactagctaacccttacgctcaaacggttgaagcggctaatgatctagaggggttcggcatagccaccaatccct
atagcattgaattgcatacacatgcagccgctaagaccatagagaataaacttctagaggtgcttggttccatcctaccacaagaacctgttacattt
atgtttcttaaacccagaaagctaaactacatgagaagaaacccgcggatcaaggacatttccaaaatgttgccattgaaccaagagacgtagc
caggtaccccaaggaaacaataattgacaaactcacagagatcacaacggaaacagcatacattagtgacactctgcacttcttggatccgagct
acatagtggagacattccaaaactgcccaaaattgcaaacattgtatgcgaccttagttctccccgttgaggcagcctttaaaatggaaagcactca
cccgaacatatacagcctcaaatacttcggagatggtttccagtatataccaggcaaccatggtggcggggcataccatcatgaattcgctcatcta
caatggctcaaagtgggaaagatcaagtggagggaccccaaggatagctttctcggacatctcaattacacgactgagcaggttgagatgcaca
cagtgacagtacagttgcaggaatcgttcgcggcaaaccacttgtactgcatcaggagaggagacttgctcacaccggaggtgcgcactttcggc
caacctgacaggtacgtgattccaccacagatcttcctcccaaaagttcacaactgcaagaagccgattctcaagaaaactatgatgcagctcttct
tgtatgttaggacagtcaaggtcgcaaaaaattgtgacattttgccaaagtcagacaattaattaaatcatctgacttggacaaatactctgctgtgga
actggtttacttagtaagctacatggagttccttgccgatttacaagctaccacctgcttctcagacacactttctggtggcttgctaacaaagaccctg
caccggtgagggcttggatacaagagaaaaagatgcagctgtttggtcttgaggactacgcgaagttagtcaaagcagttgatttccacccggtgg
atttttctttcaaagtggaaacttgggacttcagattccaccccttgcaagcgtggaaagccttccgaccaagggaagtgtcggatgtagaggaaatg
gaaagtttgttctcagatggggacctgcttgattgcttcacaagaatgccagcttatgcggtaaacgcagaggaagatttagctgcaatcaggaaaa
cgcccgagatggatgtcggtcaagaagttaaagagcctgcaggagacagaaatcaatactcaaaccctgcagaaactttcctcaacaagctcca
caggaaacacagtagggaggtgaaacaccaggccgcaaagaaagctaaacgcctagctgaaatccaggagtcaatgagagctgaaggtga
tgccgaaccaaatgaaataagcgggacgatgggggcaatacccagcaacgccgaacttcctggcacgaatgatgccagacaagaactcaca
ctcccaaccactaaacctgtccctgcaaggtgggaagatgcttcattcacagattctagtgtggaagaggagcaggttaaactccttggaaaagaa
accgttgaaacagcgacgcaacaagtcatcgaaggacttccttggaaacactggattcctcaattaaatgctgttggattcaaggcgctggaaattc
agagggataggagtggaacaatgatcatgcccatcacagaaatggtgtccgggctggaaaaagaggacttccctgaaggaactccaaaagag
ttggcacgagaattgttcgctatgaacagaagccctgccaccatccctttggacctgcttagagccagagactacggcagtgatgtaaagaacaa
gagaattggtgccatcacaaagacacaggcaacgagttggggcgaatacttgacaggaaagatagaaagcttaactgagaggaaagttgcga
cttgtgtcattcatggagctggaggttctggaaaaagtcatgccatccagaaggcattgagagaaattggcaagggctcggacatcactgtagtcct
gccgaccaatgaactgcggctagattggagtaagaaagtgcctaacactgagccctatatgttcaagacctctgaaaaggcgttaattgggggaa
caggcagcatagtcatctttgacgattactcaaaacttcctcccggttacatagaagccttagtctgtttctactctaaaatcaagctaatcattctaaca
ggagatagcagacaaagcgtctaccatgaaactgctgaggacgcctccatcaggcattgggaccagcaacagagtacttctcaaaatactgcc
gatactatctcaatgccacacaccgcaacaagaaagatcttgcgaacatgcttggtgtctacagtgagagaacggagtcaccgaaatcagcat
gagcgccgagttcttagaaggaatcccaactttggtaccctcggatgagaagagaaagctgtacatgggcaccgggaggaatgacacgttcaca
tacgctggatgccaggggctaactaagccgaaggtacaaatagtgttggaccacaacacccaagtgtgtagcgcgaatgtgatgtacacggcac
tttctagagccaccgataggattcacttcgtgaacacaagtgcaaattcctctgccttctgggaaaagttggacagcaccccttacctcaagactttcc
tatcagtggtgagagaacaagcactcagggagtacgagccggcagaggcagagccaattcaagagcctgagccccagacacacatgtgtgtc
gagaatgaggagtccgtgctagaagagtacaaagaggaactcttggaaaagtttgacagagagatccactctgaatcccatggtcattcaaactg
tgtccaaactgaagacacaaccattcagttgttttcgcatcaacaagcaaaagatgagactctcctctgggcgactatagatgcgcggctcaagac
cagcaatcaagaaacaaacttccgagaattcctgagcaagaaggacattggggacgttctgttttaaactaccaaaaagctatgggtttacccaa
agagcgtattccttttcccaagaggtctgggaagcttgtgcccacgaagtacaaagcaagtacctcagcaagtcaaagtgcaacttgatcaatgg
gactgtgagacagagcccagacttcgatgaaaataagattatggtattcctcaagtcgcagtgggtcacaaaggtggaaaaactaggtctaccca
agattaagccaggtcaaaccatagcagcctttaccagcagactgtgatgctttttggaactatggctaggtacatgcgatggttcagacaggctttcc
agccaaaagaagtcttcataaactgtgagacgacgccagatgacatgtctgcatgggccttgaacaactggaatttcagcagacctagcttggcta
atgactacacagctttcgaccagtctcaggatggagccatgttgcaatttgaggtgctcaaagccaaacaccactgcataccagaggaaatcattc
aggcatacatagatattaagactaatgcacagattttcctaggcacgttatcaattatgcgcctgactggtgaaggtcccacttttgatgcaaacactg
agtgcaacatagcttacacccatacaaagtttgacatcccagccggaactgctcaagtttatgcaggagacgactccgcactggactgtgttccag
aagtgaagcatagtttccacaggcttgaggacaaattactcctaaagtcaaagcctgtaatcacgcagcaaaagaagggcagttggcctgagtttt
gtggttggctgatcacaccaaaaggggtgatgaaagacccaattaagctccatgttagcttaaaattggctgaagctaagggtgaactcaagaaat
gtcaagattcctatgaaattgatctgagttatgcctatgaccacaaggactctctgcatgacttgttcgatgagaaacagtgtcaggcacacacactc
acttgcagaacactaatcaagtcagggagaggcactgtctcactttcccgcctcagaaactttctttaaccgttaagttaccttagagatttgaataag
atggatattctcatcagtagtttgaaaagtttaggttattctaggacttccaaatctttagattcaggaccctttggtagtacatgcagtagccggagccggt
aagtccacagccctaaggaagttgatcctcagacacccaacattcaccgtgcatacactcggtgtccctgacaaggtgagtatcagaactagagg
catacagaagccaggacctattcctgagggcaacttcgcaatcctcgatgagtatactttggacaacaccacaaggaactcataccaggcactttt
gctgacccttatcaggcaccggagtttagcctagagccccacttctacttggaaacatcatttcgagttccgaggaaagtggcagatttgatagctgg
ctgtggcttcgatttcgagacgaactcaccggaagaagggcacttagagatcactggcatattcaaagggcccctactcggaaaggtgatagcca
ttgatgaggagtctgagacaacactgtccaggcatggtgttgagtttgttaagccctgccaagtgacgggacttgagttcaaagtagtcactattgtgt
ctgccgcaccaatagaggaaattggccagtccacagctttctacaacgctatcaccaggtcaaagggattgacatatgtccgcgcaggcccatag
gctgaccgctccggtcaattctgaaaaagtgtacatagtattaggtctatcattgctttagtttcaattacctttctgctttctagaaatagcttaccccacg
 
tcggtgacaacattcacagcttgccacacggaggagcttacagagacggcaccaaagcaatcttgtacaactccccaaatctagggtcacgagt
gagtctacacaacggaaagaacgcagcatttgctgccgttttgctactgactttgctgatctatggaagtaaatacatatctcaacgcaatcatacttgt
gcttgtggtaacaatcatagcagtcattagcacttccttagtgaggactgaaccttgtgtcatcaagattactggggaatcaatcacagtgttggcttgc
aaactagatgcagaaaccataagggccattgccgatctcaagccactctccgttgaacggttaagtttccattgatactcgaaagaggtcagcacc
agctagcaacaaacaagaacatgagagacctcgcgatttaaatcgatggtctcagatcggtcgtatcactggaacaacaaccgctgaggctgttg
tcactctaccaccaccataactacgtctacataaccgacgcctaccccagtttcatagtattttctggtttgattgtatgaataatataaataaaaaaaa
aaaaaaaaaaaaaaaactagtgagctcttctgtcagcgggcccactgcatccaccccagtacattaaaaacgtccgcaatgtgttattaagttgtct
aagcgtcaatttgtttacaccacaatatatcctgccaccagccagccaacagctccccgaccggcagctcggcacaaaatcaccactcgataca
ggcagcccatc
pNMD1971 
cctgtggttggcacatacaaatggacgaacggataaaccttttcacgcccttttaaatatccgattattctaataaacgctcttttctcttaggtttacccgc
caatatatcctgtcaaacactgatagtttaaactgaaggcgggaaacgacaatctgatctaagctaggcatggaattccaatcccacaaaaatctg
agcttaacagcacagttgctcctctcagagcagaatcgggtattcaacaccctcatatcaactactacgttgtgtataacggtccacatgccggtatat
acgatgactggggttgtacaaaggcggcaacaaacggcgttcccggagttgcacacaagaaatttgccactattacagaggcaagagcagcag
ctgacgcgtacacaacaagtcagcaaacagacaggttgaacttcatccccaaaggagaagctcaactcaagcccaagagctttgctaaggccc
taacaagcccaccaaagcaaaaagcccactggctcacgctaggaaccaaaaggcccagcagtgatccagccccaaaagagatctcctttgcc
ccggagattacaatggacgatttcctctatctttacgatctaggaaggaagttcgaaggtgaaggtgacgacactatgttcaccactgataatgaga
aggttagcctcttcaatttcagaaagaatgctgacccacagatggttagagaggcctacgcagcaggtctcatcaagacgatctacccgagtaac
aatctccaggagatcaaataccttcccaagaaggttaaagatgcagtcaaaagattcaggactaattgcatcaagaacacagagaaagacatat
ttctcaagatcagaagtactattccagtatggacgattcaaggcttgcttcataaaccaaggcaagtaatagagattggagtctctaaaaaggtagtt
cctactgaatctaaggccatgcatggagtctaagattcaaatcgaggatctaacagaactcgccgtgaagactggcgaacagttcatacagagtct
tttacgactcaatgacaagaagaaaatcttcgtcaacatggtggagcacgacactctggtctactccaaaaatgtcaaagatacagtctcagaaga
ccaaagggctattgagacttttcaacaaaggataatttcgggaaacctcctcggattccattgcccagctatctgtcacttcatcgaaaggacagtag
aaaaggaaggtggctcctacaaatgccatcattgcgataaaggaaaggctatcattcaagatctctctgccgacagtggtcccaaagatggaccc
ccacccacgaggagcatcgtggaaaaagaagacgttccaaccacgtcttcaaagcaagtggattgatgtgacatctccactgacgtaagggatg
acgcacaatcccactatccttcgcaagacccttcctctatataaggaagttcatttcatttggagaggacacgctcgagtataagagctcattttacaa
caattaccaacaacaacaaacaacaaacaacattacaattacatttacaattatcgatgggtcagtcccttatgttacgtcctgtagaaaccccaac
ccgtgaaatcaaaaaactcgacggcctgtgggcattcagtctggatcgcgaaaactgtggaattgatcagcgttggtgggaaagcgcgttacaag
aaagccgggcaattgctgtgccaggcagttttaacgatcagttcgccgatgcagatattcgtaattatgcgggcaacgtctggtatcagcgcgaagt
ctttataccgaaaggtaagtagtgtttttggataactgagtttgcctatgattttgtatttactgagatgtttgtcctctttgtgcaggttgggcaggccagcgt
atcgtgctgcgtttcgatgcggtcactcattacggcaaagtgtgggtcaataatcaggaagtgatggagcatcagggcggctatacgccatttgaag
ccgatgtcacgccgtatgttattgccgggaaaagtgtacgtatcaccgtttgtgtgaacaacgaactgaactggcagactatcccgccgggaatggt
gattaccgacgaaaacggcaagaaaaagcagtcttacttccatgatttctttaactatgccggaatccatcgcagcgtaatgctctacaccacgccg
aacacctgggtggacgatatcaccgtggtgacgcatgtcgcgcaagactgtaaccacgcgtctgttgactggcaggtggtggccaatggtgatgtc
agcgttgaactgcgtgatgcggatcaacaggtggttgcaactggacaaggcactagcgggactttgcaagtggtgaatccgcacctctggcaacc
gggtgaaggttatctctatgaactgtgcgtcacagccaaaagccagacagagtgtgatatctacccgcttcgcgtcggcatccggtcagtggcagt
gaagggccaacagttcctgattaaccacaaaccgttctactttactggctttggtcgtcatgaagatgcggacttacgtggcaaaggattcgataacg
tgctgatggtgcacgaccacgcattaatggactggattggggccaactcctaccgtacctcgcattacccttacgctgaagagatgctcgactgggc
agatgaacatggcatcgtggtgattgatgaaactgctgctgtcggctttaacctctctttaggcattggtttcgaagcgggcaacaagccgaaagaac
tgtacagcgaagaggcagtcaacggggaaactcagcaagcgcacttacaggcgattaaagagctgatagcgcgtgacaaaaaccacccaag
cgtggtgatgtggagtattgccaacgaaccggatacccgtccgcaaggtgcacgggaatatttcgcgccactggcggaagcaacgcgtaaactc
gacccgacgcgtccgatcacctgcgtcaatgtaatgttctgcgacgctcacaccgataccatcagcgatctctttgatgtgctgtgcctgaaccgttatt
acggatggtatgtccaaagcggcgatttggaaacggcagagaaggtactggaaaaagaacttctggcctggcaggagaaactgcatcagccg
attatcatcaccgaatacggcgtggatacgttagccgggctgcactcaatgtacaccgacatgtggagtgaagagtatcagtgtgcatggctggata
tgtatcaccgcgtctttgatcgcgtcagcgccgtcgtcggtgaacaggtatggaatttcgccgattttgcgacctcgcaaggcatattgcgcgttggcg
gtaacaagaaagggatcttcactcgcgaccgcaaaccgaagtcggcggcttttctgctgcaaaaacgctggactggcatgaacttcggtgaaaa
accgcagcagggaggcaaacaatgaatcaacaactctcctggcgcaccatcgtcggctacagcctcgggaattgggatcctctagagtcaagc
agatcgttcaaacatttggcaataaagtttcttaagattgaatcctgttgccggtcttgcgatgattatcatataatttctgttgaattacgttaagcatgtaat
aattaacatgtaatgcatgacgttatttatgagatgggtttttatgattagagtcccgcaattatacatttaatacgcgatagaaaacaaaatatagcgcg
caaactaggataaattatcgcgcgcggtgtcatctatgttactagatcgacctgcaggcatgccaattccaatcccacaaaaatctgagcttaacag
cacagttgctcctctcagagcagaatcgggtattcaacaccctcatatcaactactacgttgtgtataacggtccacatgccggtatatacgatgactg
gggttgtacaaaggcggcaacaaacggcgttcccggagttgcacacaagaaatttgccactattacagaggcaagagcagcagctgacgcgta
cacaacaagtcagcaaacagacaggttgaacttcatccccaaaggagaagctcaactcaagcccaagagctttgctaaggccctaacaagcc
caccaaagcaaaaagcccactggctcacgctaggaaccaaaaggcccagcagtgatccagccccaaaagagatctcctttgccccggagatt
acaatggacgatttcctctatctttacgatctaggaaggaagttcgaaggtgaaggtgacgacactatgttcaccactgataatgagaaggttagcct
cttcaatttcagaaagaatgctgacccacagatggttagagaggcctacgcagcaggtctcatcaagacgatctacccgagtaacaatctccagg
agatcaaataccttcccaagaaggttaaagatgcagtcaaaagattcaggactaattgcatcaagaacacagagaaagacatatttctcaagatc
agaagtactattccagtatggacgattcaaggcttgcttcataaaccaaggcaagtaatagagattggagtctctaaaaaggtagttcctactgaatc
 
taaggccatgcatggagtctaagattcaaatcgaggatctaacagaactcgccgtgaagactggcgaacagttcatacagagtcttttacgactca
atgacaagaagaaaatcttcgtcaacatggtggagcacgacactctggtctactccaaaaatgtcaaagatacagtctcagaagaccaaagggc
tattgagacttttcaacaaaggataatttcgggaaacctcctcggattccattgcccagctatctgtcacttcatcgaaaggacagtagaaaaggaag
gtggctcctacaaatgccatcattgcgataaaggaaaggctatcattcaagatctctctgccgacagtggtcccaaagatggacccccacccacg
aggagcatcgtggaaaaagaagacgttccaaccacgtcttcaaagcaagtggattgatgtgacatctccactgacgtaaggatgacgcacaat
cccactatccttcgcaagacccttcctctatataaggaagttcatttcatttggagaggacacgctcgagtataagagctctatttttacaacaattacca
acaacaacaaacaacaaacaacattacaattacatttacaattaccatggaacgagctatacaaggaaacgatgctagggaacaagcttatggt
gaacgttggaatggaggatcaggaagttccacttctcccttcaaacttcctgacgaaagtccgagttggactgagtggcggctacataacgatgag
acgatttcgaatcaagataatccccttggtttcaaggaaagctggggtttcgggaaagttgtatttaagagatatctcagatacgacggacggaaa
cttcactgcacagagtccttggatcttggacgggagattcggttaactatgcagcatctcgatttctcggtttcgaccagatcggatgtacctatagtatt
cggtttcgaggagttagtgtcaccatttctggagggtcgcgaactcttcagcatctcagtgaaatggcaattcggtctaagcaagaactgctacagctt
accccagtcaaagtggaaagtgatgtatcaagaggatgccctgaaggtgttgaaaccttcgaagaagaaagcgagtaaggatcctctagagtcc
tgctttaatgagatatgcgagacgcctatgatcgcatgatatttgctttcaattctgttgtgcacgttgtaaaaaacctgagcatgtgtagctcagatcctta
ccgccggtttcggttcattctaatgaatatatcacccgttactatcgtatttttatgaataatattctccgttcaatttactgattgtaccctactacttatatgta
caatattaaaatgaaaacaatatattgtgctgaataggtttatagcgacatctatgatagagcgccacaataacaaacaattgcgttttattattacaaa
tccaattttaaaaaaagcggcagaaccggtcaaacctaaaagactgattacataaatcttattcaaatttcaaaagtgccccaggggctagtatcta
cgacacaccgagcggcgaactaataacgctcactgaagggaactccggttccccgccggcgcgcatgggtgagattccttgaagttgagtattgg
ccgtccgctctaccgaaagttacgggcaccattcaacccggtccagcacggcggccgggtaaccgacttgctgccccgagaattatgcagcatttt
tttggtgtatgtgggccccaaatgaagtgcaggtcaaaccttgacagtgacgacaaatcgttgggcgggtccagggcgaattttgcgacaacatgtc
gaggctcagcaggacctgcataagctcttctgtcagcgggcccactgcatccaccccagtacattaaaaacgtccgcaatgtgttattaagttgtcta
agcgtcaatttgtttacaccacaatatatcctgccaccagccagccaacagctccccgaccggcagctcggcacaaaatcaccactcgatacag
gcagcccatcag
pNMD2210 
cctgtggttggcacatacaatggacgaacggataaacctttcacgcccttttaaatatccgattattctaataaacgctcttttctcttaggtttacccgc
caatatatcctgtcaaacactgatagtttaaactgaaggcgggaaacgacaatctgatctaagctaggcatggaattgcgtgcaggtcgaggtcatt
catatgcttgagaagagagtcgggatagtccaaaataaaacaaaggtaagattacctggtcaaaagtgaaaacatcagttaaaaggtggtataa
agtaaaatatcggtaataaaaggtggcccaaagtgaaatttactcttttctactattataaaattgaggatgtttttgtcggtactttgatacgtcatttttgt
atgaattggtttttaagtttattcgcttttggaaatgcatatctgtatttgagtcgggttttaagttcgtttgcttttgtaaatacagagggatttgtataagaaata
tctttaaaaaacccatatgctaatttgacataatttttgagaaaaatatattcaggcgaattctcacaatgaacaataataagattaaaatagctttc
cccgttgcagcgcatgggtatttttctagtaaaaataaaagataaacttagactcaaaacatttacaaaaacaacccctaaagttcctaaagccc
aaagtgctatccacgatccatagcaagcccagcccaacccaacccaacccaacccaccccagtccagccaactggacaatagtctccacacc
cccccactatcaccgtgagttgtccgcacgcaccgcacgtctcgcagccaaaaaaaaaaaaagaaagaaaaaaaagaaaaagaaaaaac
agcaggtgggtccgggtcgtgggggccggaaacgcgaggaggatcgcgagccagcgacgaggccggccctccctccgcttccaaagaaac
gcccccccatcgccactatatacataccccccccctctcctcccatcccccaaccctaccaccaccaccaccacctccacctcctcccccctcg
ctgccggacgacgagctcctcccccctccccctccgccgccgcgccggtaaccaccccgcccctctcctctttctttctccgtttttttttccgtctc
ggtctcgatctttggccttggtagtttgggtgggcgagaggcggcttcgtgcgcgcccagatcggtgcgcgggaggggcgggatctcgcggctggg
gctctcgccggcgtggatccggcccggatctcgcggggaatggggctctcggatgtagatctgcgatccgccgttgttgggggagatgatgggg
gtttaaaatttccgccatgctaaacaagatcaggaagaggggaaaagggcactatggtttatatttttatatatttctgctgcttcgtcaggcttagatgtg
ctagatctttctttcttctttttgtgggtagaatttgaatccctcagcattgttcatcggtagtttttcttttcatgatttgtgacaaatgcagcctcgtgcggagctt
ttttgtaggtagacgataagctatcgatgggtcagtcccttatgttacgtcctgtagaaaccccaacccgtgaaatcaaaaaactcgacggcctgtgg
gcattcagtctggatcgcgaaaactgtggaattgatcagcgttggtgggaaagcgcgttacaagaaagccgggcaattgctgtgccaggcagtttt
aacgatcagttcgccgatgcagatattcgtaattatgcgggcaacgtctggtatcagcgcgaagtctttataccgaaaggtaagtagtgtttttggata
actgagtttgcctatgattttgtatttactgagatgtttgtcctctttgtgcaggttggcaggccagcgtatcgtgctgcgtttcgatgcggtcactcattacg
gcaaagtgtgggtcaataatcaggaagtgatggagcatcagggcggctatacgccatttgaagccgatgtcacgccgtatgttattgccgggaaa
agtgtacgtatcaccgtttgtgtgaacaacgaactgaactggcagactatcccgccgggaatggtgattaccgacgaaaacggcaagaaaaagc
agtcttacttccatgatttctttaactatgccggaatccatcgcagcgtaatgctctacaccacgccgaacacctgggtggacgatatcaccgtggtga
cgcatgtcgcgcaagactgtaaccacgcgtctgttgactggcaggtggtggccaatggtgatgtcagcgttgaactgcgtgatgcggatcaacagg
tggttgcaactggacaaggcactagcgggactttgcaagtggtgaatccgcacctctggcaaccgggtgaaggttatctctatgaactgtgcgtcac
agccaaaagccagacagagtgtgatatctacccgcttcgcgtcggcatccggtcagtggcagtgaagggccaacagttcctgattaaccacaaa
ccgttctactttactggctttggtcgtcatgaagatgcggacttacgtggcaaaggattcgataacgtgctgatggtgcacgaccacgcattaatggac
tggattggggccaactcctaccgtacctcgcattacccttacgctgaagagatgctcgactgggcagatgaacatggcatcgtggtgattgatgaaa
ctgctgctgtcggctttaacctctctttaggcattggtttcgaagcgggcaacaagccgaaagaactgtacagcgaagaggcagtcaacggggaa
actcagcaagcgcacttacaggcgattaaagagctgatagcgcgtgacaaaaaccacccaagcgtggtgatgtggagtattgccaacgaaccg
gatacccgtccgcaaggtgcacgggaatatttcgcgccactggcggaagcaacgcgtaaactcgacccgacgcgtccgatcacctgcgtcaatg
taatgttctgcgacgctcacaccgataccatcagcgatctctttgatgtgctgtgcctgaaccgttattacggatggtatgtccaaagcggcgatttgga
aacggcagagaaggtactggaaaaagaacttctggcctggcaggagaaactgcatcagccgattatcatcaccgaatacggcgtggatacgtta
gccgggctgcactcaatgtacaccgacatgtggagtgaagagtatcagtgtgcatggctggatatgtatcaccgcgtctttgatcgcgtcagcgccg
tcgtcggtgaacaggtatggaatttcgccgatttgcgacctcgcaaggcatattgcgcgttggcggtaacaagaaagggatcttcactcgcgaccg
caaaccgaagtcggcggcttttctgctgcaaaaacgctggactggcatgaacttcggtgaaaaaccgcagcagggaggcaaacaatgaatcaa
caactctcctggcgcaccatcgtcggctacagcctcgggaattgggatcctctagagtcaagcagatcgttcaaacatttggcaataaagtttcttaa
 
gattgaatcctgttgccggtcttgcgatgattatcatataatttctgttgaattacgttaagcatgtaataattaacatgtaatgcatgacgttatttatgagat
gggtttttatgattagagtcccgcaattatacatttaatacgcgatagaaaacaaaatatagcgcgcaaactaggataaattatcgcgcgcggtgtca
tctatgttactagatcgacctgcaggcatgccaattccaatcccacaaaaatctgagcttaacagcacagttgctcctctcagagcagaatcgggtat
tcaacaccctcatatcaactactacgttgtgtataacggtccacatgccggtatatacgatgactggggttgtacaaaggcggcaacaaacggcgtt
cccggagttgcacacaagaaatttgccactattacagaggcaagagcagcagctgacgcgtacacaacaagtcagcaaacagacaggttgaa
cttcatccccaaaggagaagctcaactcaagcccaagagctttgctaaggccctaacaagcccaccaaagcaaaaagcccactggctcacgct
aggaaccaaaaggcccagcagtgatccagccccaaaagagatctcctttgccccggagattacaatggacgatttcctctatctttacgatctagg
aaggaagttcgaaggtgaaggtgacgacactatgttcaccactgataatgagaaggttagcctcttcaatttcagaaagaatgctgacccacagat
ggttagagaggcctacgcagcaggtctcatcaagacgatctacccgagtaacaatctccaggagatcaaataccttcccaagaaggttaaagat
gcagtcaaaagattcaggactaattgcatcaagaacacagagaaagacatatttctcaagatcagaagtactattccagtatggacgattcaagg
cttgcttcataaaccaaggcaagtaatagagattggagtctctaaaaaggtagttcctactgaatctaaggccatgcatggagtctaagattcaaatc
gaggatctaacagaactcgccgtgaagactggcgaacagttcatacagagtcttttacgactcaatgacaagaagaaaatcttcgtcaacatggtg
gagcacgacactctggtctactccaaaaatgtcaaagatacagtctcagaagaccaaagggctattgagacttttcaacaaaggataatttcggga
aacctcctcggattccattgcccagctatctgtcacttcatcgaaaggacagtagaaaaggaaggtggctcctacaaatgccatcattgcgataaag
gaaaggctatcattcaagatctctctgccgacagtggtcccaaagatggacccccacccacgaggagcatcgtggaaaaagaagacgttccaa
ccacgtcttcaaagcaagtggattgatgtgacatctccactgacgtaagggatgacgcacaatcccactatccttcgcaagacccttcctctatataa
ggaagttcatttcatttggagaggacacgctcgagtataagagctctatttttacaacaattaccaacaacaacaacaacaaacaacattacaatt
acatttacaattaccatggaacgagctatacaaggaaacgatgctagggaacaagcttatggtgaacgttggaatggaggatcaggaagttccac
ttctcccttcaaacttcctgacgaaagtccgagttggactgagtggcggctacataacgatgagacgatttcgaatcaagataatccccttggtttcaa
ggaaagctggggtttcgggaaagttgtatttaagagatatctcagatacgacggacggaaacttcactgcacagagtccttggatcttggacggg
agattcggttaactatgcagcatctcgatttctcggtttcgaccagatcggatgtacctatagtattcggtttcgaggagttagtgtcaccatttctggagg
gtcgcgaactcttcagcatctcagtgaaatggcaattcggtctaagcaagaactgctacagcttaccccagtcaaagtggaaagtgatgtatcaag
aggatgccctgaaggtgttgaaaccttcgaagaagaaagcgagtaaggatcctctagagtcctgctttaatgagatatgcgagacgcctatgatcg
catgatatttgctttcaattctgttgtgcacgttgtaaaaaacctgagcatgtgtagctcagatccttaccgccggtttcggttcattctaatgaatatatcac
ccgttactatcgtatttttatgaataatattctccgttcaatttactgattgtaccctactacttatatgtacaatattaaaatgaaaacaatatattgtgctgaa
taggtttatagcgacatctatgatagagcgccacaataacaaacaattgcgttttattattacaaatccaattttaaaaaaagcggcagaaccggtca
aacctaaaagactgattacataaatcttattcaaatttcaaaagtgccccaggggctagtatctacgacacaccgagcggcgaactaataacgctc
actgaagggaactccggttccccgccggcgcgcatgggtgagattccttgaagttgagtattggccgtccgctctaccgaaagttacgggcaccatt
caacccggtccagcacggcggccgggtaaccgacttgctgccccgagaattatgcagcatttttttggtgtatgtgggccccaaatgaagtgcaggt
caaaccttgacagtgacgacaaatcgttgggcgggtccagggcgaattttgcgacaacatgtcgaggctcagcaggacctgcataagctcttctgt
cagcgggcccactgcatccaccccagtacattaaaaacgtccgcaatgtgttattaagttgtctaagcgtcaatttgtttacaccacaatatatcctgc
caccagccagccaacagctccccgaccggcagctcggcacaaaatcaccactcgatacaggcagcccatcag
pNMD050 
cctgtggttggcacatacaatggacgaacggataaaccttttcacgcccttttaaatatccgattattctaataaacgctcttttctcttaggtttacccgc
caatatatcctgtcaaacactgatagtttaaactgaaggcgggaaacgacaatctgatctaagctaggcatgcctgcaggtcaacatggtggagca
cgacacgcttgtctactccaaaaatatcaaagatacagtctcagaagaccaaagggcaattgagacttttcaacaaagggtaatatccggaaacc
tcctcggattccattgcccagctatctgtcactttattgtgaagatagtggaaaaggaaggtggctcctacaaatgccatcattgcgataaaggaaag
gccatcgttgaagatgcctctgccgacagtggtcccaaagatggacccccacccacgaggagcatcgtggaaaaagaagacgttccaaccac
gtcttcaaagcaagtggattgatgtgatatctccactgacgtaagggatgacgcacaatcccactatccttcgcaagacccttcctctatataaggaa
gttcatttcatttggagaggagaaaactaaaccatacaccaccaacacaaccaaacccaccacgcccaattgttacacacccgcttgaaaaaga
aagtttaacaaatggccaaggtgcgcgaggtttaccaatcttttacagactccaccacaaaaactctcatccaagatgaggcttatagaaacattcg
ccccatcatggaaaaacacaaactagctaacccttacgctcaaacggttgaagcggctaatgatctagaggggttcggcatagccaccaatccct
atagcattgaattgcatacacatgcagccgctaagaccatagagaataaacttctagaggtgcttggttccatcctaccacaagaacctgttacattt
atgtttcttaaacccagaaagctaaactacatgagaagaaacccgcggatcaaggacattttccaaaatgttgccattgaaccaagagacgtagc
caggtaccccaaggaaacaataattgacaaactcacagagatcacaacggaaacagcatacattagtgacactctgcacttcttggatccgagct
acatagtggagacattccaaaactgcccaaaattgcaaacattgtatgcgaccttagttctccccgttgaggcagcctttaaaatggaaagcactca
cccgaacatatacagcctcaaatacttcggagatggtttccagtatataccaggcaaccatggtggcggggcataccatcatgaattcgctcatcta
caatggctcaaagtgggaaagatcaagtggagggaccccaaggatagctttctcggacatctcaattacacgactgagcaggttgagatgcaca
cagtgacagtacagttgcaggaatcgttcgcggcaaaccacttgtactgcatcaggagaggagacttgctcacaccggaggtgcgcactttcggc
caacctgacaggtacgtgattccaccacagatcttcctcccaaaagttcacaactgcaagaagccgattctcaagaaaactatgatgcagctcttct
tgtatgttaggacagtcaaggtcgcaaaaaattgtgacatttttgccaaagtcagacaattaattaaatcatctgacttggacaaatactctgctgtgga
actggtttacttagtaagctacatggagttccttgccgatttacaagctaccacctgcttctcagacacactttctggtggcttgctaacaaagacccttg
caccggtgagggcttggatacaagagaaaaagatgcagctgtttggtcttgaggactacgcgaagttagtcaaagcagttgatttccacccggtgg
atttttctttcaaagtggaaacttgggacttcagattccaccccttgcaagcgtggaaagccttccgaccaagggaagtgtcggatgtagaggaaatg
gaaagtttgttctcagatggggacctgcttgattgcttcacaagaatgccagcttatgcggtaaacgcagaggaagatttagctgcaatcaggaaaa
cgcccgagatggatgtcggtcaagaagttaaagagcctgcaggagacagaaatcaatactcaaaccctgcagaaactttcctcaacaagctcca
caggaaacacagtagggaggtgaaacaccaggccgcaaagaaagctaaacgcctagctgaaatccaggagtcaatgagagctgaaggtga
tgccgaaccaaatgaaataagcggacgatggggcaatacccagcaacgccgaacttcctggcacgaatgatgccagacaagaactcaca
ctcccaaccactaaacctgtccctgcaaggtgggaagatgcttcattcacagattctagtgtggaagaggagcaggttaaactccttggaaaagaa
 
accgttgaaacagcgacgcaacaagtcatcgaaggacttccttggaaacactggattcctcaattaaatgctgttggattcaaggcgctggaaattc
agagggataggagtggaacaatgatcatgcccatcacagaaatggtgtccgggctggaaaaagaggacttccctgaaggaactccaaaagag
ttggcacgagaattgttcgctatgaacagaagccctgccaccatccctttggacctgcttagagccagagactacggcagtgatgtaaagaacaa
gagaattggtgccatcacaaagacacaggcaacgagttggggcgaatacttgacaggaaagatagaaagcttaactgagaggaaagttgcga
cttgtgtcattcatggagctggaggttctggaaaaagtcatgccatccagaaggcattgagagaaattggcaagggctcggacatcactgtagtcct
gccgaccaatgaactgcggctagattggagtaagaaagtgcctaacactgagccctatatgttcaagacctctgaaaaggcgttaattgggggaa
caggcagcatagtcatctttgacgattactcaaaacttcctcccggttacatagaagccttagtctgtttctactctaaaatcaagctaatcattctaaca
ggagatagcagacaaagcgtctaccatgaaactgctgaggacgcctccatcaggcattgggaccagcaacagagtacttctcaaaatactgcc
gatactatctcaatgccacacaccgcaacaagaaagatcttgcgaacatgcttggtgtctacagtgagagaacgggagtcaccgaaatcagcat
gagcgccgagttcttagaaggaatcccaactttggtaccctcggatgagaagagaaagctgtacatgggcaccgggaggaatgacacgttcaca
tacgctggatgccaggggctaactaagccgaaggtacaaatagtgttggaccacaacacccaagtgtgtagcgcgaatgtgatgtacacggcac
tttctagagccaccgataggattcacttcgtgaacacaagtgcaaattcctctgccttctgggaaaagttggacagcaccccttacctcaagactttcc
tatcagtggtgagagaacaagcactcagggagtacgagccggcagaggcagagccaattcaagagcctgagccccagacacacatgtgtgtc
gagaatgaggagtccgtgctagaagagtacaaagaggaactcttggaaaagtttgacagagagatccactctgaatcccatggtcattcaaactg
tgtccaaactgaagacacaaccattcagttgttttcgcatcaacaagcaaaagatgagactctcctctgggcgactatagatgcgcggctcaagac
cagcaatcaagaaacaaacttccgagaattcctgagcaagaaggacattggggacgttctgtttttaaactaccaaaaagctatgggtttacccaa
agagcgtattcctttttcccaagaggtctgggaagcttgtgcccacgaagtacaaagcaagtacctcagcaagtcaaagtgcaacttgatcaatgg
gactgtgagacagagcccagacttcgatgaaaataagattatggtattcctcaagtcgcagtgggtcacaaaggtggaaaaactaggtctaccca
agattaagccaggtcaaaccatagcagcctttaccagcagactgtgatgctttttggaactatggctaggtacatgcgatggttcagacaggctttcc
agccaaaagaagtcttcataaactgtgagacgacgccagatgacatgtctgcatgggccttgaacaactggaatttcagcagacctagcttggcta
atgactacacagctttcgaccagtctcaggatggagccatgttgcaatttgaggtgctcaaagccaaacaccactgcataccagaggaaatcattc
aggcatacatagatattaagactaatgcacagatttcctaggcacgttatcaattatgcgcctgactggtgaaggtcccacttttgatgcaaacactg
agtgcaacatagcttacacccatacaaagtttgacatcccagccggaactgctcaagtttatgcaggagacgactccgcactggactgtgttccag
aagtgaagcatagtttccacaggcttgaggacaaattactcctaaagtcaaagcctgtaatcacgcagcaaaagaagggcagttggcctgagtttt
gtggttggctgatcacaccaaaaggggtgatgaaagacccaattaagctccatgttagcttaaaattggctgaagctaagggtgaactcaagaaat
gtcaagattcctatgaaattgatctgagttatgcctatgaccacaaggactctctgcatgacttgttcgatgagaaacagtgtcaggcacacacactc
acttgcagaacactaatcaagtcagggagaggcactgtctcactttcccgcctcagaaactttctttaaccgttaagttaccttagagatttgaataag
atgtcagcaccagctagtacaacacagcccatagggtcaactacctcaactaccacaaaaactgcaggcgcaactcctgccacagcttcaggc
ctgttcactatcccggatggggatttctttagtacagcccgtgccatagtagccagcaatgctgtcgcaacaaatgaggacctcagcaagattgagg
ctatttggaaggacatgaaggtgcccacagacactatggcacaggctgcttgggacttagtcagacactgtgctgatgtaggatcatccgctcaaa
cagaaatgatagatacaggtccctattccaacggcatcagcagagctagactggcagcagcaattaaagaggtgtgcacacttaggcaatttgc
atgaagtatgccccagtggtatggaactggatgttaactaacaacagtccacctgctaactggcaagcacaaggtttcaagcctgagcacaaattc
gctgcattcgacttcttcaatggagtcaccaacccagctgccatcatgcccaaagaggggctcatccggccaccgtctgaagctgaaatgaatgct
gcccaaactgctgcctttgtgaagattacaaaggccagggcacaatccaacgactttgccagcctagatgcagctgtcactcgaggaaggatcac
cggaacgaccacagcagaggcagtcgttactctgcctcctccataacagaaactttctttaaccgttaagttaccttagagatttgaataagatggat
attctcatcagtagtttgaaaagtttaggttattctaggacttccaaatctttagattcaggacctttggtagtacatgcagtagccggagccggtaagtcc
acagccctaaggaagttgatcctcagacacccaacattcaccgtgcatacactcggtgtccctgacaaggtgagtatcagaactagaggcataca
gaagccaggacctattcctgagggcaacttcgcaatcctcgatgagtatactttggacaacaccacaaggaactcataccaggcactttttgctgac
ccttatcaggcaccggagtttagcctagagccccacttctacttggaaacatcatttcgagttccgaggaaagtggcagatttgatagctggctgtgg
cttcgatttcgagacgaactcaccggaagaagggcacttagagatcactggcatattcaaagggcccctactcggaaaggtgatagccattgatg
aggagtctgagacaacactgtccaggcatggtgttgagtttgttaagccctgccaagtgacgggacttgagttcaaagtagtcactattgtgtctgccg
caccaatagaggaaattggccagtccacagctttctacaacgctatcaccaggtcaaagggattgacatatgtccgcgcagggccataggctgac
cgctccggtcaattctgaaaaagtgtacatagtattaggtctatcatttgctttagtttcaattacctttctgctttctagaaatagcttaccccacgtcggtg
acaacattcacagcttgccacacggaggagcttacagagacggcaccaaagcaatcttgtacaactccccaaatctagggtcacgagtgagtct
acacaacggaaagaacgcagcattgctgccgtttgctactgactttgctgatctatggaagtaaatacatatctcaacgcaatcatacttgtgcttgt
ggtaacaatcatagcagtcattagcacttccttagtgaggactgaaccttgtgtcatcaagattactggggaatcaatcacagtgttggcttgcaaact
agatgcagaaaccataaggccattgccgatctcaagccactctccgttgaacggttaagtttccattgatactcgaaagaggtcagcaccagcta
gcaacaaacaagaacatgtgacggctgaactccccctggcttgttaggcatcgcaaatatcattgaatgttccttccactgcaaccgatcatcaatcc
ctagttctccaaacaggttctgcatatttgggtaagccccaaagaatatgtgcaacccagtctcgtaccaatctccatcatcatctttccatgcagctac
cttcccacctaggacatctcttgcctccagcaatatcggtttgtgaccagcatctgccagatattttgctgtagacaaaccacccaaacctgcaccag
caataacaatctccaatggtttagttgggcgtgaggaagtacgaaagatcggtcgtatcactggaacaacaaccgctgaggctgttgtcactctacc
accaccataactacgtctacataaccgacgcctaccccagtttcatagtattttctggtttgattgtatgaataatataaataaaaaaaaaaaaaaaa
aaaaaaactagtgagctcttctgtcagcgggcccactgcatccaccccagtacattaaaaacgtccgcaatgtgttattaagttgtctaagcgtcaatt
tgtttacaccacaatatatcctgccaccagccagccaacagctccccgaccggcagctcggcacaaaatcaccactcgatacaggcagcccatc
ag
pNMD1953

cctgtggttggcacatacaaatggacgaacggataaaccttttcacgcccttttaaatatccgattattctaataaacgctcttttctcttaggtttacccgc
caatatatcctgtcaaacactgatagtttaaactgaaggcgggaaacgacaatctgatctaagctaggcatgcctgcaggtcaacatggtggagca
cgacacgcttgtctactccaaaaatatcaaagatacagtctcagaagaccaaagggcaattgagacttttcaacaaagggtaatatccggaaacc
tcctcggattccattgcccagctatctgtcactttattgtgaagatagtggaaaaggaaggtggctcctacaaatgccatcattgcgataaaggaaag

gccatcgttgaagatgcctctgccgacagtggtcccaaagatggacccccacccacgaggagcatcgtggaaaaagaagacgttccaaccac
gtcttcaaagcaagtggattgatgtgatatctccactgacgtaagggatgacgcacaatcccactatccttcgcaagacccttcctctatataaggaa
gttcatttcatttggagaggagaaaactaaaccatacaccaccaacacaaccaaacccaccacgcccaattgttacacacccgcttgaaaaaga
aagtttaacaaatggccaaggtgcgcgaggtttaccaatcttttacagactccaccacaaaaactctcatccaagatgaggcttatagaaacattcg
ccccatcatggaaaaacacaaactagctaacccttacgctcaaacggttgaagcggctaatgatctagaggggttcggcatagccaccaatccct
atagcattgaattgcatacacatgcagccgctaagaccatagagaataaacttctagaggtgcttggttccatcctaccacaagaacctgttacattt
atgtttcttaaacccagaaagctaaactacatgagaagaaacccgcggatcaaggacattttccaaaatgttgccattgaaccaagagacgtagc
caggtaccccaaggaaacaataattgacaaactcacagagatcacaacggaaacagcatacattagtgacactctgcacttcttggatccgagct
acatagtggagacattccaaaactgcccaaaattgcaaacattgtatgcgaccttagttctccccgttgaggcagcctttaaaatggaaagcactca
cccgaacatatacagcctcaaatacttcggagatggtttccagtatataccaggcaaccatggtggcggggcataccatcatgaattcgctcatcta
caatggctcaaagtgggaaagatcaagtggagggaccccaaggatagctttctcggacatctcaattacacgactgagcaggttgagatgcaca
cagtgacagtacagttgcaggaatcgttcgcggcaaaccacttgtactgcatcaggagaggagacttgctcacaccggaggtgcgcactttcggc
caacctgacaggtacgtgattccaccacagatcttcctcccaaaagttcacaactgcaagaagccgattctcaagaaaactatgatgcagctcttct
tgtatgttaggacagtcaaggtcgcaaaaaattgtgacattttgccaaagtcagacaattaattaaatcatctgacttggacaaatactctgctgtgga
actggtttacttagtaagctacatggagttccttgccgatttacaagctaccacctgcttctcagacacactttctggtggcttgctaacaaagaccctg
caccggtgagggcttggatacaagagaaaaagatgcagctgtttggtcttgaggactacgcgaagttagtcaaagcagttgatttccacccggtgg
atttttctttcaaagtggaaacttgggacttcagattccaccccttgcaagcgtggaaagccttccgaccaagggaagtgtcggatgtagaggaaatg
gaaagtttgttctcagatggggacctgcttgattgcttcacaagaatgccagcttatgcggtaaacgcagaggaagatttagctgcaatcaggaaaa
cgcccgagatggatgtcggtcaagaagttaaagagcctgcaggagacagaaatcaatactcaaaccctgcagaaactttcctcaacaagctcca
caggaaacacagtagggaggtgaaacaccaggccgcaaagaaagctaaacgcctagctgaaatccaggagtcaatgagagctgaaggtga
tgccgaaccaaatgaaataagcgggacgatgggggcaatacccagcaacgccgaacttcctggcacgaatgatgccagacaagaactcaca
ctcccaaccactaaacctgtccctgcaaggtgggaagatgcttcattcacagattctagtgtggaagaggagcaggttaaactccttggaaaagaa
accgttgaaacagcgacgcaacaagtcatcgaaggacttccttggaaacactggattcctcaattaaatgctgttggattcaaggcgctggaaattc
agagggataggagtggaacaatgatcatgcccatcacagaaatggtgtccgggctggaaaaagaggacttccctgaaggaactccaaaagag
ttggcacgagaattgttcgctatgaacagaagccctgccaccatccctttggacctgcttagagccagagactacggcagtgatgtaaagaacaa
gagaattggtgccatcacaaagacacaggcaacgagttggggcgaatacttgacaggaaagatagaaagcttaactgagaggaaagttgcga
cttgtgtcattcatggagctggaggttctggaaaaagtcatgccatccagaaggcattgagagaaattggcaagggctcggacatcactgtagtcct
gccgaccaatgaactgcggctagattggagtaagaaagtgcctaacactgagccctatatgttcaagacctctgaaaaggcgttaattgggggaa
caggcagcatagtcatctttgacgattactcaaaacttcctcccggttacatagaagccttagtctgtttctactctaaaatcaagctaatcattctaaca
ggagatagcagacaaagcgtctaccatgaaactgctgaggacgcctccatcaggcatttgggaccagcaacagagtacttctcaaaatactgcc
gatactatctcaatgccacacaccgcaacaagaaagatcttgcgaacatgcttggtgtctacagtgagagaacgggagtcaccgaaatcagcat
gagcgccgagttcttagaaggaatcccaactttggtaccctcggatgagaagagaaagctgtacatgggcaccgggaggaatgacacgttcaca
tacgctggatgccaggggctaactaagccgaaggtacaaatagtgttggaccacaacacccaagtgtgtagcgcgaatgtgatgtacacggcac
tttctagagccaccgataggattcacttcgtgaacacaagtgcaaattcctctgccttctgggaaaagttggacagcaccccttacctcaagactttcc
tatcagtggtgagagaacaagcactcagggagtacgagccggcagaggcagagccaattcaagagcctgagccccagacacacatgtgtgtc
gagaatgaggagtccgtgctagaagagtacaaagaggaactcttggaaaagtttgacagagagatccactctgaatcccatggtcattcaaactg
tgtccaaactgaagacacaaccattcagttgttttcgcatcaacaagcaaaagatgagactctcctctgggcgactatagatgcgcggctcaagac
cagcaatcaagaaacaaacttccgagaattcctgagcaagaaggacattggggacgttctgtttttaaactaccaaaaagctatgggtttacccaa
agagcgtattcctttttcccaagaggtctgggaagcttgtgcccacgaagtacaaagcaagtacctcagcaagtcaaagtgcaacttgatcaatgg
gactgtgagacagagcccagacttcgatgaaaataagattatggtattcctcaagtcgcagtgggtcacaaaggtggaaaaactaggtctaccca
agattaagccaggtcaaaccatagcagccttttaccagcagactgtgatgctttttggaactatggctaggtacatgcgatggttcagacaggctttcc
agccaaaagaagtcttcataaactgtgagacgacgccagatgacatgtctgcatgggccttgaacaactggaatttcagcagacctagcttggcta
atgactacacagctttcgaccagtctcaggatggagccatgttgcaatttgaggtgctcaaagccaaacaccactgcataccagaggaaatcattc
aggcatacatagatattaagactaatgcacagattttcctaggcacgttatcaattatgcgcctgactggtgaaggtcccacttttgatgcaaacactg
agtgcaacatagcttacacccatacaaagtttgacatcccagccggaactgctcaagtttatgcaggagacgactccgcactggactgtgttccag
aagtgaagcatagtttccacaggcttgaggacaaattactcctaaagtcaaagcctgtaatcacgcagcaaaagaagggcagttggcctgagtttt
gtggttggctgatcacaccaaaaggggtgatgaaagacccaattaagctccatgttagcttaaaattggctgaagctaagggtgaactcaagaaat
gtcaagattcctatgaaattgatctgagttatgcctatgaccacaaggactctctgcatgacttgttcgatgagaaacagtgtcaggcacacacactc
acttgcagaacactaatcaagtcagggagaggcactgtctcactttcccgcctcagaaactttctttaaccgttaagttaccttagagatttgaataag
atggatattctcatcagtagtttgaaaagtttaggttattctaggacttccaaatctttagattcaggacctttggtagtacatgcagtagccggagccggt
aagtccacagccctaaggaagttgatcctcagacacccaacattcaccgtgcatacactcggtgtccctgacaaggtgagtatcagaactagagg
catacagaagccaggacctattcctgagggcaacttcgcaatcctcgatgagtatactttggacaacaccacaaggaactcataccaggcactttt
gctgacccttatcaggcaccggagtttagcctagagccccacttctacttggaaacatcatttcgagttccgaggaaagtggcagatttgatagctgg
ctgtggcttcgatttcgagacgaactcaccggaagaagggcacttagagatcactggcatattcaaagggcccctactcggaaaggtgatagcca
ttgatgaggagtctgagacaacactgtccaggcatggtgttgagtttgttaagccctgccaagtgacgggacttgagttcaaagtagtcactattgtgt
ctgccgcaccaatagaggaaattggccagtccacagctttctacaacgctatcaccaggtcaaagggattgacatatgtccgcgcagggccatag
gctgaccgctccggtcaattctgaaaaagtgtacatagtattaggtctatcatttgctttagtttcaattacctttctgctttctagaaatagcttaccccacg
tcggtgacaacattcacagcttgccacacggaggagcttacagagacggcaccaaagcaatcttgtacaactccccaaatctagggtcacgagt
gagtctacacaacggaaagaacgcagcatttgctgccgtttgctactgactttgctgatctatggaagtaaatacatatctcaacgcaatcatacttgt
gcttgtggtaacaatcatagcagtcattagcacttccttagtgaggactgaaccttgtgtcatcaagattactggggaatcaatcacagtgttggcttgc
aaactagatgcagaaaccataagggccattgccgatctcaagccactctccgttgaacggttaagtttccattgatactcgaaagatgtcagcacca

gctagtacaacacagcccatagggtcaactacctcaactaccacaaaaactgcaggcgcaactcctgccacagcttcaggcctgttcactatccc
ggatggggatttctttagtacagcccgtgccatagtagccagcaatgctgtcgcaacaaatgaggacctcagcaagattgaggctatttggaagga
catgaaggtgcccacagacactatggcacaggctgcttgggacttagtcagacactgtgctgatgtaggatcatccgctcaaacagaaatgatag
atacaggtccctattccaacggcatcagcagagctagactggcagcagcaattaaagaggtgtgcacacttaggcaattttgcatgaagtatgccc
cagtggtatggaactggatgttaactaacaacagtccacctgctaactggcaagcacaaggtttcaagcctgagcacaaattcgctgcattcgactt
cttcaatggagtcaccaacccagctgccatcatgcccaaagaggggctcatccggccaccgtctgaagctgaaatgaatgctgcccaaactgctg
cctttgtgaagattacaaaggccagggcacaatccaacgactttgccagcctagatgcagctgtcactcgaggaaggatcaccggaacgaccac
agcagaggcagtcgttactctgcctcctccataatgaacggttaagtttccattgatactcgaaagaggtcagcaccagctagcaagaaacaaga
aaccatggcgaacaaacacttgtccctctccctcttcctcgtcctccttggcctgtcggccagcttggcctcaggtgctttaacagtaaacaccaacgt
aacatcgttgagcgtccagaagaacctgagccgcgcctccgacgcactgtcgacgtcgatgggtcgtttgtcttccggcttgaagatcatgagctcg
aaagatgacgccgccggcctgaacattgctaccaagatcaactcgcagatcaaaggtcagaccatggcgatcaaaaacgccaacgacggtat
gtccattgctcagaccgctgaaggcgctctgcaagagtcgaccaacattctgcagcgtatgcgtgaactggctgttcagtcgcgaaacgacagca
acagcgctactgaccgcgttgcgctgaacaaagaattcacccagatgagttcggaactgacccgtatcgccaacagtaccaacctgaacggca
aaaacctgattgacggttctgccagcaccatgactttccaggttggctccaactccggcgcctcgaaccagatctcgctgtctttgagcgccagttttg
acgcgaacaccctgggtgtcggttcggcgatcaccatcgtcggctccgacagcgccgcagcggagactaacttctctgcttcaatcgccgcgatc
gactcggctctgcagaccatcaacaacacccgttcggatctgggtgctgcgcaaaaccgtctgtccagcaccatctccaacctgcagaacatcaa
cgaaaacgccagtgctgcactgggtcgtatccaggataccgactttgctgctgaaactgcacagctgaccaagcaacagaccctgcaacaggct
tccacctcgatcctggcacaggctaaccagctgccgtccgctgtactgaaactgcttcagtaagcttggtcgtatcactggaacaacaaccgctgag
gctgttgtcactctaccaccaccataactacgtctacataaccgacgcctaccccagtttcatagtattttctggtttgattgtatgaataatataaataaa
aaaaaaaaaaaaaaaaaaaactagtgagctcttctgtcagcgggcccactgcatccaccccagtacattaaaaacgtccgcaatgtgttattaa
gttgtctaagcgtcaatttgtttacaccacaatatatcctgccaccagccagccaacagctccccgaccggcagctcggcacaaaatcaccactcg
atacaggcagcccatcag

## Claims

1. A process of generating or altering a trait in a plant, comprising
(i) growing said plant up to a desired growth state;
(ii) expressing, in said plant, a protein or an RNA capable of generating or altering said trait, comprising transient transfection by spraying aerial parts of said plant with an aqueous suspension containing cells of an *Agrobacterium* strain, at least one abrasive suspended in said suspension, and, as agricultural spray adjuvant, a non-ionic surfactant in a concentration between 0.25 and 5.0 g per liter of said suspension,
said *Agrobacterium* strain comprising a DNA molecule comprising a nucleic acid construct containing a DNA sequence of interest, said DNA sequence of interest encoding said protein or said RNA, and
said abrasive being a particulate material that is essentially insoluble in the aqueous suspension of *Agrobacterium* cells.

2. A process of producing a protein of interest in a plant, comprising
(i) growing said plant up to a desired growth state;
(ii) expressing, in said plant, said protein of interest, comprising transient transfection by spraying aerial parts of said plant with an aqueous suspension containing cells of an *Agrobacterium* strain, at least one abrasive suspended in said suspension, and, as agricultural spray adjuvant, a non-ionic surfactant in a concentration between 0.25 and 5.0 g per liter of said suspension,
said *Agrobacterium* strain comprising a DNA molecule comprising a nucleic acid construct containing a DNA sequence of interest, said DNA sequence of interest encoding said protein of interest, and
said abrasive being a particulate material that is essentially insoluble in the aqueous suspension of *Agrobacterium* cells.

3. A process of protecting crop plants on a field from a pest, comprising
(i) growing said plants in soil of said field;
(ii) determining, in a desired growth state of said plants, infestation of at least one of said plants by a pest;
(iii) expressing, in said plants, a protein or an RNA that is detrimental to the pest determined in the previous step, comprising transient transfection by spraying aerial parts of said plants with an aqueous suspension containing cells of an *Agrobacterium* strain, at least one abrasive suspended in said suspension, and, as agricultural spray adjuvant, a non-ionic surfactant in a concentration between 0.25 and 5.0 g per liter of said suspension,
said *Agrobacterium* strain comprising a DNA molecule comprising a nucleic acid construct containing a DNA sequence of interest operably linked to a promoter, said DNA sequence of interest encoding said protein or said RNA, and
said abrasive being a particulate material that is essentially insoluble in the aqueous suspension of *Agrobacterium* cells.

4. The process according to any one of claims 1 to 3, wherein said aqueous suspension contains said cells of said *Agrobacterium* strain in a concentration of at most 2.2·10⁷, preferably of at most 1.1·10⁷, more preferably of at most 4.4·10⁶, more preferably of at most 1.1·10⁶ cfu/ml of said suspension.

5. The process according to any one of claims 1 to 4, wherein said abrasive is a particulate inorganic carrier for wettable powders, such as silica or carborundum.

6. The process according to claim 5, wherein said aqueous suspension contains said abrasive in an amount of between 0.02 and 2, preferably between 0.05 and 1 and more preferably between 0.1 and 0.5 % by weight of said suspension.

7. The process according to claim 5 or 6, wherein the median particle size of the abrasive added to the suspension is between 0.1 and 30, preferably between 0.1 and 10, more preferably between 0.5 and 10, and most preferably between 0.5 and 5 µm.

8. The process according to any one of claims 5 to 7, wherein the abrasive has a D90 value of at most 40 µm, preferably of at most 30 µm; and wherein the abrasive does not contain particles having a size above 45 µm, preferably not above 40 µm.

9. The process according to any one of claims 1 to 8, wherein the spray adjuvant is an organo-silicone surfactant.

10. The process according to claim 9, wherein the organo-silicone surfactant is polyalkyleneoxide-modified heptamethyltrisiloxane.

11. The process according to any one of claims 1 to 10, wherein said nucleic acid construct is flanked by a T-DNA border sequence on at least one side, which permits the transfer of said nucleic acid construct into cells of said plant.

12. The process according to any one of claims 1 to 11, wherein said nucleic acid construct encodes a replicating viral vector encoding said protein of interest, said replicating viral vector being incapable of system movement in said plant.

13. The process according to any one of claims 1 to 11, wherein said DNA sequence of interest is operably linked to a promoter active in plant cells.

14. The process according to any one of claims 1 to 3, wherein said non-ionic surfactant is an organo-silicone surfactant, and the concentration of the organo-silicone surfactant in the agrobacterial suspension used for spraying is between 0.5 and 5.0 g per liter of said suspension.

## Patentansprüche

1. Verfahren zum Erzeugen oder zum Verändern eines Merkmals in einer Pflanze, umfassend:
(i) Aufziehen der Pflanze bis zu einem gewünschten Wachstumszustand;
(ii) Exprimieren, in der Pflanze, eines Proteins oder einer RNA, das/die das Merkmal erzeugen oder verändern kann, umfassend die transiente Transfektion durch Besprühen der oberirdischen Teile der Pflanze mit einer wässrigen Suspension, die Zellen eines *Agrobacterium*-Stamms enthält, wenigstens einen Abrasivstoff, der in der Suspension suspendiert ist, und als landwirtschaftlichen Sprühmittel-Hilfsstoff ein nichtionisches Tensid in einer Konzentration zwischen 0,25 und 5,0 g pro Liter der Suspension,
wobei der *Agrobacterium*-Stamm ein DNA-Molekül umfasst, das ein Nukleinsäure-Konstrukt umfasst, das die gewünschte DNA-Sequenz enthält, wobei die gewünschte DNA-Sequenz für das Protein oder die RNA codiert, und
wobei der Abrasivstoff ein partikuläres Material ist, das im Wesentlichen in der wässrigen Suspension der *Agrobacterium-*Zellen unlöslich ist.

2. Verfahren zur Erzeugung eines gewünschten Proteins in einer Pflanze, umfassend:
(i) Aufziehen der Pflanze bis zu einem gewünschten Wachstumszustand;
(ii) Exprimieren, in der Pflanze, eines gewünschten Proteins, umfassend die transiente Transfektion durch Besprühen der oberirdischen Teile der Pflanze mit einer wässrigen Suspension, die Zellen eines *Agrobacterium*-Stamms enthält, wenigstens einen Abrasivstoff, der in der Suspension suspendiert ist, und als landwirtschaftlichen Sprühmittel-Hilfsstoff ein nichtionisches Tensid in einer Konzentration zwischen 0,25 und 5,0 g pro Liter der Suspension,
wobei der *Agrobacterium*-Stamm ein DNA-Molekül umfasst, das ein Nukleinsäure-Konstrukt umfasst, das die gewünschte DNA-Sequenz enthält, wobei die gewünschte DNA-Sequenz für das gewünschte Protein codiert, und
wobei der Abrasivstoff ein partikuläres Material ist, das im Wesentlichen in der wässrigen Suspension der *Agrobacterium*-Zellen unlöslich ist.

3. Verfahren zum Schutz von Nutzpflanzen auf einem Feld vor einem Schädling, umfassend:
(i) Aufziehen der Pflanzen im Boden des Feldes;
(ii) Bestimmen, in einem gewünschten Wachstumszustand der Pflanzen, des Befalls wenigstens einer der Pflanzen durch einen Schädling;
(iii) Exprimieren, in den Pflanzen, eines Proteins oder einer RNA, das/die für den Schädling, der im vorigen Schritt bestimmt wurde, schädlich ist, umfassend die transiente Transfektion durch Besprühen der oberirdischen Teile der Pflanzen mit einer wässrigen Suspension, die Zellen eines *Agrobacterium*-Stamms enthält, wenigstens einen Abrasivstoff, der in der Suspension suspendiert ist, und als landwirtschaftlichen Sprühmittel-Hilfsstoff ein nichtionisches Tensid in einer Konzentration zwischen 0,25 und 5,0 g pro Liter der Suspension,
wobei der *Agrobacterium*-Stamm ein DNA-Molekül umfasst, das ein Nukleinsäure-Konstrukt umfasst, das die gewünschte DNA-Sequenz, die operativ mit einem Promotor verbunden ist, enthält, wobei die gewünschte DNA-Sequenz für das Protein oder die RNA codiert, und
wobei der Abrasivstoff ein partikuläres Material ist, das im Wesentlichen in der wässrigen Suspension der *Agrobacterium-*Zellen unlöslich ist.

4. Das Verfahren nach einem der Ansprüche 1 bis 3, wobei die wässrige Suspension die Zellen des *Agrobacterium*-Stamms in einer Konzentration von höchstens 2,2 x 10⁷, bevorzugt von höchstens 1,1 x 10⁷, stärker bevorzugt von höchstens 4,4 x 10⁶, stärker bevorzugt von höchstens 1,1 x 10⁶ KbE/ml der Suspension enthält.

5. Das Verfahren nach einem der Ansprüche 1 bis 4, wobei der Abrasivstoff ein partikulärer anorganischer Träger für benetzbare Pulver, wie Kieselerde oder Karborund, ist.

6. Das Verfahren nach Anspruch 5, wobei die wässrige Suspension den Abrasivstoff in einer Menge von zwischen 0,02 und 2, bevorzugt zwischen 0,05 und 1 und stärker bevorzugt zwischen 0,1 und 0,5 Gewichtsprozent der Suspension enthält.

7. Das Verfahren nach Anspruch 5 oder 6, wobei die mittlere Partikelgröße des zu der Suspension zugegebenen Abrasivstoffs zwischen 0,1 und 30, bevorzugt zwischen 0,1 und 10, stärker bevorzugt zwischen 0,5 und 10 und am stärksten bevorzugt zwischen 0,5 und 5 µm beträgt.

8. Das Verfahren nach einem der Ansprüche 5 bis 7, wobei der Abrasivstoff einen D90-Wert von höchstens 40 µm, bevorzugt 30 µm aufweist; und wobei der Abrasivstoff keine Partikel enthält, die eine Größe über 45 µm, bevorzugt 40 µm aufweisen.

9. Das Verfahren nach einem der Ansprüche 1 bis 8, wobei der Sprühmittel-Hilfsstoff ein Organosilikontensid ist.

10. Das Verfahren nach Anspruch 9, wobei das Organosilikontensid Polyalkylenoxidmodifiziertes Heptamethyltrisiloxan ist.

11. Das Verfahren nach einem der Ansprüche 1 bis 10, wobei das Nukleinsäure-Konstrukt auf wenigstens einer Seite von einer T-DNA-Grenzsequenz flankiert wird, welche den Transfer des Nukleinsäure-Konstrukts in die Zellen der Pflanze erlaubt.

12. Das Verfahren nach einem der Ansprüche 1 bis 11, wobei das Nukleinsäure-Konstrukt einen replizierenden viralen Vektor codiert, der das gewünschte Protein codiert, wobei der replizierende virale Vektor unfähig zur systemischen Bewegung in der Pflanze ist.

13. Das Verfahren nach einem der Ansprüche 1 bis 11, wobei die gewünschte DNA-Sequenz operativ mit einem in Pflanzenzellen aktiven Promotor verbunden ist.

14. Das Verfahren nach einem der Ansprüche 1 bis 3, wobei das nichtionische Tensid ein Organosilikontensid ist und die Konzentration des Organosilikontensids in der Suspension der Agrobakterien, die für das Sprühen verwendet wird, zwischen 0,5 und 5,0 g pro Liter Suspension beträgt.

## Revendications

1. Procédé pour générer ou altérer un trait dans une plante, comprenant
(i) la croissance de ladite plante jusqu'à un état de croissance souhaité ;
(ii) l'expression, dans ladite plante, d'une protéine ou d'un ARN capable de générer ou d'altérer ledit trait, comprenant une transfection transitoire par pulvérisation des parties aériennes de ladite plante avec une suspension aqueuse contenant des cellules d'une souche d'*Agrobacterium,* au moins un abrasif en suspension dans ladite suspension, et, à titre d'adjuvant de pulvérisation à usage agricole, un tensioactif non-ionique à une concentration comprise entre 0,25 et 5,0 g par litre de ladite suspension,
ladite souche d'*Agrobacterium* comprenant une molécule d'ADN comprenant un produit d'assemblage d'acide nucléique contenant une séquence d'ADN présentant un intérêt, ladite séquence d'ADN présentant un intérêt codant ladite protéine ou ledit ARN, et
ledit abrasif étant un matériau particulaire qui est pratiquement insoluble dans la suspension aqueuse des cellules d'*Agrobacterium.*

2. Procédé pour produire une protéine présentant un intérêt dans une plante, comprenant
(i) la croissance de ladite plante jusqu'à un état de croissance souhaité ;
(ii) l'expression, dans ladite plante, de ladite protéine comprenant un intérêt, comprenant une transfection transitoire par pulvérisation des parties aériennes de ladite plante avec une suspension aqueuse contenant des cellules d'une souche d'*Agrobacterium,* au moins un abrasif en suspension dans ladite suspension, et, à titre d'adjuvant de pulvérisation à usage agricole, un tensioactif non-ionique à une concentration comprise entre 0,25 et 5,0 g par litre de ladite suspension,
ladite souche d'*Agrobacterium* comprenant une molécule d'ADN comprenant un produit d'assemblage d'acide nucléique contenant une séquence d'ADN présentant un intérêt, ladite séquence d'ADN présentant un intérêt codant ladite protéine présentant un intérêt, et
ledit abrasif étant un matériau particulaire qui est pratiquement insoluble dans la suspension aqueuse des cellules d'*Agrobacterium.*

3. Procédé pour protéger contre un nuisible des plantes cultivées dans un champ, comprenant
(i) la croissance desdites plantes dans le sol dudit champ ;
(ii) la détermination, dans un état de croissance souhaité desdites plantes, de l'infestation d'au moins l'une desdites plantes par un nuisible ;
(iii) l'expression, dans lesdites plantes, d'une protéine ou d'un ARN qui est nocif pour le nuisible déterminé dans l'étape précédentes, comprenant une transfection transitoire par pulvérisation des parties aériennes desdites plantes avec une suspension aqueuse contenant des cellules d'une souche d'*Agrobacterium,* au moins un abrasif en suspension dans ladite suspension, et, à titre d'adjuvant de pulvérisation à usage agricole, un tensioactif non-ionique à une concentration comprise entre 0,25 et 5,0 g par litre de ladite suspension,
ladite souche d'*Agrobacterium* comprenant une molécule d'ADN comprenant un produit d'assemblage d'acide nucléique contenant une séquence d'ADN présentant un intérêt liée de manière fonctionnelle à un promoteur, ladite séquence d'ADN présentant un intérêt codant ladite protéine ou ledit ARN, et
ledit abrasif étant un matériau particulaire qui est pratiquement insoluble dans la suspension aqueuse des cellules d'*Agrobacterium.*

4. Procédé selon l'une quelconque des revendications 1 à 3, dans lequel ladite suspension aqueuse contient lesdites cellules de ladite souche *d'Agrobacterium* à une concentration d'au plus 2,2 x 10⁷, de préférence d'au plus 1,1 x 10⁷, plus préférablement d'au plus 4,4 x 10⁶, encore plus préférablement d'au plus 1,1 x 10⁶ cfu/ml de ladite suspension.

5. Procédé selon l'une quelconque des revendications 1 à 4, dans lequel ledit abrasif est un support inorganique particulaire pour poudres mouillables, tel que la silice ou le carborundum.

6. Procédé selon la revendication 5, dans lequel ladite suspension aqueuse contient ledit abrasif en une quantité comprise entre 0,02 et 2, de préférence entre 0,05 et 1 et plus préférablement entre 0,1 et 0,5 % en poids de ladite suspension.

7. Procédé selon la revendication 5 ou 6, dans lequel la granulométrie moyenne de l'abrasif ajouté à la suspension est comprise entre 0,1 et 30, de préférence entre 0,1 et 10, plus préférablement entre 0,5 et 10, et le plus préférablement entre 0,5 et 5 µm.

8. Procédé selon l'une quelconque des revendications 5 à 7, dans lequel l'abrasif présente une valeur D90 d'au plus 40 µm, de préférence d'au plus 30 µm ; et dans lequel l'abrasif ne contient pas de particules présentant une taille supérieure à 45 µm, de préférence supérieure à 40 µm.

9. Procédé selon l'une quelconque des revendications 1 à 8, dans lequel l'adjuvant de pulvérisation est un tensioactif siliconé organique.

10. Procédé selon la revendication 9, dans lequel le tensioactif siliconé organique est un heptaméthyltrisiloxane modifié par un poly(oxyde d'alkylène).

11. Procédé selon l'une quelconque des revendications 1 à 10, dans lequel ledit produit d'assemblage d'acide nucléique est flanqué par une séquence de bordure d'ADN-T sur au moins un côté, ce qui permet le transfert dudit produit d'assemblage d'acide nucléique dans des cellules de ladite plante.

12. Procédé selon l'une quelconque des revendications 1 à 11, dans lequel ledit produit d'assemblage d'acide nucléique code un vecteur viral répliquant codant ladite protéine présentant un intérêt, ledit vecteur viral répliquant étant incapable d'un mouvement systémique dans ladite plante.

13. Procédé selon l'une quelconque des revendications 1 à 11, dans lequel ladite séquence d'ADN présentant un intérêt est liée de manière fonctionnelle à un promoteur actif dans des cellules de plante.

14. Procédé selon l'une quelconque des revendications 1 à 3, dans lequel ledit tensioactif non-ionique est un tensioactif siliconé organique, et la concentration du tensioactif siliconé organique dans la suspension d'agrobactéries utilisée pour la pulvérisation est comprise entre 0,5 et 5,0 g par litre de ladite suspension.
